# EUROPEAN PATENT APPLICATION

(11) **EP 2 383 278 A1**
(43) Date of publication of application: **02.11.2011**
(21) Application number: 11151848.6
(22) Date of filing: 08.07.2003
(51) Int. Cl.: C07H 21/04, C12Q 1/68, C12N 5/00

(54) **Method to determine B cell mediated diseases**

(30) Priority: 08.07.2002 US 394485 P
(62) Divisional of application: 10004913.9
(71) Applicant: Genentech, Inc., South San Francisco CA 94080-4990 (US)
(72) Inventor: Chiu, Henry, San Francisco, CA 94116 (US); Clark, Hilary, San Francisco, CA 94131 (US); Dennis, Kathryn, Burlingame, CA 94010 (US); Fong, Sherman, Kalaheo, HI 96741 (US); Schoenfeld, Jill, Burlingame, CA 94010 (US); Wood, William, Cupertino, CA 95014 (US); Wu, Thomas, San Francisco, CA 94110 (US)
(74) Representative: Walton, Seán Malcolm

(57) **Abstract**

The present invention relates to compositions containing novel proteins and methods of using those compositions for the diagnosis and treatment of immune related diseases.

## Description

### Field of the Invention

The present invention relates to compositions and methods useful for the diagnosis and treatment of immune related diseases.

### Background of the Invention

The B lymphocytes play a major role in the humoral immune response as the antibody producing cells. The B cells can generate a highly diverse antibody repertoire that is reactive to almost all potential antigens. Through a process of maturation and clonal selection in the bone marrow, a highly diverse B cell population develops with each B cell clone expressing an antigen specific cell surface receptor, the B cell receptor (BCR), which bear the same specificity as the secreted antibody made by the B cell. These mature cells are involved in immunity against foreign and infectious agents, as well as autoimmunity, whereby they produce autoantibodies against self constituents.

The BCR complex on mature cells is composed of membrane IgM and IgD molecules associated with the invariant Igα and Igβ heterodimers, which contain two immunoreceptor tyrosine-based activation motifs (ITAM) in their cytoplasmic tails. Mature BCR bearing B cells seed the peripheral blood and recirculate through the primary lymphoid tissues, such as the lymph nodes, spleen, and mucosal lymphoid tissues. Cross-linking of membrane Ig by multivalent antigen triggers clustering of the Igα and Igβ heterodimers and leads to tyrosine phosphorylation of the ITAMs by the SRC-family protein tyrosine kinases (PTKs), such as Lyn, Fyn, Blk, and Lck. Since the BCR complex lacks intrinsic kinase activity and is believed excluded from lipid rafts in the membrane, oligomerized BCR are translocated to lipid rafts, where Lyn resides constitutively to mediate tyrosine phosphorylation of the ITAM domains. This BCR signaling process is dependent on a receptor-inducible assembly mechanism, associated with the recruitment of PTKs, adaptors or linker proteins, and effector enzymes to the cytoplasmic side of the plasma membrane. The linker proteins, such as BLNK, BCAP, GAB, PAG, and LAT help localize enzymatic complexes to the appropriate subcellular site for signaling. These linker proteins link cell surface receptors with effector enzymes and help modulate signal transduction by mediating protein-protein or protein-lipid interactions.

The stimulation of B cells with anti-CD40 can mimic B cell activation via BCR. CD40 ligation has been shown to induce B cell growth, survival, differentiation, Ig switching, germinal center formation, and enhancement of antigen presentation by B cells. CD40 ligation not only enhances the expression of PIM-1, a protooncogene that encodes a serine/threonine protein kinase, via NF-κB activation, but stimulates JNK, p38 kinases, and protein kinase C independent activation of ERK2, similar to stimulation ofB cells with anti-IgM. CD40 ligation also induces phosphorylation of tyrosine kinases Lyn, Fyn, and Syk. The combination of IL-4 and anti-CD40 stimulation leads to enhanced B cell proliferation and Ig secretion. Therefore, a DNA microarray experiment comparing differential expression of RNA from anti-CD40 and IL-4 stimulated vs resting B cells, can reveal new genes associated with B cell activation. Gene products associated with B cell activation can be targets for therapeutic drug development in the treatment of autoimmune mediated inflammatory diseases and B cell malignancies, as well as provide insights into genes that are defective in immune deficiency disorders. Therapeutic molecules can be antibodies, peptides, or small molecules.

### Summary of the Invention

### A. Embodiments

The present invention concerns compositions and methods useful for the diagnosis and treatment of immune related disease in mammals, including humans. The present invention is based on the identification of proteins (including agonist and antagonist antibodies) which are a result of stimulation of the immune response in mammals. Immune related diseases can be treated by suppressing or enhancing the immune response. Molecules that enhance the immune response stimulate or potentiate the immune response to an antigen. Molecules which stimulate the immune response can be used therapeutically where enhancement of the immune response would be beneficial. Alternatively, molecules that suppress the immune response attenuate or reduce the immune response to an antigen (e.g., neutralizing antibodies) can be used therapeutically where attenuation of the immune response would be beneficial (e.g., inflammation). Accordingly, the PRO polypeptides, agonists and antagonists thereof are also useful to prepare medicines and medicaments for the treatment of immune-related and inflammatory diseases. In a specific aspect, such medicines and medicaments comprise a therapeutically effective amount of a PRO polypeptide, agonist or antagonist thereof with a pharmaceutically acceptable carrier. Preferably, the admixture is sterile.

In a further embodiment, the invention concerns a method of identifying agonists or antagonists to a PRO polypeptide which comprises contacting the PRO polypeptide with a candidate molecule and monitoring a biological activity mediated by said PRO polypeptide. Preferably, the PRO polypeptide is a native sequence PRO polypeptide. In a specific aspect, the PRO agonist or antagonist is an anti-PRO antibody.

In another embodiment, the invention concerns a composition of matter comprising a PRO polypeptide or an agonist or antagonist antibody which binds the polypeptide in admixture with a carrier or excipient. In one aspect, the composition comprises a therapeutically effective amount of the polypeptide or antibody. In another aspect, when the composition comprises an immune stimulating molecule, the composition is useful for: (a) stimulating or enhancing an immune response in a mammal in need thereof, (b) increasing the proliferation of B-lymphocytes in a mammal in need thereof in response to an antigen, (c) increasing the Ig secretion of B-lymphocytes. In a further aspect, when the composition comprises an immune inhibiting molecule, the composition is useful for: (a) inhibiting or reducing an immune response in a mammal in need thereof, (b) decreasing the proliferation of B-lymphocytes or (c) decreasing the Ig secretion by B-lymphocytes in a mammal in need thereof in response to an antigen. In another aspect, the composition comprises a further active ingredient, which may, for example, be a further antibody or a cytotoxic or chemotherapeutic agent. Preferably, the composition is sterile.

In another embodiment, the invention concerns a method of treating an immune related disorder in a mammal in need thereof, comprising administering to the mammal an effective amount of a PRO polypeptide, an agonist thereof, or an antagonist thereto. In a preferred aspect, the immune related disorder is selected from the group consisting of: systemic lupus erythematosis, X-linked infantile hypogammaglobulinemia, polysaccaride antigen unresponsiveness, selective IgA deficiency, selective IgM deficiency, selective deficiency of IgG subclasses, immunodeficiency with hyper Ig-M, transient hypogammaglobulinemia of infancy, Burkitt's lymphoma, Intermediate lymphoma, follicular lymphoma, typeII hypersensitivity, rheumatoid arthritis, autoimmune mediated hemolytic anemia, myesthenia gravis, hypoadrenocorticism, glomerulonephritis and ankylosing spondylitis.

In another embodiment, the invention provides an antibody which specifically binds to any of the above or below described polypeptides. Optionally, the antibody is a monoclonal antibody, humanized antibody, antibody fragment or single-chain antibody. In one aspect, the present invention concerns an isolated antibody which binds a PRO polypeptide. In another aspect, the antibody mimics the activity of a PRO polypeptide (an agonist antibody) or conversely the antibody inhibits or neutralizes the activity of a PRO polypeptide (an antagonist antibody). In another aspect, the antibody is a monoclonal antibody, which preferably has nonhuman complementarity determining region (CDR) residues and human framework region (FR) residues. The antibody may be labeled and may be immobilized on a solid support. In a further aspect, the antibody is an antibody fragment, a monoclonal antibody, a single-chain antibody, or an anti-idiotypic antibody.

In yet another embodiment, the present invention provides a composition comprising an anti-PRO antibody in admixture with a pharmaceutically acceptable carrier. In one aspect, the composition comprises a therapeutically effective amount of the antibody. Preferably, the composition is sterile. The composition may be administered in the form of a liquid pharmaceutical formulation, which may be preserved to achieve extended storage stability. Alternatively, the antibody is a monoclonal antibody, an antibody fragment, a humanized antibody, or a single-chain antibody.

In a further embodiment, the invention concerns an article of manufacture, comprising:
(a) a composition of matter comprising a PRO polypeptide or agonist or antagonist thereof;
(b) a container containing said composition; and
(c) a label affixed to said container, or a package insert included in said container referring to the use of said PRO polypeptide or agonist or antagonist thereof in the treatment of an immune related disease. The composition may comprise a therapeutically effective amount of the PRO polypeptide or the agonist or antagonist thereof.

In yet another embodiment, the present invention concerns a method of diagnosing an immune related disease in a mammal, comprising detecting the level of expression of a gene encoding a PRO polypeptide (a) in a test sample of tissue cells obtained from the mammal, and (b) in a control sample of known normal tissue cells of the same cell type, wherein a higher or lower expression level in the test sample as compared to the control sample indicates the presence of immune related disease in the mammal from which the test tissue cells were obtained.

In another embodiment, the present invention concerns a method of diagnosing an immune disease in a mammal, comprising (a) contacting an anti-PRO antibody with a test sample of tissue cells obtained from the mammal, and (b) detecting the formation of a complex between the antibody and a PRO polypeptide, in the test sample; wherein the formation of said complex is indicative of the presence or absence of said disease. The detection may be qualitative or quantitative, and may be performed in comparison with monitoring the complex formation in a control sample of known normal tissue cells of the same cell type. A larger quantity of complexes formed in the test sample indicates the presence or absence of an immune disease in the mammal from which the test tissue cells were obtained. The antibody preferably carries a detectable label. Complex formation can be monitored, for example, by light microscopy, flow cytometry, fluorimetry, or other techniques known in the art. The test sample is usually obtained from an individual suspected of having a deficiency or abnormality of the immune system.

In another embodiment, the invention provides a method for determining the presence of a PRO polypeptide in a sample comprising exposing a test sample of cells suspected of containing the PRO polypeptide to an anti-PRO antibody and determining the binding of said antibody to said cell sample. In a specific aspect, the sample comprises a cell suspected of containing the PRO polypeptide and the antibody binds to the cell. The antibody is preferably detectably labeled and/or bound to a solid support.

In another embodiment, the present invention concerns an immune-related disease diagnostic kit, comprising an anti-PRO antibody and a carrier in suitable packaging. The kit preferably contains instructions for using the antibody to detect the presence of the PRO polypeptide. Preferably the carrier is pharmaceutically acceptable.

In another embodiment, the present invention concerns a diagnostic kit, containing an anti-PRO antibody in suitable packaging. The kit preferably contains instructions for using the antibody to detect the PRO polypeptide.

In another embodiment, the invention provides a method of diagnosing an immune-related disease in a mammal which comprises detecting the presence or absence or a PRO polypeptide in a test sample of tissue cells obtained from said mammal, wherein the presence or absence of the PRO polypeptide in said test sample is indicative of the presence of an immune-related disease in said mammal.

In another embodiment, the present invention concerns a method for identifying an agonist of a PRO polypeptide comprising:
(a) contacting cells and a test compound to be screened under conditions suitable for the induction of a cellular response normally induced by a PRO polypeptide; and
(b) determining the induction of said cellular response to determine if the test compound is an effective agonist, wherein the induction of said cellular response is indicative of said test compound being an effective agonist.

In another embodiment, the invention concerns a method for identifying a compound capable of inhibiting the activity of a PRO polypeptide comprising contacting a candidate compound with a PRO polypeptide under conditions and for a time sufficient to allow these two components to interact and determining whether the activity of the PRO polypeptide is inhibited. In a specific aspect, either the candidate compound or the PRO polypeptide is immobilized on a solid support. In another aspect, the non-immobilized component carries a detectable label. In a preferred aspect, this method comprises the steps of:
(a) contacting cells and a test compound to be screened in the presence of a PRO polypeptide under conditions suitable for the induction of a cellular response normally induced by a PRO polypeptide; and
(b) determining the induction of said cellular response to determine if the test compound is an effective antagonist.

In another embodiment, the invention provides a method for identifying a compound that inhibits the expression of a PRO polypeptide in cells that normally express the polypeptide, wherein the method comprises contacting the cells with a test compound and determining whether the expression of the PRO polypeptide is inhibited. In a preferred aspect, this method comprises the steps of:
(a) contacting cells and a test compound to be screened under conditions suitable for allowing expression of the PRO polypeptide; and
(b) determining the inhibition of expression of said polypeptide.

In yet another embodiment, the present invention concerns a method for treating an immune-related disorder in a mammal that suffers therefrom comprising administering to the mammal a nucleic acid molecule that codes for either (a) a PRO polypeptide, (b) an agonist of a PRO polypeptide or (c) an antagonist of a PRO polypeptide, wherein said agonist or antagonist may be an anti-PRO antibody. In a preferred embodiment, the mammal is human. In another preferred embodiment, the nucleic acid is administered via ex vivo gene therapy. In a further preferred embodiment, the nucleic acid is comprised within a vector, more preferably an adenoviral, adeno-associated viral, lentiviral or retroviral vector.

In yet another aspect, the invention provides a recombinant viral particle comprising a viral vector consisting essentially of a promoter, nucleic acid encoding (a) a PRO polypeptide, (b) an agonist polypeptide of a PRO polypeptide, or (c) an antagonist polypeptide of a PRO polypeptide, and a signal sequence for cellular secretion of the polypeptide, wherein the viral vector is in association with viral structural proteins. Preferably, the signal sequence is from a mammal, such as from a native PRO polypeptide.

In a still further embodiment, the invention concerns an ex vivo producer cell comprising a nucleic acid construct that expresses retroviral structural proteins and also comprises a retroviral vector consisting essentially of a promoter, nucleic acid encoding (a) a PRO polypeptide, (b) an agonist polypeptide of a PRO polypeptide or (c) an antagonist polypeptide of a PRO polypeptide, and a signal sequence for cellular secretion of the polypeptide, wherein said producer cell packages the retroviral vector in association with the structural proteins to produce recombinant retroviral particles.

In a still further embodiment, the invention provides a method of increasing the activity of B-lymphocytes in a mammal comprising administering to said mammal (a) a PRO polypeptide, (b) an agonist of a PRO polypeptide, or (c) an antagonist of a PRO polypeptide, wherein the activity of B-lymphocytes in the mammal is increased.

In a still further embodiment, the invention provides a method of decreasing the activity of B-lymphocytes in a mammal comprising administering to said mammal (a) a PRO polypeptide, (b) an agonist of a PRO polypeptide, or (c) an antagonist of a PRO polypeptide, wherein the activity of B-lymphocytes in the mammal is decreased.

In a still further embodiment, the invention provides a method of increasing the proliferation of B-lymphocytes in a mammal comprising administering to said mammal (a) a PRO polypeptide, (b) an agonist of a PRO polypeptide, or (c) an antagonist of a PRO polypeptide, wherein the proliferation of B-lymphocytes in the mammal is increased.

In a still further embodiment, the invention provides a method of decreasing the proliferation of B-lymphocytes in a mammal comprising administering to said mammal (a) a PRO polypeptide, (b) an agonist of a PRO polypeptide, or (c) an antagonist of a PRO polypeptide, wherein the proliferation of B-lymphocytes in the mammal is decreased.

### B. Additional Embodiments

In other embodiments of the present invention, the invention provides vectors comprising DNA encoding any of the herein described polypeptides. Host cell comprising any such vector are also provided. By way of example, the host cells may be CHO cells, *E. coli*, or yeast. A process for producing any of the herein described polypeptides is further provided and comprises culturing host cells under conditions suitable for expression of the desired polypeptide and recovering the desired polypeptide from the cell culture.

In other embodiments, the invention provides chimeric molecules comprising any of the herein described polypeptides fused to a heterologous polypeptide or amino acid sequence. Example of such chimeric molecules comprise any of the herein described polypeptides fused to an epitope tag sequence or a Fc region of an immunoglobulin.

In another embodiment, the invention provides an antibody which specifically binds to any of the above or below described polypeptides. Optionally, the antibody is a monoclonal antibody, humanized antibody, antibody fragment or single-chain antibody.

In yet other embodiments, the invention provides oligonucleotide probes useful for isolating genomic and cDNA nucleotide sequences or as antisense probes, wherein those probes may be derived from any of the above or below described nucleotide sequences.

In other embodiments, the invention provides an isolated nucleic acid molecule comprising a nucleotide sequence that encodes a PRO polypeptide.

In one aspect, the isolated nucleic acid molecule comprises a nucleotide sequence having at least about 80% nucleic acid sequence identity, alternatively at least about 8 1 % nucleic acid sequence identity, alternatively at least about 82% nucleic acid sequence identity, alternatively at least about 83% nucleic acid sequence identity, alternatively at least about 84% nucleic acid sequence identity, alternatively at least about 85% nucleic acid sequence identity, alternatively at least about 86% nucleic acid sequence identity, alternatively at least about 87% nucleic acid sequence identity, alternatively at least about 88% nucleic acid sequence identity, alternatively at least about 89% nucleic acid sequence identity, alternatively at least about 90% nucleic acid sequence identity, alternatively at least about 91% nucleic acid sequence identity, alternatively at least about 92% nucleic acid sequence identity, alternatively at least about 93% nucleic acid sequence identity, alternatively at least about 94% nucleic acid sequence identity, alternatively at least about 95% nucleic acid sequence identity, alternatively at least about 96% nucleic acid sequence identity, alternatively at least about 97% nucleic acid sequence identity, alternatively at least about 98% nucleic acid sequence identity and alternatively at least about 99% nucleic acid sequence identity to (a) a DNA molecule encoding a PRO polypeptide having a full-length amino acid sequence as disclosed herein, an amino acid sequence lacking the signal peptide as disclosed herein, an extracellular domain of a transmembrane protein, with or without the signal peptide, as disclosed herein or any other specifically defined fragment of the full-length amino acid sequence as disclosed herein, or (b) the complement of the DNA molecule of (a).

In other aspects, the isolated nucleic acid molecule comprises a nucleotide sequence having at least about 80% nucleic acid sequence identity, alternatively at least about 81 % nucleic acid sequence identity, alternatively at least about 82% nucleic acid sequence identity, alternatively at least about 83% nucleic acid sequence identity, alternatively at least about 84% nucleic acid sequence identity, alternatively at least about 85% nucleic acid sequence identity, alternatively at least about 86% nucleic acid sequence identity, alternatively at least about 87% nucleic acid sequence identity, alternatively at least about 88% nucleic acid sequence identity, alternatively at least about 89% nucleic acid sequence identity, alternatively at least about 90% nucleic acid sequence identity, alternatively at least about 91 % nucleic acid sequence identity, alternatively at least about 92% nucleic acid sequence identity, alternatively at least about 93% nucleic acid sequence identity, alternatively at least about 94% nucleic acid sequence identity, alternatively at least about 95% nucleic acid sequence identity, alternatively at least about 96% nucleic acid sequence identity, alternatively at least about 97% nucleic acid sequence identity, alternatively at least about 98% nucleic acid sequence identity and alternatively at least about 99% nucleic acid sequence identity to (a) a DNA molecule comprising the coding sequence of a full-length PRO polypeptide cDNA as disclosed herein, the coding sequence of a PRO polypeptide lacking the signal peptide as disclosed herein, the coding sequence of an extracellular domain of a transmembrane PRO polypeptide, with or without the signal peptide, as disclosed herein or the coding sequence of any other specifically defined fragment of the full-length amino acid sequence as disclosed herein, or (b) the complement of the DNA molecule of (a).

In a further aspect, the invention concerns an isolated nucleic acid molecule comprising a nucleotide sequence having at least about 80% nucleic acid sequence identity, alternatively at least about 81 % nucleic acid sequence identity, alternatively at least about 82% nucleic acid sequence identity, alternatively at least about 83% nucleic acid sequence identity, alternatively at least about 84% nucleic acid sequence identity, alternatively at least about 85% nucleic acid sequence identity, alternatively at least about 86% nucleic acid sequence identity, alternatively at least about 87% nucleic acid sequence identity, alternatively at least about 88% nucleic acid sequence identity, alternatively at least about 89% nucleic acid sequence identity, alternatively at least about 90% nucleic acid sequence identity, alternatively at least about 91% nucleic acid sequence identity, alternatively at least about 92% nucleic acid sequence identity, alternatively at least about 93% nucleic acid sequence identity, alternatively at least about 94% nucleic acid sequence identity, alternatively at least about 95% nucleic acid sequence identity, alternatively at least about 96% nucleic acid sequence identity, alternatively at least about 97% nucleic acid sequence identity, alternatively at least about 98% nucleic acid sequence identity and alternatively at least about 99% nucleic acid sequence identity to (a) a DNA molecule that encodes the same mature polypeptide encoded by any of the human protein cDNAs deposited with the ATCC as disclosed herein, or (b) the complement of the DNA molecule of (a).

Another aspect the invention provides an isolated nucleic acid molecule comprising a nucleotide sequence encoding a PRO polypeptide which is either transmembrane domain-deleted or transmembrane domain-inactivated, or is complementary to such encoding nucleotide sequence, wherein the transmembrane domain(s) of such polypeptide are disclosed herein. Therefore, soluble extracellular domains of the herein described PRO polypeptides are contemplated.

Another embodiment is directed to fragments of a PRO polypeptide coding sequence, or the complement thereof, that may find use as, for example, hybridization probes, for encoding fragments of a PRO polypeptide that may optionally encode a polypeptide comprising a binding site for an anti-PRO antibody or as antisense oligonucleotide probes. Such nucleic acid fragments are usually at least about 20 nucleotides in length, alternatively at least about 30 nucleotides in length, alternatively at least about 40 nucleotides in length, alternatively at least about 50 nucleotides in length, alternatively at least about 60 nucleotides in length, alternatively at least about 70 nucleotides in length, alternatively at least about 80 nucleotides in length, alternatively at least about 90 nucleotides in length, alternatively at least about 100 nucleotides in length, alternatively at least about 110 nucleotides in length, alternatively at least about 120 nucleotides in length, alternatively at least about 130 nucleotides in length, alternatively at least about 140 nucleotides in length, alternatively at least about 150 nucleotides in length, alternatively at least about 160 nucleotides in length, alternatively at least about 170 nucleotides in length, alternatively at least about 180 nucleotides in length, alternatively at least about 190 nucleotides in length, alternatively at least about 200 nucleotides in length, alternatively at least about 250 nucleotides in length, alternatively at least about 300 nucleotides in length, alternatively at least about 350 nucleotides in length, alternatively at least about 400 nucleotides in length, alternatively at least about 450 nucleotides in length, alternatively at least about 500 nucleotides in length, alternatively at least about 600 nucleotides in length, alternatively at least about 700 nucleotides in length, alternatively at least about 800 nucleotides in length, alternatively at least about 900 nucleotides in length and alternatively at least about 1000 nucleotides in length, wherein in this context the term "about" means the referenced nucleotide sequence length plus or minus 10% of that referenced length. It is noted that novel fragments of a PRO polypeptide-encoding nucleotide sequence may be determined in a routine manner by aligning the PRO polypeptide-encoding nucleotide sequence with other known nucleotide sequences using any of a number of well known sequence alignment programs and determining which PRO polypeptide-encoding nucleotide sequence fragment(s) are novel. All of such PRO polypeptide-encoding nucleotide sequences are contemplated herein. Also contemplated are the PRO polypeptide fragments encoded by these nucleotide molecule fragments, preferably those PRO polypeptide fragments that comprise a binding site for an anti-PRO antibody.

In another embodiment, the invention provides isolated PRO polypeptide encoded by any of the isolated nucleic acid sequences herein above identified.

In a certain aspect, the invention concerns an isolated PRO polypeptide, comprising an amino acid sequence having at least about 80% amino acid sequence identity, alternatively at least about 81% amino acid sequence identity, alternatively at least about 82% amino acid sequence identity, alternatively at least about 83% amino acid sequence identity, alternatively at least about 84% amino acid sequence identity, alternatively at least about 85% amino acid sequence identity, alternatively at least about 86% amino acid sequence identity, alternatively at least about 87% amino acid sequence identity, alternatively at least about 88% amino acid sequence identity, alternatively at least about 89% amino acid sequence identity, alternatively at least about 90% amino acid sequence identity, alternatively at least about 91% amino acid sequence identity, alternatively at least about 92% amino acid sequence identity, alternatively at least about 93% amino acid sequence identity, alternatively at least about 94% amino acid sequence identity, alternatively at least about 95% amino acid sequence identity, alternatively at least about 96% amino acid sequence identity, alternatively at least about 97% amino acid sequence identity, alternatively at least about 98% amino acid sequence identity and alternatively at least about 99% amino acid sequence identity to a PRO polypeptide having a full-length amino acid sequence as disclosed herein, an amino acid sequence lacking the signal peptide as disclosed herein, an extracellular domain of a transmembrane protein, with or without the signal peptide, as disclosed herein or any other specifically defined fragment of the full-length amino acid sequence as disclosed herein.

In a further aspect, the invention concerns an isolated PRO polypeptide comprising an amino acid sequence having at least about 80% amino acid sequence identity, alternatively at least about 81 % amino acid sequence identity, alternatively at least about 82% amino acid sequence identity, alternatively at least about 83% amino acid sequence identity, alternatively at least about 84% amino acid sequence identity, alternatively at least about 85% amino acid sequence identity, alternatively at least about 86% amino acid sequence identity, alternatively at least about 87% amino acid sequence identity, alternatively at least about 88% amino acid sequence identity, alternatively at least about 89% amino acid sequence identity, alternatively at least about 90% amino acid sequence identity, alternatively at least about 91% amino acid sequence identity, alternatively at least about 92% amino acid sequence identity, alternatively at least about 93% amino acid sequence identity, alternatively at least about 94% amino acid sequence identity, alternatively at least about 95% amino acid sequence identity, alternatively at least about 96% amino acid sequence identity, alternatively at least about 97% amino acid sequence identity, alternatively at least about 98% amino acid sequence identity and alternatively at least about 99% amino acid sequence identity to an amino acid sequence encoded by any of the human protein cDNAs deposited with the ATCC as disclosed herein.

In a specific aspect, the invention provides an isolated PRO polypeptide without the N-terminal signal sequence and/or the initiating methionine and is encoded by a nucleotide sequence that encodes such an amino acid sequence as herein before described. Processes for producing the same are also herein described, wherein those processes comprise culturing a host cell comprising a vector which comprises the appropriate encoding nucleic acid molecule under conditions suitable for expression of the PRO polypeptide and recovering the PRO polypeptide from the cell culture.

Another aspect the invention provides an isolated PRO polypeptide which is either transmembrane domain-deleted or transmembrane domain-inactivated. Processes for producing the same are also herein described, wherein those processes comprise culturing a host cell comprising a vector which comprises the appropriate encoding nucleic acid molecule under conditions suitable for expression of the PRO polypeptide and recovering the PRO polypeptide from the cell culture.

In yet another embodiment, the invention concerns agonists and antagonists of a native PRO polypeptide as defined herein. In a particular embodiment, the agonist or antagonist is an anti-PRO antibody or a small molecule.

In a further embodiment, the invention concerns a method of identifying agonists or antagonists to a PRO polypeptide which comprise contacting the PRO polypeptide with a candidate molecule and monitoring a biological activity mediated by said PRO polypeptide. Preferably, the PRO polypeptide is a native PRO polypeptide.

In a still further embodiment, the invention concerns a composition of matter comprising a PRO polypeptide, or an agonist or antagonist of a PRO polypeptide as herein described, or an anti-PRO antibody, in combination with a carrier. Optionally, the carrier is a pharmaceutically acceptable carrier.

Another embodiment of the present invention is directed to the use of a PRO polypeptide, or an agonist or antagonist thereof as herein before described, or an anti-PRO antibody, for the preparation of a medicament useful in the treatment of a condition which is responsive to the PRO polypeptide, an agonist or antagonist thereof or an anti-PRO antibody.

### BRIEF DESCRIPTION OF THE DRAWINGS

Figure 1 shows a nucleotide sequence (SEQ ID NO:1) of a native sequence PRO71061 cDNA, wherein SEQ ID NO:1 is a clone designated herein as "DNA304494".
Figure 2 shows the amino acid sequence (SEQ ID NO:2) derived from the coding sequence of SEQ ID NO:1 shown in Figure 1.
Figure 3 shows a nucleotide sequence (SEQ ID NO:3) of a native sequence PRO 1265 cDNA, wherein SEQ ID NO:3 is a clone designated herein as "DNA304827".
Figure 4 shows the amino acid sequence (SEQ ID NO:4) derived from the coding sequence of SEQ ID NO:3 shown in Figure 3.
Figure 5 shows a nucleotide sequence (SEQ ID NO:5) of a native sequence PRO6013 cDNA, wherein SEQ ID NO:5 is a clone designated herein as "DNA304828".
Figure 6 shows the amino acid sequence (SEQ ID NO:6) derived from the coding sequence of SEQ ID NO:5 shown in Figure 5.
Figure 7A-B shows a nucleotide sequence (SEQ ID NO:7) of a native sequence PRO71042 cDNA, wherein SEQ ID NO:7 is a clone designated herein as "DNA304464".
Figure 8 shows the amino acid sequence (SEQ ID NO:8) derived from the coding sequence of SEQ ID NO:7 shown in Figure 7A-B.
Figure 9 shows a nucleotide sequence (SEQ ID NO:9) of a native sequence PRO71236 cDNA, wherein SEQ ID NO:9 is a clone designated herein as "DNA304829".
Figure 10 shows the amino acid sequence (SEQ ID NO:10) derived from the coding sequence of SEQ ID NO:9 shown in Figure 9.
Figure 11 shows a nucleotide sequence (SEQ ID NO:11) of a native sequence PRO3813 cDNA, wherein SEQ ID NO:11 is a clone designated herein as "DNA 196579".
Figure 12 shows the amino acid sequence (SEQ ID NO:12) derived from the coding sequence of SEQ ID NO:11 shown in Figure 11.
Figure 13 shows a nucleotide sequence (SEQ ID NO:13) of a native sequence PRO71237 cDNA, wherein SEQ ID NO: 13 is a clone designated herein as "DNA304830".
Figure 14 shows the amino acid sequence (SEQ ID NO: 14) derived from the coding sequence of SEQ ID NO:14 shown in Figure 14.
Figure 15 shows a nucleotide sequence (SEQ ID NO:15) of a native sequence PRO38838 cDNA, wherein SEQ ID NO:15 is a clone designated herein as "DNA233283".
Figure 16 shows the amino acid sequence (SEQ ID NO:16) derived from the coding sequence of SEQ ID NO:1 5 shown in Figure 15.
Figure 17 shows a nucleotide sequence (SEQ ID NO:17) of a native sequence PRO71238 cDNA, wherein SEQ ID NO:17 is a clone designated herein as "DNA304831".
Figure 18 shows the amino acid sequence (SEQ ID NO:18) derived from the coding sequence of SEQ ID NO:17 shown in Figure 17.
Figure 19 shows a nucleotide sequence (SEQ ID NO:19) of a native sequence PRO71239 cDNA, wherein SEQ ID NO:19 is a clone designated herein as "DNA304832".
Figure 20 shows the amino acid sequence (SEQ ID NO:20) derived from the coding sequence of SEQ ID NO:19 shown in Figure 19.
Figure 21 shows a nucleotide sequence (SEQ ID NO:21) of a native sequence PRO71240 cDNA, wherein SEQ ID NO:21 is a clone designated herein as "DNA304833".
Figure 22 shows the amino acid sequence (SEQ ID NO:22) derived from the coding sequence of SEQ ID NO:21 shown in Figure 21.
Figure 23A-B shows a nucleotide sequence (SEQ ID NO:23) of a native sequence PRO71241 cDNA, wherein SEQ ID NO:23 is a clone designated herein as "DNA304834".
Figure 24 shows the amino acid sequence (SEQ ID NO:24) derived from the coding sequence of SEQ ID NO:23 shown in Figure 23A-B.
Figure 25 shows a nucleotide sequence (SEQ ID NO:25) of a native sequence PRO71242 cDNA, wherein SEQ ID NO:25 is a clone designated herein as "DNA304835".
Figure 26 shows the amino acid sequence (SEQ ID NO:26) derived from the coding sequence of SEQ ID NO:25 shown in Figure 25.
Figure 27 shows a nucleotide sequence (SEQ ID NO:27) of a native sequence PRO71044 cDNA, wherein SEQ ID NO:27 is a clone designated herein as "DNA304468".
Figure 28 shows the amino acid sequence (SEQ ID NO:28) derived from the coding sequence of SEQ ID NO:27 shown in Figure 27.

### DETAILED DESCRIPTION OF THE PREFERRED EMBODIMENTS

### I. Definitions

The terms "PRO polypeptide" and "PRO" as used herein and when immediately followed by a numerical designation refer to various polypeptides, wherein the complete designation (i.e., PRO/number) refers to specific polypeptide sequences as described herein. The terms "PRO/number polypeptide" and "PRO/number" wherein the term "number" is provided as an actual numerical designation as used herein encompass native sequence polypeptides and polypeptide variants (which are further defined herein). The PRO polypeptides described herein may be isolated from a variety of sources, such as from human tissue types or from another source, or prepared by recombinant or synthetic methods. The term "PRO polypeptide" refers to each individual PRO/number polypeptide disclosed herein. All disclosures in this specification which refer to the "PRO polypeptide" refer to each of the polypeptides individually as well as jointly. For example, descriptions of the preparation of, purification of, derivation of, formation of antibodies to or against, administration of, compositions containing, treatment of a disease with, etc., pertain to each polypeptide of the invention individually. The term "PRO polypeptide" also includes variants of the PRO/number polypeptides disclosed herein.

A "native sequence PRO polypeptide" comprises a polypeptide having the same amino acid sequence as the corresponding PRO polypeptide derived from nature. Such native sequence PRO polypeptides can be isolated from nature or can be produced by recombinant or synthetic means. The term "native sequence PRO polypeptide" specifically encompasses naturally-occurring truncated or secreted forms of the specific PRO polypeptide (e.g., an extracellular domain sequence), naturally-occurring variant forms (*e.g*., alternatively spliced forms) and naturally-occurring allelic variants of the polypeptide. In various embodiments of the invention, the native sequence PRO polypeptides disclosed herein are mature or full-length native sequence polypeptides comprising the full-length amino acids sequences shown in the accompanying figures. Start and stop codons are shown in bold font and underlined in the figures. However, while the PRO polypeptide disclosed in the accompanying figures are shown to begin with methionine residues designated herein as amino acid position 1 in the figures, it is conceivable and possible that other methionine residues located either upstream or downstream from the amino acid position 1 in the figures may be employed as the starting amino acid residue for the PRO polypeptides.

The PRO polypeptide "extracellular domain" or "ECD" refers to a form of the PRO polypeptide which is essentially free of the transmembrane and cytoplasmic domains. Ordinarily, a PRO polypeptide ECD will have less than 1% of such transmembrane and/or cytoplasmic domains and preferably, will have less than 0.5% of such domains. It will be understood that any transmembrane domains identified for the PRO polypeptides of the present invention are identified pursuant to criteria routinely employed in the art for identifying that type of hydrophobic domain. The exact boundaries of a transmembrane domain may vary but most likely by no more than about 5 amino acids at either end of the domain as initially identified herein. Optionally, therefore, an extracellular domain of a PRO polypeptide may contain from about 5 or fewer amino acids on either side of the transmembrane domain/extracellular domain boundary as identified in the Examples or specification and such polypeptides, with or without the associated signal peptide, and nucleic acid encoding them, are contemplated by the present invention.

The approximate location of the "signal peptides" of the various PRO polypeptides disclosed herein are shown in the present specification and/or the accompanying figures. It is noted, however, that the C-terminal boundary of a signal peptide may vary, but most likely by no more than about 5 amino acids on either side of the signal peptide C-terminal boundary as initially identified herein, wherein the C-terminal boundary of the signal peptide may be identified pursuant to criteria routinely employed in the art for identifying that type of amino acid sequence element (e.g., Nielsen et al., Prot. Eng. 10:1-6 (1997) and von Heinje et al., Nucl. Acids. Res. 14:4683-4690 (1986)). Moreover, it is also recognized that, in some cases, cleavage of a signal sequence from a secreted polypeptide is not entirely uniform, resulting in more than one secreted species. These mature polypeptides, where the signal peptide is cleaved within no more than about 5 amino acids on either side of the C-terminal boundary of the signal peptide as identified herein, and the polynucleotides encoding them, are contemplated by the present invention.

"PRO polypeptide variant" means an active PRO polypeptide as defined above or below having at least about 80% amino acid sequence identity with a full-length native sequence PRO polypeptide sequence as disclosed herein, a PRO polypeptide sequence lacking the signal peptide as disclosed herein, an extracellular domain of a PRO polypeptide, with or without the signal peptide, as disclosed herein or any other fragment of a full-length PRO polypeptide sequence as disclosed herein. Such PRO polypeptide variants include, for instance, PRO polypeptides wherein one or more amino acid residues are added, or deleted, at the N- or C-terminus of the full-length native amino acid sequence. Ordinarily, a PRO polypeptide variant will have at least about 80% amino acid sequence identity, alternatively at least about 81% amino acid sequence identity, alternatively at least about 82% amino acid sequence identity, alternatively at least about 83% amino acid sequence identity, alternatively at least about 84% amino acid sequence identity, alternatively at least about 85% amino acid sequence identity, alternatively at least about 86% amino acid sequence identity, alternatively at least about 87% amino acid sequence identity, alternatively at least about 88% amino acid sequence identity, alternatively at least about 89% amino acid sequence identity, alternatively at least about 90% amino acid sequence identity, alternatively at least about 91 % amino acid sequence identity, alternatively at least about 92% amino acid sequence identity, alternatively at least about 93% amino acid sequence identity, alternatively at least about 94% amino acid sequence identity, alternatively at least about 95% amino acid sequence identity, alternatively at least about 96% amino acid sequence identity, alternatively at least about 97% amino acid sequence identity, alternatively at least about 98% amino acid sequence identity and alternatively at least about 99% amino acid sequence identity to a full-length native sequence PRO polypeptide sequence as disclosed herein, a PRO polypeptide sequence lacking the signal peptide as disclosed herein, an extracellular domain of a PRO polypeptide, with or without the signal peptide, as disclosed herein or any other specifically defined fragment of a full-length PRO polypeptide sequence as disclosed herein. Ordinarily, PRO variant polypeptides are at least about 10 amino acids in length, alternatively at least about 20 amino acids in length, alternatively at least about 30 amino acids in length, alternatively at least about 40 amino acids in length, alternatively at least about 50 amino acids in length, alternatively at least about 60 amino acids in length, alternatively at least about 70 amino acids in length, alternatively at least about 80 amino acids in length, alternatively at least about 90 amino acids in length, alternatively at least about 100 amino acids in length, alternatively at least about 150 amino acids in length, alternatively at least about 200 amino acids in length, alternatively at least about 300 amino acids in length, or more.

"Percent (%) amino acid sequence identity" with respect to the PRO polypeptide sequences identified herein is defined as the percentage of amino acid residues in a candidate sequence that are identical with the amino acid residues in the specific PRO polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN or Megalign (DNASTAR) software. Those skilled in the art can determine appropriate parameters for measuring alignment, including any algorithms needed to achieve maximal alignment over the full length of the sequences being compared. For purposes herein, however, % amino acid sequence identity values are generated using the sequence comparison computer program ALIGN-2, wherein the complete source code for the ALIGN-2 program is provided in Table 1 below. The ALIGN-2 sequence comparison computer program was authored by Genentech, Inc. and the source code shown in Table 1 below has been filed with user documentation in the U.S. Copyright Office, Washington D.C., 20559, where it is registered under U.S. Copyright Registration No. TXU510087. The ALIGN-2 program is publicly available through Genentech, Inc., South San Francisco, California or may be compiled from the source code provided in Table 1 below. The ALIGN-2 program should be compiled for use on a UNIX operating system, preferably digital UNIX V4.0D. All sequence comparison parameters are set by the ALIGN-2 program and do not vary.

In situations where ALIGN-2 is employed for amino acid sequence comparisons, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows: $100 times the fraction X / Y$
where X is the number of amino acid residues scored as identical matches by the sequence alignment program ALIGN-2 in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity ofB to A. As examples of % amino acid sequence identity calculations using this method, Tables 2 and 3 demonstrate how to calculate the % amino acid sequence identity of the amino acid sequence designated "Comparison Protein" to the amino acid sequence designated "PRO", wherein "PRO" represents the amino acid sequence of a hypothetical PRO polypeptide of interest, "Comparison Protein" represents the amino acid sequence of a polypeptide against which the "PRO" polypeptide of interest is being compared, and "X, "Y" and "Z" each represent different hypothetical amino acid residues.

Unless specifically stated otherwise, all % amino acid sequence identity values used herein are obtained as described in the immediately preceding paragraph using the ALIGN-2 computer program. However, % amino acid sequence identity values may also be obtained as described below by using the WU-BLAST-2 computer program (Altschul et al., Methods in Enzymology 266:460-480 (1996)). Most of the WU-BLAST-2 search parameters are set to the default values. Those not set to default values, i.e., the adjustable parameters, are set with the following values: overlap span = 1, overlap fraction = 0.125, word threshold (T) = 11, and scoring matrix = BLOSUM62. When WU-BLAST-2 is employed, a % amino acid sequence identity value is determined by dividing (a) the number of matching identical amino acid residues between the amino acid sequence of the PRO polypeptide of interest having a sequence derived from the native PRO polypeptide and the comparison amino acid sequence of interest (i.e., the sequence against which the PRO polypeptide of interest is being compared which may be a PRO variant polypeptide) as determined by WU-BLAST-2 by (b) the total number of amino acid residues of the PRO polypeptide of interest. For example, in the statement "a polypeptide comprising an the amino acid sequence A which has or having at least 80% amino acid sequence identity to the amino acid sequence B", the amino acid sequence A is the comparison amino acid sequence of interest and the amino acid sequence B is the amino acid sequence of the PRO polypeptide of interest.

Percent amino acid sequence identity may also be determined using the sequence comparison program NCBI-BLAST2 (Altschul et al., Nucleic Acids Res. 25:3389-3402 (1997)). The NCBI-BLAST2 sequence comparison program may be downloaded from http://www.ncbi.nlm.nih.gov or otherwise obtained from the National Institute of Health, Bethesda, MD. NCBI-BLAST2 uses several search parameters, wherein all of those search parameters are set to default values including, for example, unmask = yes, strand = all, expected occurrences = 10, minimum low complexity length = 15/5, multi-pass e-value = 0.01, constant for multi-pass = 25, dropoff for final gapped alignment = 25 and scoring matrix = BLOSUM62.

In situations where NCBI-BLAST2 is employed for amino acid sequence comparisons, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows: $100 times the fraction X / Y$
where X is the number of amino acid residues scored as identical matches by the sequence alignment program NCBI-BLAST2 in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A.

"PRO variant polynucleotide" or "PRO variant nucleic acid sequence" means a nucleic acid molecule which encodes an active PRO polypeptide as defined below and which has at least about 80% nucleic acid sequence identity with a nucleotide acid sequence encoding a full-length native sequence PRO polypeptide sequence as disclosed herein, a full-length native sequence PRO polypeptide sequence lacking the signal peptide as disclosed herein, an extracellular domain of a PRO polypeptide, with or without the signal peptide, as disclosed herein or any other fragment of a full-length PRO polypeptide sequence as disclosed herein. Ordinarily, a PRO variant polynucleotide will have at least about 80% nucleic acid sequence identity, alternatively at least about 81% nucleic acid sequence identity, alternatively at least about 82% nucleic acid sequence identity, alternatively at least about 83% nucleic acid sequence identity, alternatively at least about 84% nucleic acid sequence identity, alternatively at least about 85% nucleic acid sequence identity, alternatively at least about 86% nucleic acid sequence identity, alternatively at least about 87% nucleic acid sequence identity, alternatively at least about 88% nucleic acid sequence identity, alternatively at least about 89% nucleic acid sequence identity, alternatively at least about 90% nucleic acid sequence identity, alternatively at least about 91 % nucleic acid sequence identity, alternatively at least about 92% nucleic acid sequence identity, alternatively at least about 93% nucleic acid sequence identity, alternatively at least about 94% nucleic acid sequence identity, alternatively at least about 95% nucleic acid sequence identity, alternatively at least about 96% nucleic acid sequence identity, alternatively at least about 97% nucleic acid sequence identity, alternatively at least about 98% nucleic acid sequence identity and alternatively at least about 99% nucleic acid sequence identity with a nucleic acid sequence encoding a full-length native sequence PRO polypeptide sequence as disclosed herein, a full-length native sequence PRO polypeptide sequence lacking the signal peptide as disclosed herein, an extracellular domain of a PRO polypeptide, with or without the signal sequence, as disclosed herein or any other fragment of a full-length PRO polypeptide sequence as disclosed herein. Variants do not encompass the native nucleotide sequence.

Ordinarily, PRO variant polynucleotides are at least about 30 nucleotides in length, alternatively at least about 60 nucleotides in length, alternatively at least about 90 nucleotides in length, alternatively at least about 120 nucleotides in length, alternatively at least about 150 nucleotides in length, alternatively at least about 180 nucleotides in length, alternatively at least about 210 nucleotides in length, alternatively at least about 240 nucleotides in length, alternatively at least about 270 nucleotides in length, alternatively at least about 300 nucleotides in length, alternatively at least about 450 nucleotides in length, alternatively at least about 600 nucleotides in length, alternatively at least about 900 nucleotides in length, or more.

"Percent (%) nucleic acid sequence identity" with respect to PRO-encoding nucleic acid sequences identified herein is defined as the percentage of nucleotides in a candidate sequence that are identical with the nucleotides in the PRO nucleic acid sequence of interest, after aligning the sequences and introducing gaps, if necessary, to achieve the maximum percent sequence identity. Alignment for purposes of determining percent nucleic acid sequence identity can be achieved in various ways that are within the skill in the art, for instance, using publicly available computer software such as BLAST, BLAST-2, ALIGN or Megalign (DNASTAR) software. For purposes herein, however, % nucleic acid sequence identity values are generated using the sequence comparison computer program ALIGN-2, wherein the complete source code for the ALIGN-2 program is provided in Table 1 below. The ALIGN-2 sequence comparison computer program was authored by Genentech, Inc. and the source code shown in Table 1 below has been filed with user documentation in the U.S. Copyright Office, Washington D.C., 20559, where it is registered under U.S. Copyright Registration No. TXU510087. The ALIGN-2 program is publicly available through Genentech, Inc., South San Francisco, California or may be compiled from the source code provided in Table 1 below. The ALIGN-2 program should be compiled for use on a UNIX operating system, preferably digital UNIX V4.0D. All sequence comparison parameters are set by the ALIGN-2 program and do not vary.

In situations where ALIGN-2 is employed for nucleic acid sequence comparisons, the % nucleic acid sequence identity of a given nucleic acid sequence C to, with, or against a given nucleic acid sequence D (which can alternatively be phrased as a given nucleic acid sequence C that has or comprises a certain % nucleic acid sequence identity to, with, or against a given nucleic acid sequence D) is calculated as follows: $100 times the fraction W / Z$
where W is the number of nucleotides scored as identical matches by the sequence alignment program ALIGN-2 in that program's alignment of C and D, and where Z is the total number of nucleotides in D. It will be appreciated that where the length of nucleic acid sequence C is not equal to the length of nucleic acid sequence D, the % nucleic acid sequence identity of C to D will not equal the % nucleic acid sequence identity ofD to C. As examples of% nucleic acid sequence identity calculations, Tables 4 and 5, demonstrate how to calculate the % nucleic acid sequence identity of the nucleic acid sequence designated "Comparison DNA" to the nucleic acid sequence designated "PRO-DNA", wherein "PRO-DNA" represents a hypothetical PRO-encoding nucleic acid sequence of interest, "Comparison DNA" represents the nucleotide sequence of a nucleic acid molecule against which the "PRO-DNA" nucleic acid molecule of interest is being compared, and "N", "L" and "V" each represent different hypothetical nucleotides.

Unless specifically stated otherwise, all % nucleic acid sequence identity values used herein are obtained as described in the immediately preceding paragraph using the ALIGN-2 computer program. However, % nucleic acid sequence identity values may also be obtained as described below by using the WU-BLAST-2 computer program (Altschul et al., Methods in Enzymology 266:460-480 (1996)). Most of the WU-BLAST-2 search parameters are set to the default values. Those not set to default values, i.e., the adjustable parameters, are set with the following values: overlap span = 1, overlap fraction = 0.125, word threshold (T) = 11, and scoring matrix = BLOSUM62. When WU-BLAST-2 is employed, a % nucleic acid sequence identity value is determined by dividing (a) the number of matching identical nucleotides between the nucleic acid sequence of the PRO polypeptide-encoding nucleic acid molecule of interest having a sequence derived from the native sequence PRO polypeptide-encoding nucleic acid and the comparison nucleic acid molecule of interest (i.e., the sequence against which the PRO polypeptide-encoding nucleic acid molecule of interest is being compared which may be a variant PRO polynucleotide) as determined by WU-BLAST-2 by (b) the total number of nucleotides of the PRO polypeptide-encoding nucleic acid molecule of interest. For example, in the statement "an isolated nucleic acid molecule comprising a nucleic acid sequence A which has or having at least 80% nucleic acid sequence identity to the nucleic acid sequence B", the nucleic acid sequence A is the comparison nucleic acid molecule of interest and the nucleic acid sequence B is the nucleic acid sequence of the PRO polypeptide-encoding nucleic acid molecule of interest.

Percent nucleic acid sequence identity may also be determined using the sequence comparison program NCBI-BLAST2 (Altschul et al., Nucleic Acids Res. 25:3389-3402 (1997)). The NCBI-BLAST2 sequence comparison program may be downloaded from http://www.ncbi.nlm.nih.gov or otherwise obtained from the National Institute of Health, Bethesda, MD. NCBI-BLAST2 uses several search parameters, wherein all of those search parameters are set to default values including, for example, unmask = yes, strand = all, expected occurrences = 10, minimum low complexity length = 15/5, multi-pass e-value = 0.01, constant for multi-pass = 25, dropoff for final gapped alignment = 25 and scoring matrix = BLOSUM62.

In situations where NCBI-BLAST2 is employed for sequence comparisons, the % nucleic acid sequence identity of a given nucleic acid sequence C to, with, or against a given nucleic acid sequence D (which can alternatively be phrased as a given nucleic acid sequence C that has or comprises a certain % nucleic acid sequence identity to, with, or against a given nucleic acid sequence D) is calculated as follows: $100 times the fraction W / Z$
where W is the number of nucleotides scored as identical matches by the sequence alignment program NCBI-BLAST2 in that program's alignment of C and D, and where Z is the total number of nucleotides in D. It will be appreciated that where the length of nucleic acid sequence C is not equal to the length of nucleic acid sequence D, the % nucleic acid sequence identity of C to D will not equal the % nucleic acid sequence identity ofD to C.

In other embodiments, PRO variant polynucleotides are nucleic acid molecules that encode an active PRO polypeptide and which are capable of hybridizing, preferably under stringent hybridization and wash conditions, to nucleotide sequences encoding a full-length PRO polypeptide as disclosed herein. PRO variant polypeptides may be those that are encoded by a PRO variant polynucleotide.

"Isolated," when used to describe the various polypeptides disclosed herein, means polypeptide that has been identified and separated and/or recovered from a component of its natural environment. Contaminant components of its natural environment are materials that would typically interfere with diagnostic or therapeutic uses for the polypeptide, and may include enzymes, hormones, and other proteinaceous or non-proteinaceous solutes. In preferred embodiments, the polypeptide will be purified (1) to a degree sufficient to obtain at least 15 residues of N-terminal or internal amino acid sequence by use of a spinning cup sequenator, or (2) to homogeneity by SDS-PAGE under non-reducing or reducing conditions using Coomassie blue or, preferably, silver stain. Isolated polypeptide includes polypeptide *in situ* within recombinant cells, since at least one component of the PRO polypeptide natural environment will not be present. Ordinarily, however, isolated polypeptide will be prepared by at least one purification step.

An "isolated" PRO polypeptide-encoding nucleic acid or other polypeptide-encoding nucleic acid is a nucleic acid molecule that is identified and separated from at least one contaminant nucleic acid molecule with which it is ordinarily associated in the natural source of the polypeptide-encoding nucleic acid. An isolated polypeptide-encoding nucleic acid molecule is other than in the form or setting in which it is found in nature. Isolated polypeptide-encoding nucleic acid molecules therefore are distinguished from the specific polypeptide-encoding nucleic acid molecule as it exists in natural cells. However, an isolated polypeptide-encoding nucleic acid molecule includes polypeptide-encoding nucleic acid molecules contained in cells that ordinarily express the polypeptide where, for example, the nucleic acid molecule is in a chromosomal location different from that of natural cells.

The term "control sequences" refers to DNA sequences necessary for the expression of an operably linked coding sequence in a particular host organism. The control sequences that are suitable for prokaryotes, for example, include a promoter, optionally an operator sequence, and a ribosome binding site. Eukaryotic cells are known to utilize promoters, polyadenylation signals, and enhancers.

Nucleic acid is "operably linked" when it is placed into a functional relationship with another nucleic acid sequence. For example, DNA for a presequence or secretory leader is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous, and, in the case of a secretory leader, contiguous and in reading phase. However, enhancers do not have to be contiguous. Linking is accomplished by ligation at convenient restriction sites. If such sites do not exist, the synthetic oligonucleotide adaptors or linkers are used in accordance with conventional practice.

The term "antibody" is used in the broadest sense and specifically covers, for example, single anti-PRO monoclonal antibodies (including agonist, antagonist, and neutralizing antibodies), anti-PRO antibody compositions with polyepitopic specificity, single chain anti-PRO antibodies, and fragments of anti-PRO antibodies (see below). The term "monoclonal antibody" as used herein refers to an antibody obtained from a population of substantially homogeneous antibodies, i.e., the individual antibodies comprising the population are identical except for possible naturally-occurring mutations that may be present in minor amounts.

"Stringency" of hybridization reactions is readily determinable by one of ordinary skill in the art, and generally is an empirical calculation dependent upon probe length, washing temperature, and salt concentration. In general, longer probes require higher temperatures for proper annealing, while shorter probes need lower temperatures. Hybridization generally depends on the ability of denatured DNA to reanneal when complementary strands are present in an environment below their melting temperature. The higher the degree of desired homology between the probe and hybridizable sequence, the higher the relative temperature which can be used. As a result, it follows that higher relative temperatures would tend to make the reaction conditions more stringent, while lower temperatures less so. For additional details and explanation of stringency of hybridization reactions, see Ausubel et al., Current Protocols in Molecular Biology, Wiley Interscience Publishers, (1995).

"Stringent conditions" or "high stringency conditions", as defined herein, may be identified by those that: (1) employ low ionic strength and high temperature for washing, for example 0.015 M sodium chloride/0.0015 M sodium citrate/0.1 % sodium dodecyl sulfate at 50°C; (2) employ during hybridization a denaturing agent, such as formamide, for example, 50% (v/v) formamide with 0.1 % bovine serum albumin/0.1% Ficoll/0. 1% polyvinylpyrrolidone/50mM sodium phosphate buffer at pH 6.5 with 750 mM sodium chloride, 75 mM sodium citrate at 42°C; or (3) employ 50% formamide, 5 x SSC (0.75 M NaCl, 0.075 M sodium citrate), 50 mM sodium phosphate (pH 6.8), 0.1% sodium pyrophosphate, 5 x Denhardt's solution, sonicated salmon sperm DNA (50 µg/ml), 0.1% SDS, and 10% dextran sulfate at 42°C, with washes at 42°C in 0.2 x SSC (sodium chloride/sodium citrate) and 50% formamide at 55°C, followed by a high-stringency wash consisting of 0.1 x SSC containing EDTA at 55°C.

"Moderately stringent conditions" may be identified as described by Sambrook et al., Molecular Cloning: A Laboratory Manual, New York: Cold Spring Harbor Press, 1989, and include the use of washing solution and hybridization conditions (e.g., temperature, ionic strength and %SDS) less stringent that those described above. An example of moderately stringent conditions is overnight incubation at 37°C in a solution comprising: 20% formamide, 5 x SSC (150 mM NaCl, 15 mM trisodium citrate), 50 mM sodium phosphate (pH 7.6), 5 x Denhardt's solution, 10% dextran sulfate, and 20 mg/ml denatured sheared salmon sperm DNA, followed by washing the filters in 1 x SSC at about 37-50°C. The skilled artisan will recognize how to adjust the temperature, ionic strength, etc. as necessary to accommodate factors such as probe length and the like.

The term "epitope tagged" when used herein refers to a chimeric polypeptide comprising a PRO polypeptide fused to a "tag polypeptide". The tag polypeptide has enough residues to provide an epitope against which an antibody can be made, yet is short enough such that it does not interfere with activity of the polypeptide to which it is fused. The tag polypeptide preferably also is fairly unique so that the antibody does not substantially cross-react with other epitopes. Suitable tag polypeptides generally have at least six amino acid residues and usually between about 8 and 50 amino acid residues (preferably, between about 10 and 20 amino acid residues).

As used herein, the term "immunoadhesin" designates antibody-like molecules which combine the binding specificity of a heterologous protein (an "adhesin") with the effector functions of immunoglobulin constant domains. Structurally, the immunoadhesins comprise a fusion of an amino acid sequence with the desired binding specificity which is other than the antigen recognition and binding site of an antibody (i.e., is "heterologous"), and an immunoglobulin constant domain sequence. The adhesin part of an immunoadhesin molecule typically is a contiguous amino acid sequence comprising at least the binding site of a receptor or a ligand. The immunoglobulin constant domain sequence in the immunoadhesin may be obtained from any immunoglobulin, such as IgG-1, IgG-2, IgG-3, or IgG-4 subtypes, IgA (including IgA-1 and IgA-2), IgE, IgD or IgM.

"Active" or "activity" for the purposes herein refers to form(s) of a PRO polypeptide which retain a biological and/or an immunological activity of native or naturally-occurring PRO, wherein "biological" activity refers to a biological function (either inhibitory or stimulatory) caused by a native or naturally-occurring PRO other than the ability to induce the production of an antibody against an antigenic epitope possessed by a native or naturally-occurring PRO and an "immunological" activity refers to the ability to induce the production of an antibody against an antigenic epitope possessed by a native or naturally-occurring PRO.

The term "antagonist" is used in the broadest sense, and includes any molecule that partially or fully blocks, inhibits, or neutralizes a biological activity of a native PRO polypeptide disclosed herein. In a similar manner, the term "agonist" is used in the broadest sense and includes any molecule that mimics a biological activity of a native PRO polypeptide disclosed herein. Suitable agonist or antagonist molecules specifically include agonist or antagonist antibodies or antibody fragments, fragments or amino acid sequence variants of native PRO polypeptides, peptides, antisense oligonucleotides, small organic molecules, etc. Methods for identifying agonists or antagonists of a PRO polypeptide may comprise contacting a PRO polypeptide with a candidate agonist or antagonist molecule and measuring a detectable change in one or more biological activities normally associated with the PRO polypeptide.

"Treatment" refers to both therapeutic treatment and prophylactic or preventative measures, wherein the object is to prevent or slow down (lessen) the targeted pathologic condition or disorder. Those in need of treatment include those already with the disorder as well as those prone to have the disorder or those in whom the disorder is to be prevented.

"Chronic" administration refers to administration of the agent(s) in a continuous mode as opposed to an acute mode, so as to maintain the initial therapeutic effect (activity) for an extended period of time. "Intermittent" administration is treatment that is not consecutively done without interruption, but rather is cyclic in nature.

"Mammal" for purposes of treatment refers to any animal classified as a mammal, including humans, domestic and farm animals, and zoo, sports, or pet animals, such as dogs, cats, cattle, horses, sheep, pigs, goats, rabbits, etc. Preferably, the mammal is human.

Administration "in combination with" one or more further therapeutic agents includes simultaneous (concurrent) and consecutive administration in any order.

"Carriers" as used herein include pharmaceutically acceptable carriers, excipients, or stabilizers which are nontoxic to the cell or mammal being exposed thereto at the dosages and concentrations employed. Often the physiologically acceptable carrier is an aqueous pH buffered solution. Examples of physiologically acceptable carriers include buffers such as phosphate, citrate, and other organic acids; antioxidants including ascorbic acid; low molecular weight (less than about 10 residues) polypeptide; proteins, such as serum albumin, gelatin, or immunoglobulins; hydrophilic polymers such as polyvinylpyrrolidone; amino acids such as glycine, glutamine, asparagine, arginine or lysine; monosaccharides, disaccharides, and other carbohydrates including glucose, mannose, or dextrins; chelating agents such as EDTA; sugar alcohols such as mannitol or sorbitol; salt-forming counterions such as sodium; and/or nonionic surfactants such as TWEEN™, polyethylene glycol (PEG), and PLURONICS™.

"Antibody fragments" comprise a portion of an intact antibody, preferably the antigen binding or variable region of the intact antibody. Examples of antibody fragments include Fab, Fab', F(ab')₂, and Fv fragments; diabodies; linear antibodies (Zapata et al., Protein Eng. 8(10): 1057-1062 [1995]); single-chain antibody molecules; and multispecific antibodies formed from antibody fragments.

Papain digestion of antibodies produces two identical antigen-binding fragments, called "Fab" fragments, each with a single antigen-binding site, and a residual "Fc" fragment, a designation reflecting the ability to crystallize readily. Pepsin treatment yields an F(ab')₂ fragment that has two antigen-combining sites and is still capable of cross-linking antigen.

"Fv" is the minimum antibody fragment which contains a complete antigen-recognition and - binding site. This region consists of a dimer of one heavy- and one light-chain variable domain in tight, non-covalent association. It is in this configuration that the three CDRs of each variable domain interact to define an antigen-binding site on the surface of the V_{H}-V_{L} dimer. Collectively, the six CDRs confer antigen-binding specificity to the antibody. However, even a single variable domain (or half of an Fv comprising only three CDRs specific for an antigen) has the ability to recognize and bind antigen, although at a lower affinity than the entire binding site.

The Fab fragment also contains the constant domain of the light chain and the first constant domain (CH1) of the heavy chain. Fab fragments differ from Fab' fragments by the addition of a few residues at the carboxy terminus of the heavy chain CH 1 domain including one or more cysteines from the antibody hinge region. Fab'-SH is the designation herein for Fab' in which the cysteine residue(s) of the constant domains bear a free thiol group. F(ab')₂ antibody fragments originally were produced as pairs of Fab' fragments which have hinge cysteines between them. Other chemical couplings of antibody fragments are also known.

The "light chains" of antibodies (immunoglobulins) from any vertebrate species can be assigned to one of two clearly distinct types, called kappa and lambda, based on the amino acid sequences of their constant domains.

Depending on the amino acid sequence of the constant domain of their heavy chains, immunoglobulins can be assigned to different classes. There are five major classes of immunoglobulins: IgA, IgD, IgE, IgG, and IgM, and several of these maybe further divided into subclasses (isotypes), e.g., IgG1, IgG2, IgG3, IgG4, IgA, and IgA2.

"Single-chain Fv" or "sFv" antibody fragments comprise the V_{H} and V_{L} domains of antibody, wherein these domains are present in a single polypeptide chain. Preferably, the Fv polypeptide further comprises a polypeptide linker between the V_{H} and V_{L} domains which enables the sFv to form the desired structure for antigen binding. For a review of sFv, see Pluckthun in The Pharmacology of Monoclonal Antibodies, vol. 113, Rosenburg and Moore eds., Springer-Verlag, New York, pp. 269-315 (1994).

The term "diabodies" refers to small antibody fragments with two antigen-binding sites, which fragments comprise a heavy-chain variable domain (V_{H}) connected to a light-chain variable domain (V_{L}) in the same polypeptide chain (V_{H}-V_{L}). By using a linker that is too short to allow pairing between the two domains on the same chain, the domains are forced to pair with the complementary domains of another chain and create two antigen-binding sites. Diabodies are described more fully in, for example, EP 404,097; WO 93/11161; and Hollinger et al., Proc. Natl. Acad. Sci. USA, 90:6444-6448 (1993).

An "isolated" antibody is one which has been identified and separated and/or recovered from a component of its natural environment. Contaminant components of its natural environment are materials which would interfere with diagnostic or therapeutic uses for the antibody, and may include enzymes, hormones, and other proteinaceous or nonproteinaceous solutes. In preferred embodiments, the antibody will be purified (1) to greater than 95% by weight of antibody as determined by the Lowry method, and most preferably more than 99% by weight, (2) to a degree sufficient to obtain at least 15 residues of N-terminal or internal amino acid sequence by use of a spinning cup sequenator, or (3) to homogeneity by SDS-PAGE under reducing or nonreducing conditions using Coomassie blue or, preferably, silver stain. Isolated antibody includes the antibody in situ within recombinant cells since at least one component of the antibody's natural environment will not be present. Ordinarily, however, isolated antibody will be prepared by at least one purification step.

An antibody that "specifically binds to" or is "specific for" a particular polypeptide or an epitope on a particular polypeptide is one that binds to that particular polypeptide or epitope on a particular polypeptide without substantially binding to any other polypeptide or polypeptide epitope.

The word "label" when used herein refers to a detectable compound or composition which is conjugated directly or indirectly to the antibody so as to generate a "labeled" antibody. The label may be detectable by itself (e.g. radioisotope labels or fluorescent labels) or, in the case of an enzymatic label, may catalyze chemical alteration of a substrate compound or composition which is detectable.

By "solid phase" is meant a non-aqueous matrix to which the antibody of the present invention can adhere. Examples of solid phases encompassed herein include those formed partially or entirely of glass (e.g., controlled pore glass), polysaccharides (e.g., agarose), polyacrylamides, polystyrene, polyvinyl alcohol and silicones. In certain embodiments, depending on the context, the solid phase can comprise the well of an assay plate; in others it is a purification column (e.g., an affinity chromatography column). This term also includes a discontinuous solid phase of discrete particles, such as those described in U.S. Patent No. 4,275,149.

A "liposome" is a small vesicle composed of various types of lipids, phospholipids and/or surfactant which is useful for delivery of a drug (such as a PRO polypeptide or antibody thereto) to a mammal. The components of the liposome are commonly arranged in a bilayer formation, similar to the lipid arrangement of biological membranes.

A "small molecule" is defined herein to have a molecular weight below about 500 Daltons.

The term "immune related disease" means a disease in which a component of the immune system of a mammal causes, mediates or otherwise contributes to a morbidity in the mammal. Also included are diseases in which stimulation or intervention of the immune response has an ameliorative effect on progression of the disease. Included within this term are immune-mediated inflammatory diseases, non-immune-mediated inflammatory diseases, infectious diseases, immunodeficiency diseases, neoplasia, *etc*.

The term "B cell mediated disease" means a disease in which B cells directly or indirectly mediate or otherwise contribute to a morbidity in a mammal. The B cell mediated disease may be associated with cell mediated effects, Ig mediated effects, *etc*., and even effects associated with T cells if the T cells are stimulated, for example, by the lymphokines secreted by B cells.

Examples of immune-related and inflammatory diseases, some of which are immune or B cell mediated, which can be treated according to the invention include: systemic lupus erythematosis, X-linked infantile hypogammaglobulinemia, polysaccaride antigen unresponsiveness, selective IgA deficiency, selective IgM deficiency, selective deficiency of IgG subclasses, immunodeficiency with hyper Ig-M, transient hypogammaglobulinemia of infancy, Burkitt's lymphoma, Intermediate lymphoma, follicular lymphoma, typeII hypersensitivity, rheumatoid arthritis, autoimmune mediated hemolytic anemia, myesthenia gravis, hypoadrenocorticism, glomerulonephritis and ankylosing spondylitis.

The term "effective amount" is a concentration or amount of a PRO polypeptide and/or agonist/antagonist which results in achieving a particular stated purpose. An "effective amount" of a PRO polypeptide or agonist or antagonist thereof may be determined empirically. Furthermore, a "therapeutically effective amount" is a concentration or amount of a PRO polypeptide and/or agonist/antagonist which is effective for achieving a stated therapeutic effect. This amount may also be determined empirically.

The term "cytotoxic agent" as used herein refers to a substance that inhibits or prevents the function of cells and/or causes destruction of cells. The term is intended to include radioactive isotopes (*e.g*., I¹³¹, I¹²⁵, Y⁹⁰ and Re¹⁸⁶), chemotherapeutic agents, and toxins such as enzymatically active toxins of bacterial, fungal, plant or animal origin, or fragments thereof.

A "chemotherapeutic agent" is a chemical compound useful in the treatment of cancer. Examples of chemotherapeutic agents include adriamycin, doxorubicin, epirubicin, 5-fluorouracil, cytosine arabinoside ("Ara-C"), cyclophosphamide, thiotepa, busulfan, cytoxin, taxoids, *e.g*., paclitaxel (Taxol, Bristol-Myers Squibb Oncology, Princeton, NJ), and doxetaxel (Taxotere, Rhône-Poulenc Rorer, Antony, France), toxotere, methotrexate, cisplatin, melphalan, vinblastine, bleomycin, etoposide, ifosfamide, mitomycin C, mitoxantrone, vincristine, vinorelbine, carboplatin, teniposide, daunomycin, carminomycin, aminopterin, dactinomycin, mitomycins, esperamicins (see U.S. Pat. No. 4,675,187), melphalan and other related nitrogen mustards. Also included in this definition are hormonal agents that act to regulate or inhibit hormone action on tumors such as tamoxifen and onapristone.

A "growth inhibitory agent" when used herein refers to a compound or composition which inhibits growth of a cell, especially cancer cell overexpressing any of the genes identified herein, either *in vitro* or *in vivo.* Thus, the growth inhibitory agent is one which significantly reduces the percentage of cells overexpressing such genes in S phase. Examples of growth inhibitory agents include agents that block cell cycle progression (at a place other than S phase), such as agents that induce G 1 arrest and M-phase arrest. Classical M-phase blockers include the vincas (vincristine and vinblastine), taxol, and topo II inhibitors such as doxorubicin, epirubicin, daunorubicin, etoposide, and bleomycin. Those agents that arrest G1 also spill over into S-phase arrest, for example, DNA alkylating agents such as tamoxifen, prednisone, dacarbazine, mechlorethamine, cisplatin, methotrexate, 5-fluorouracil, and ara-C. Further information can be found in The Molecular Basis of Cancer, Mendelsohn and Israel, eds., Chapter 1, entitled "Cell cycle regulation, oncogens, and antineoplastic drugs" by Murakami et al. (WB Saunders: Philadelphia, 1995), especially p. 13.

The term "cytokine" is a generic term for proteins released by one cell population which act on another cell as intercellular mediators. Examples of such cytokines are lymphokines, monokines, and traditional polypeptide hormones. Included among the cytokines are growth hormone such as human growth hormone, N-methionyl human growth hormone, and bovine growth hormone; parathyroid hormone; thyroxine; insulin; proinsulin; relaxin; prorelaxin; glycoprotein hormones such as follicle stimulating hormone (FSH), thyroid stimulating hormone (TSH), and luteinizing hormone (LH); hepatic growth factor; fibroblast growth factor; prolactin; placental lactogen; tumor necrosis factor-α and -β; mullerian-inhibiting substance; mouse gonadotropin-associated peptide; inhibin; activin; vascular endothelial growth factor; integrin; thrombopoietin (TPO); nerve growth factors such as NGF-β; platelet-growth factor; transforming growth factors (TGFs) such as TGF-α and TGF-β; insulin-like growth factor-I and -II; erythropoietin (EPO); osteoinductive factors; interferons such as interferon-α, -β, and -γ; colony stimulating factors (CSFs) such as macrophage-CSF (M-CSF); granulocyte-macrophage-CSF (GM-CSF); and granulocyte-CSF (G-CSF); interleukins (ILs) such as IL-1, IL-1α, IL-2, IL-3, IL-4, IL-5, IL-6, IL-7, IL-8, IL-9, IL-11, IL-12; a tumor necrosis factor such as TNF-α or TNF-β; and other polypeptide factors including LIF and kit ligand (KL). As used herein, the term cytokine includes proteins from natural sources or from recombinant cell culture and biologically active equivalents of the native sequence cytokines.

As used herein, the term "immunoadhesin" designates antibody-like molecules which combine the binding specificity of a heterologous protein (an "adhesin") with the effector functions of immunoglobulin constant domains. Structurally, the immunoadhesins comprise a fusion of an amino acid sequence with the desired binding specificity which is other than the antigen recognition and binding site of an antibody (*i.e*., is "heterologous"), and an immunoglobulin constant domain sequence. The adhesin part of an immunoadhesin molecule typically is a contiguous amino acid sequence comprising at least the binding site of a receptor or a ligand. The immunoglobulin constant domain sequence in the immunoadhesin may be obtained from any immunoglobulin, such as IgG-1, IgG-2, IgG-3, or IgG-4 subtypes, IgA (including IgA-1 and IgA-2), IgE, IgD or IgM.

As used herein, the term "inflammatory cells" designates cells that enhance the inflammatory response such as mononuclear cells, eosinophils, macrophages, and polymorphonuclear neutrophils (PMN).

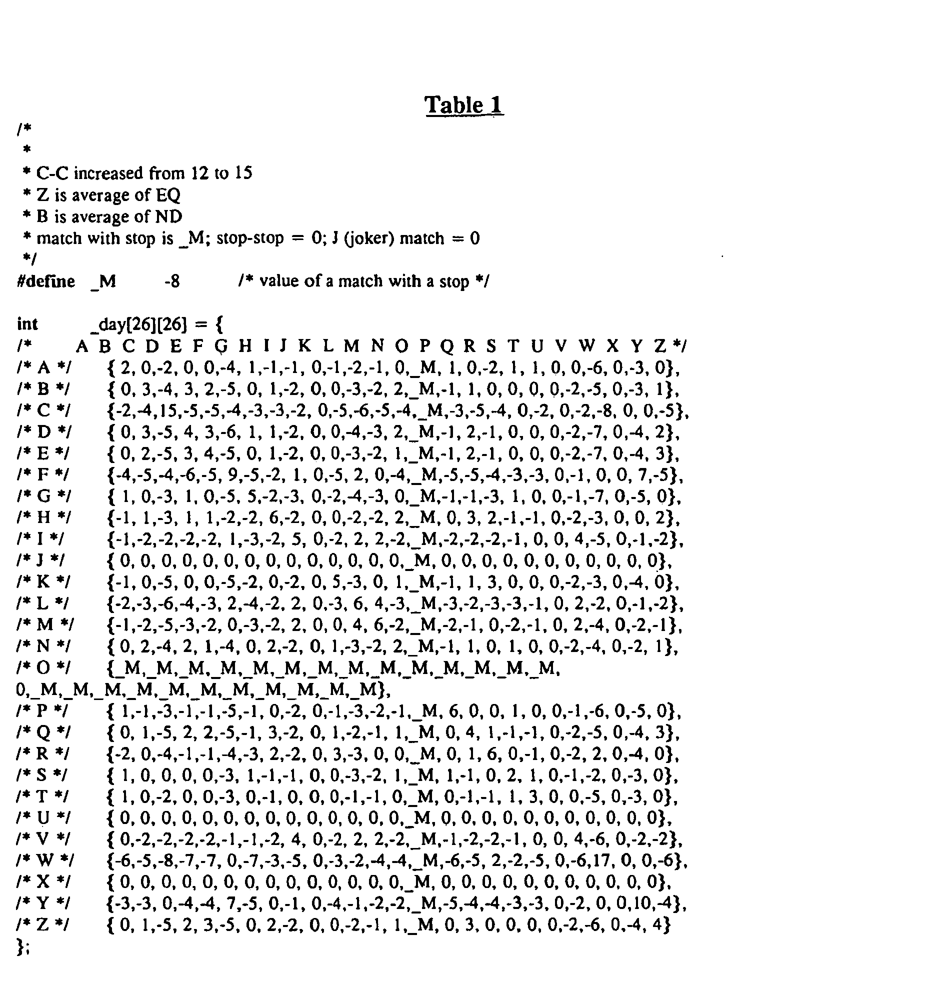

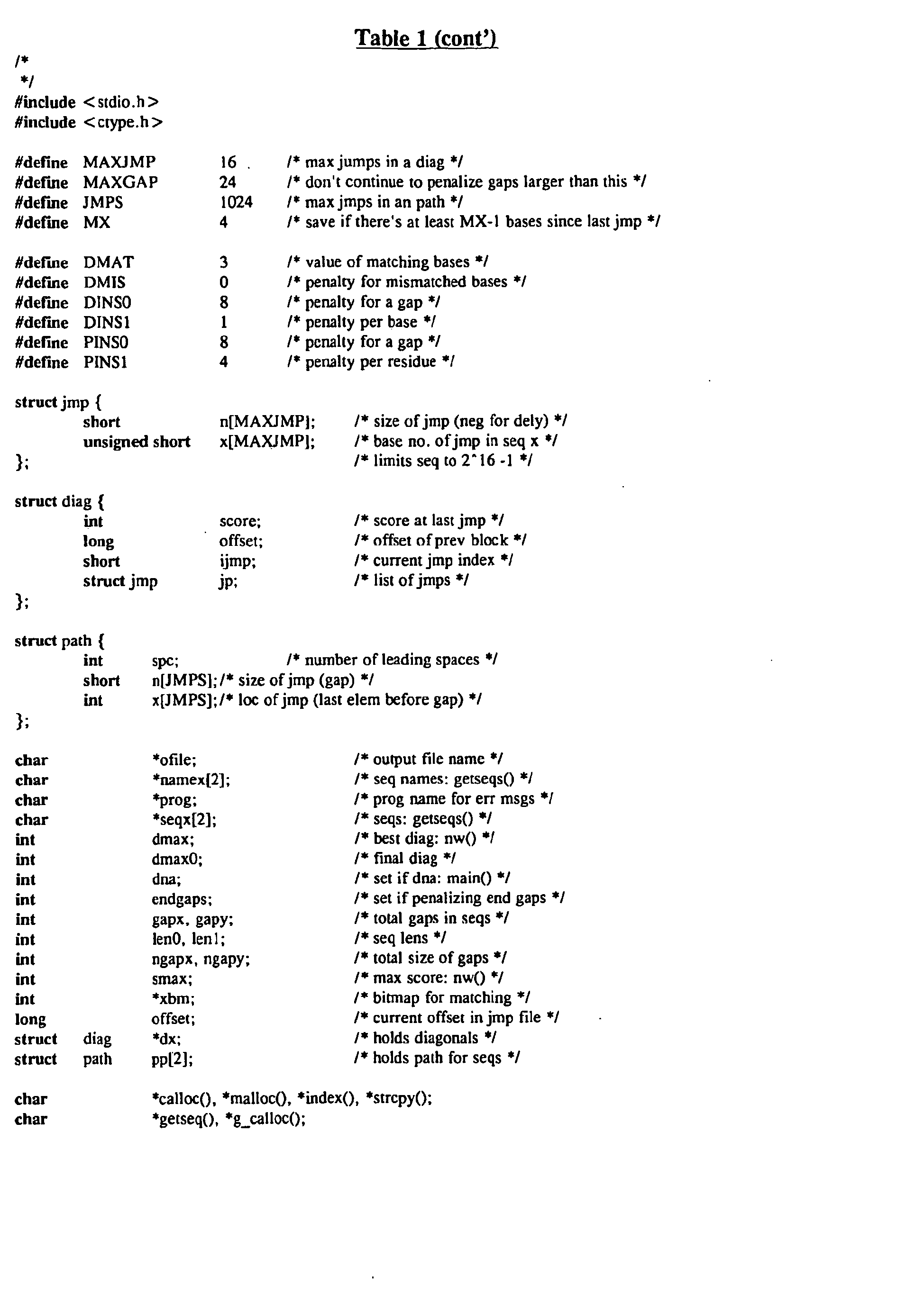

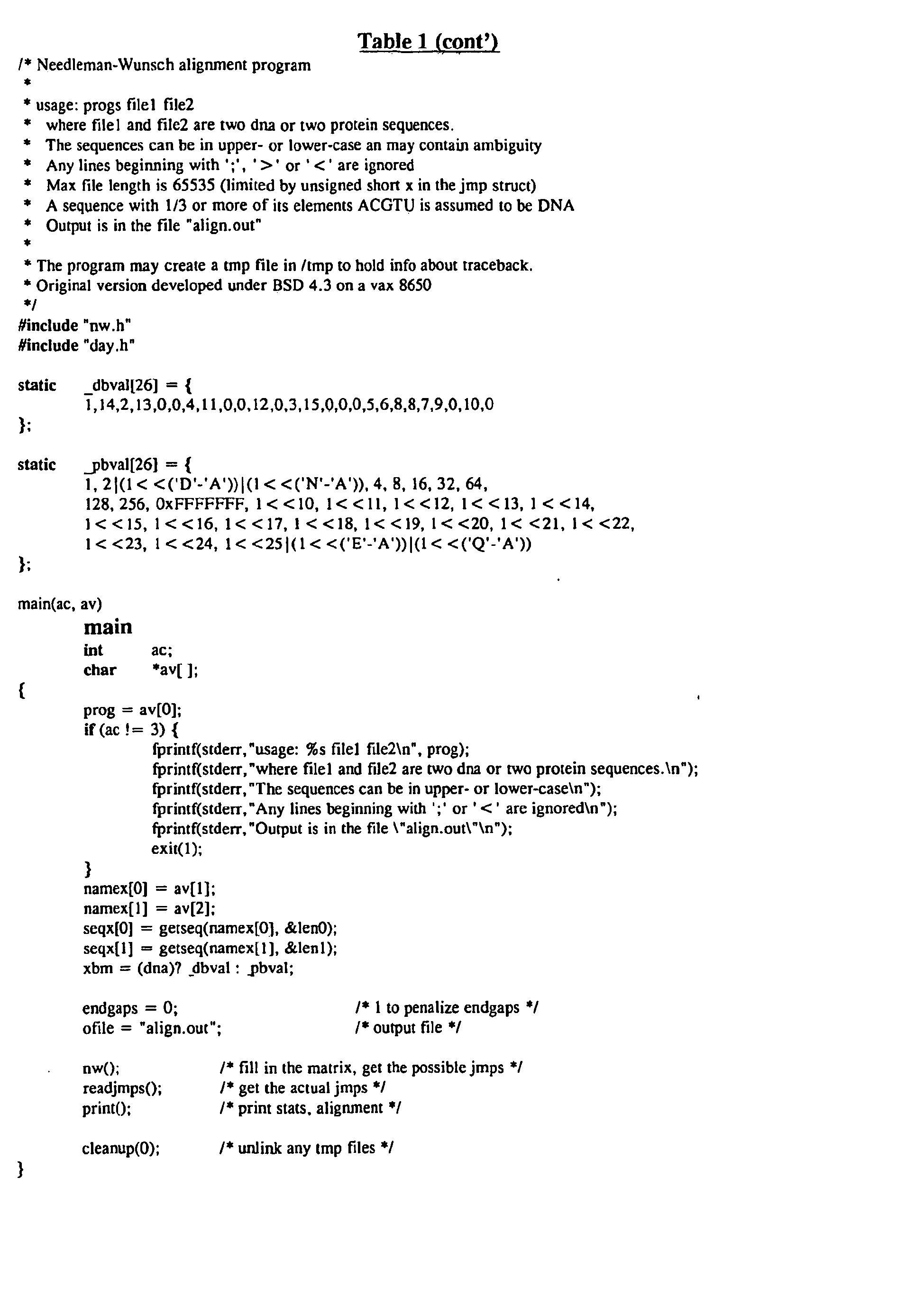

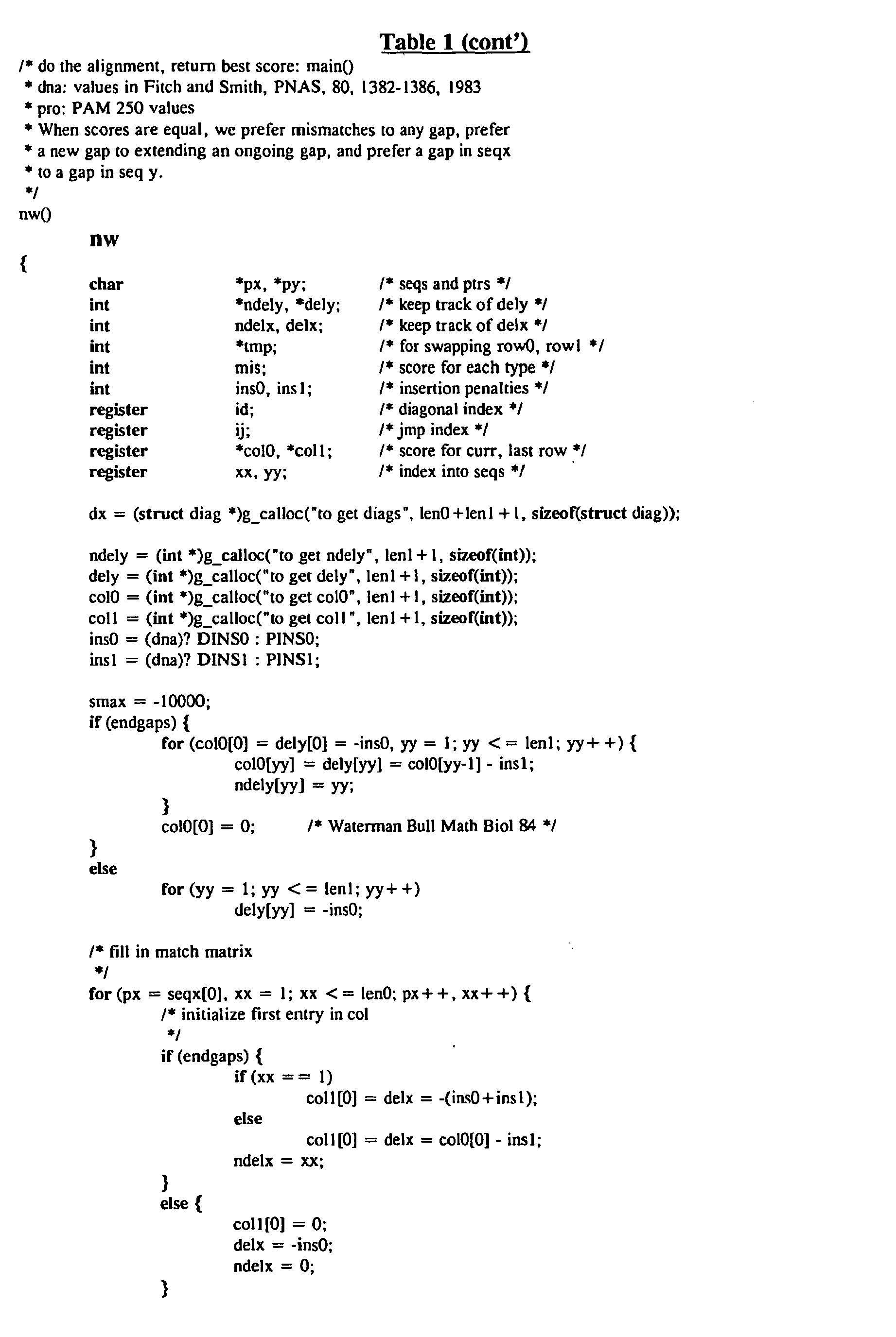

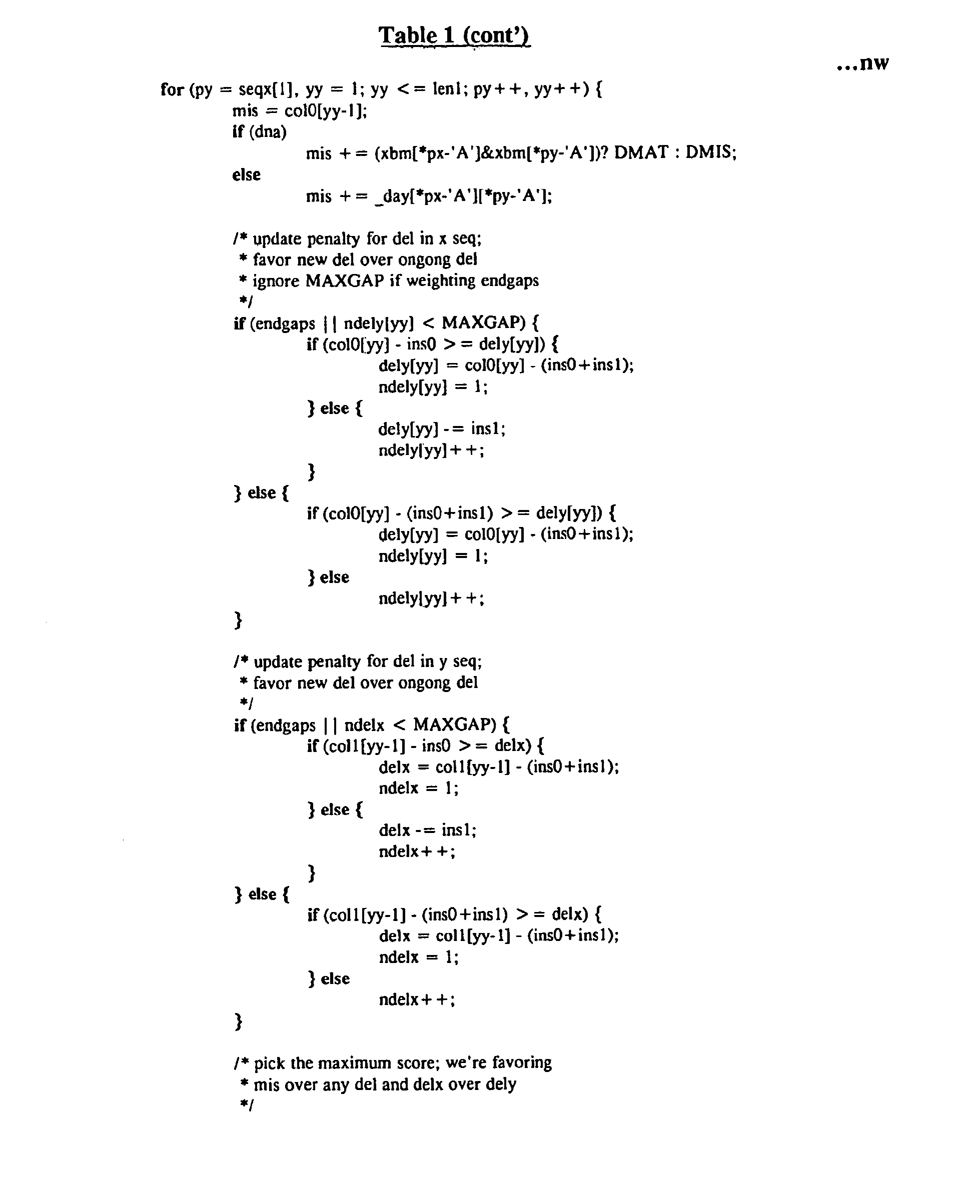

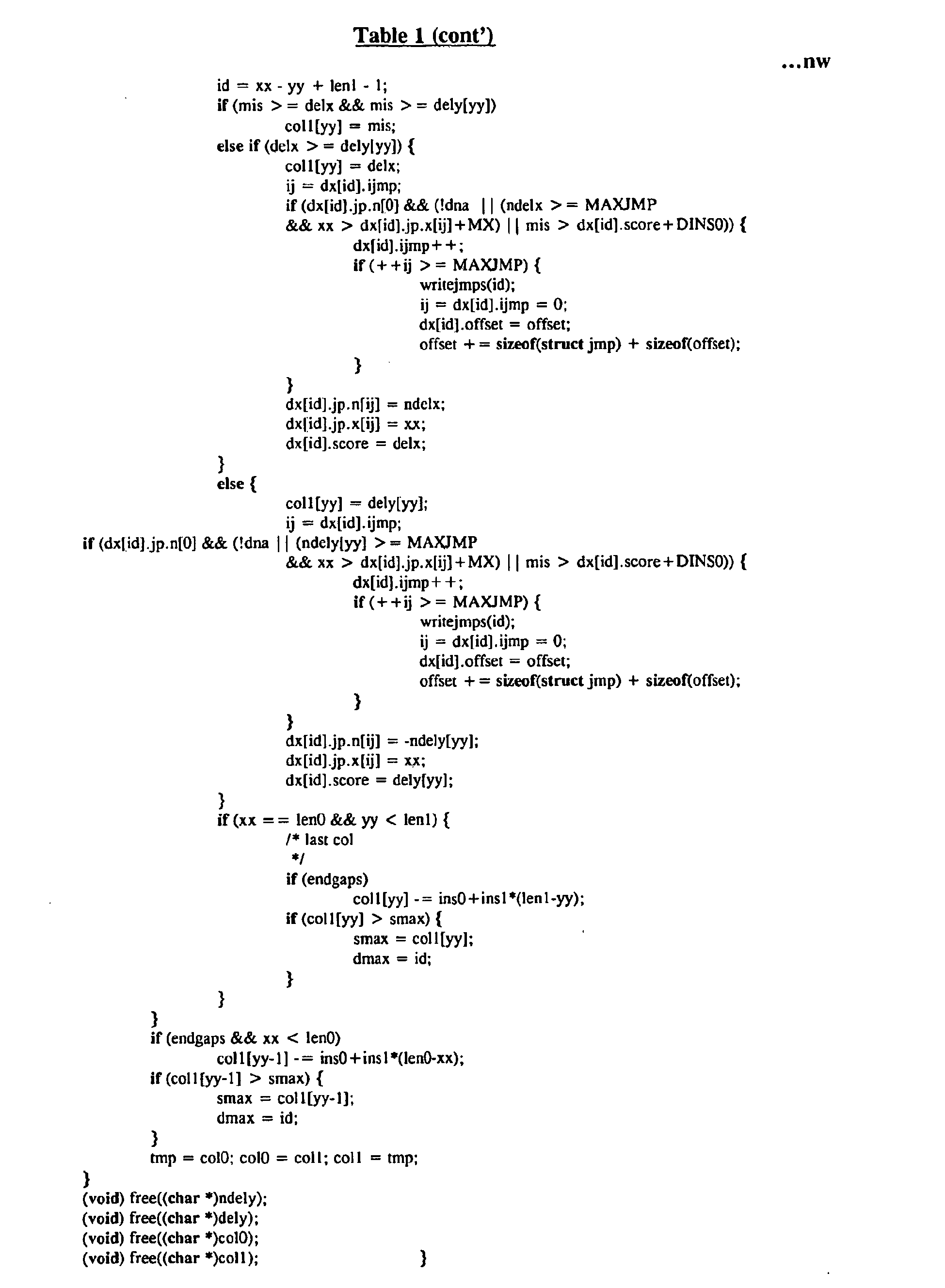

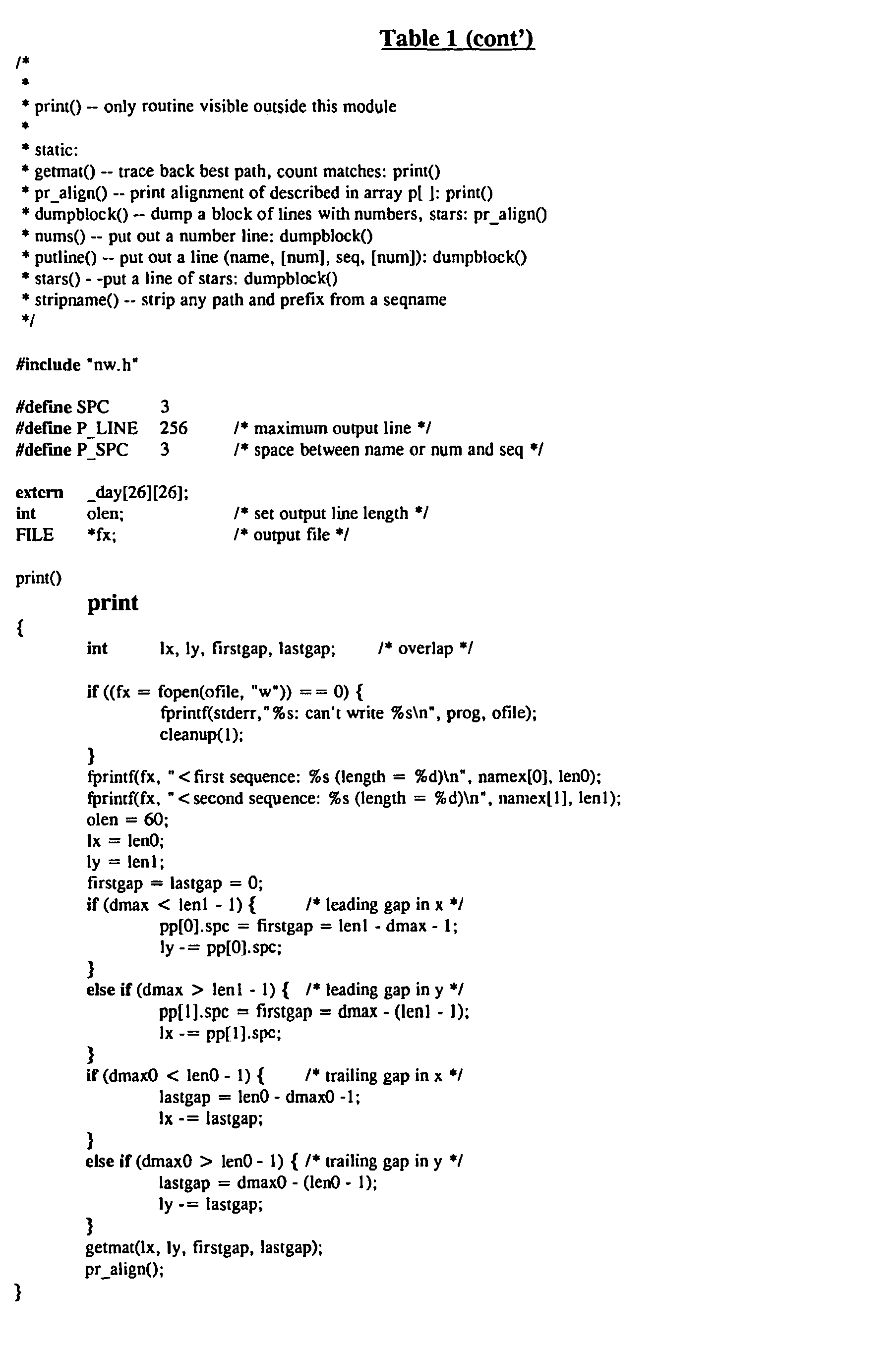

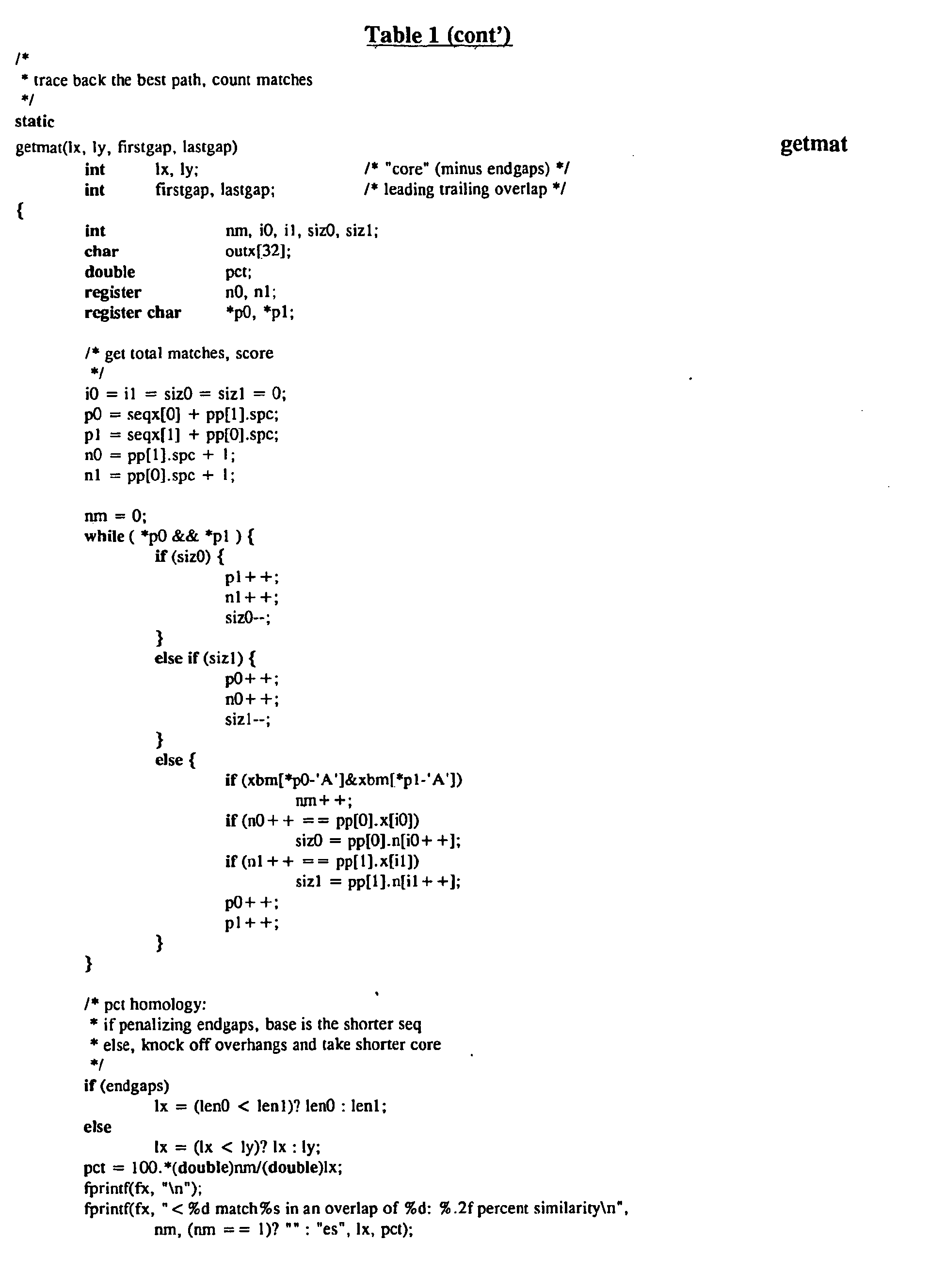

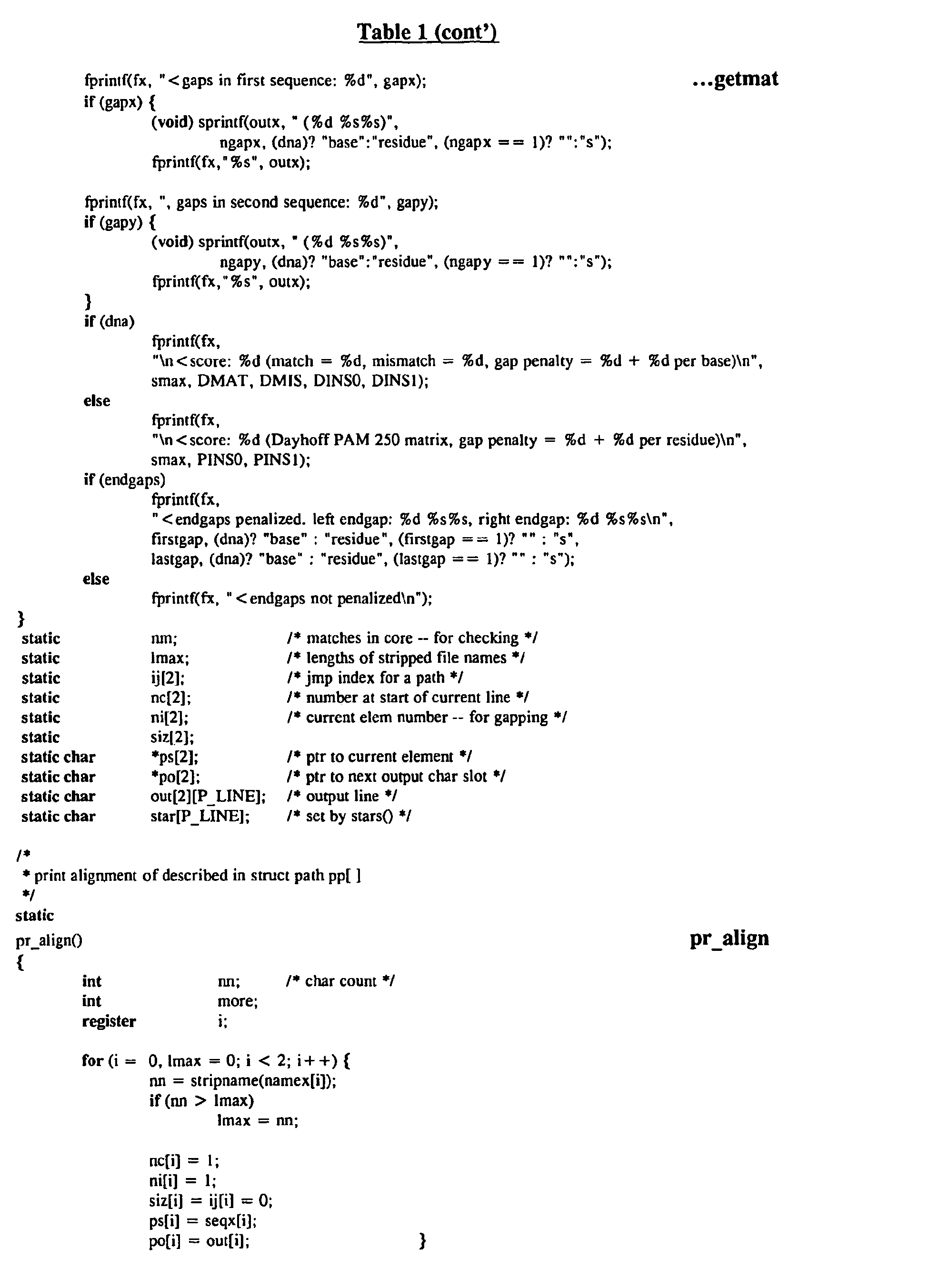

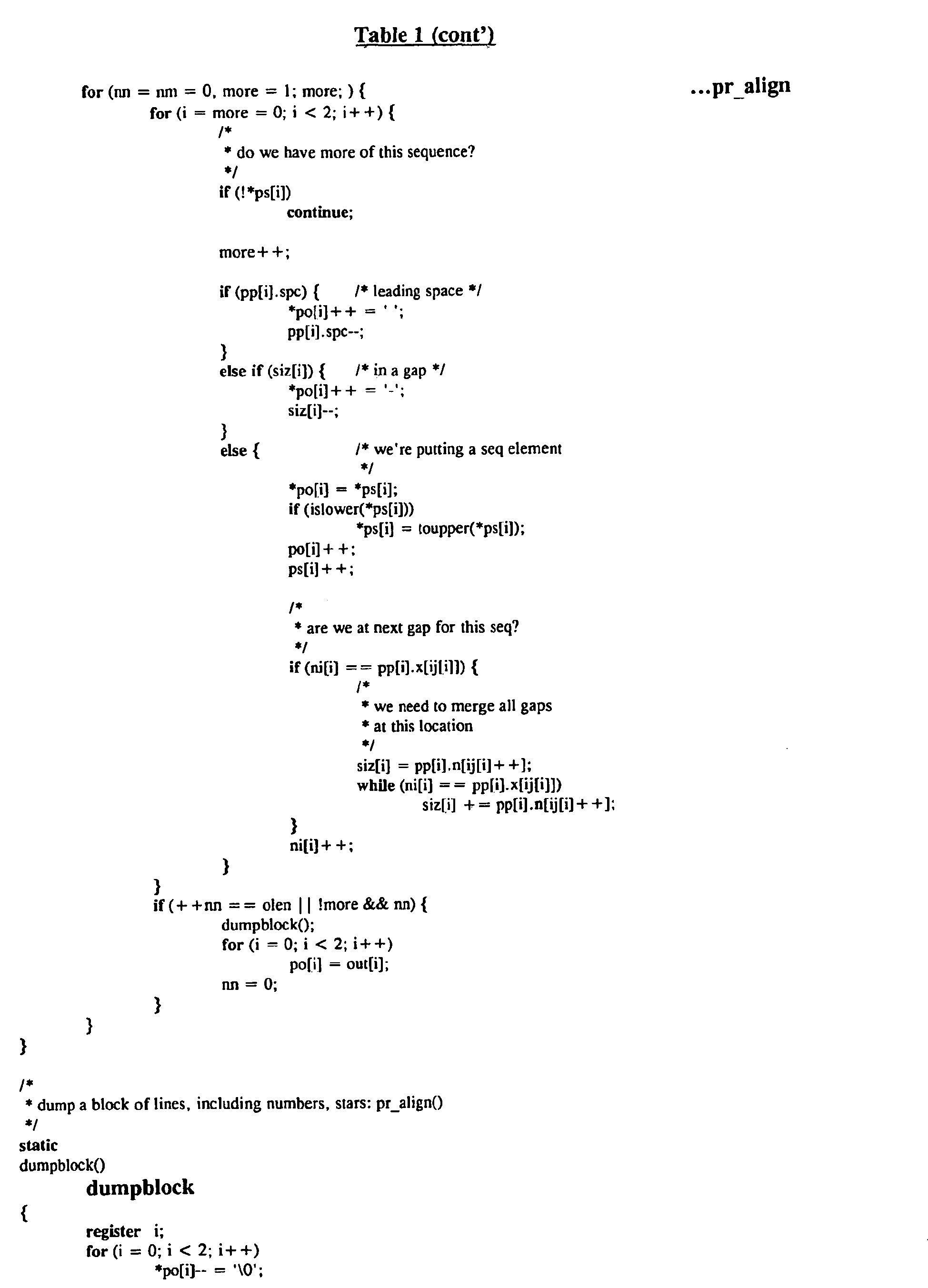

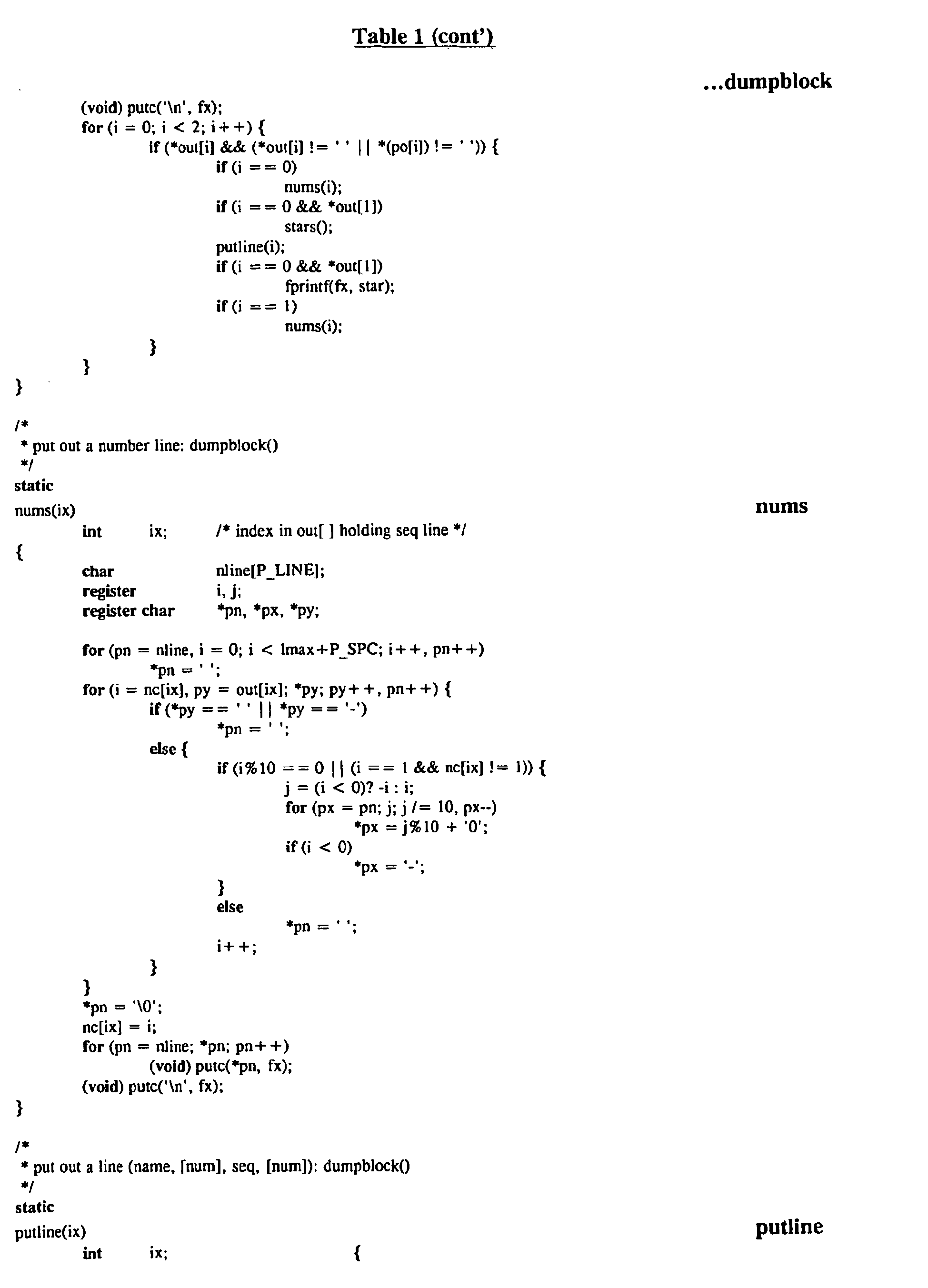

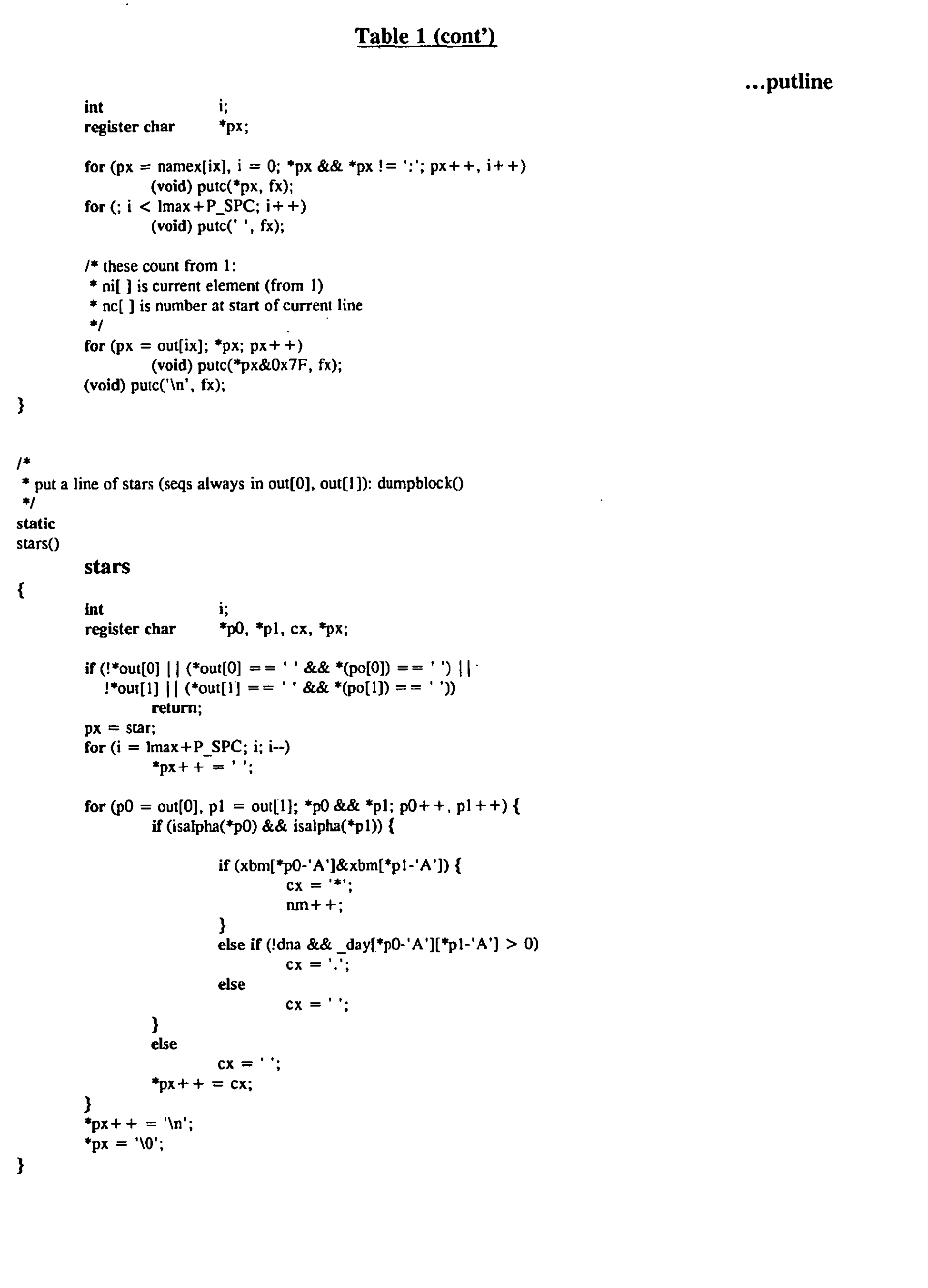

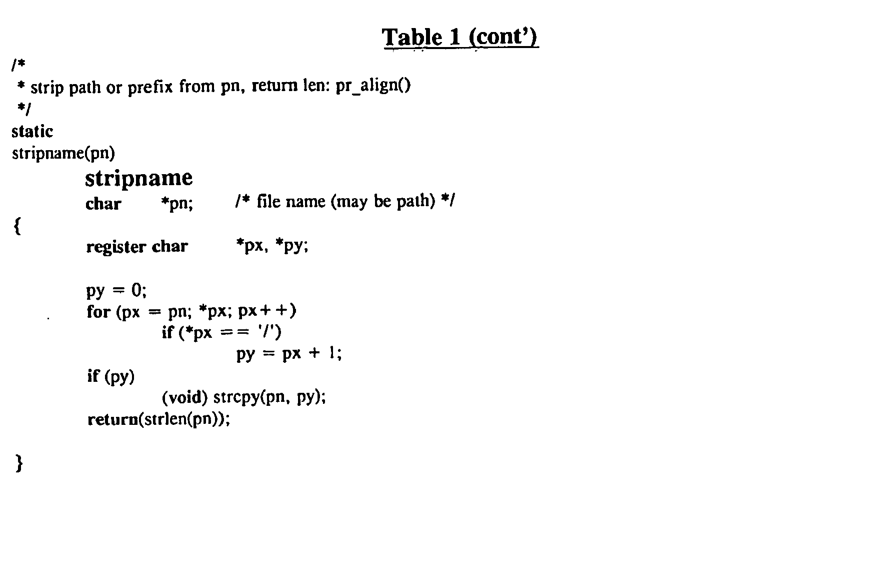

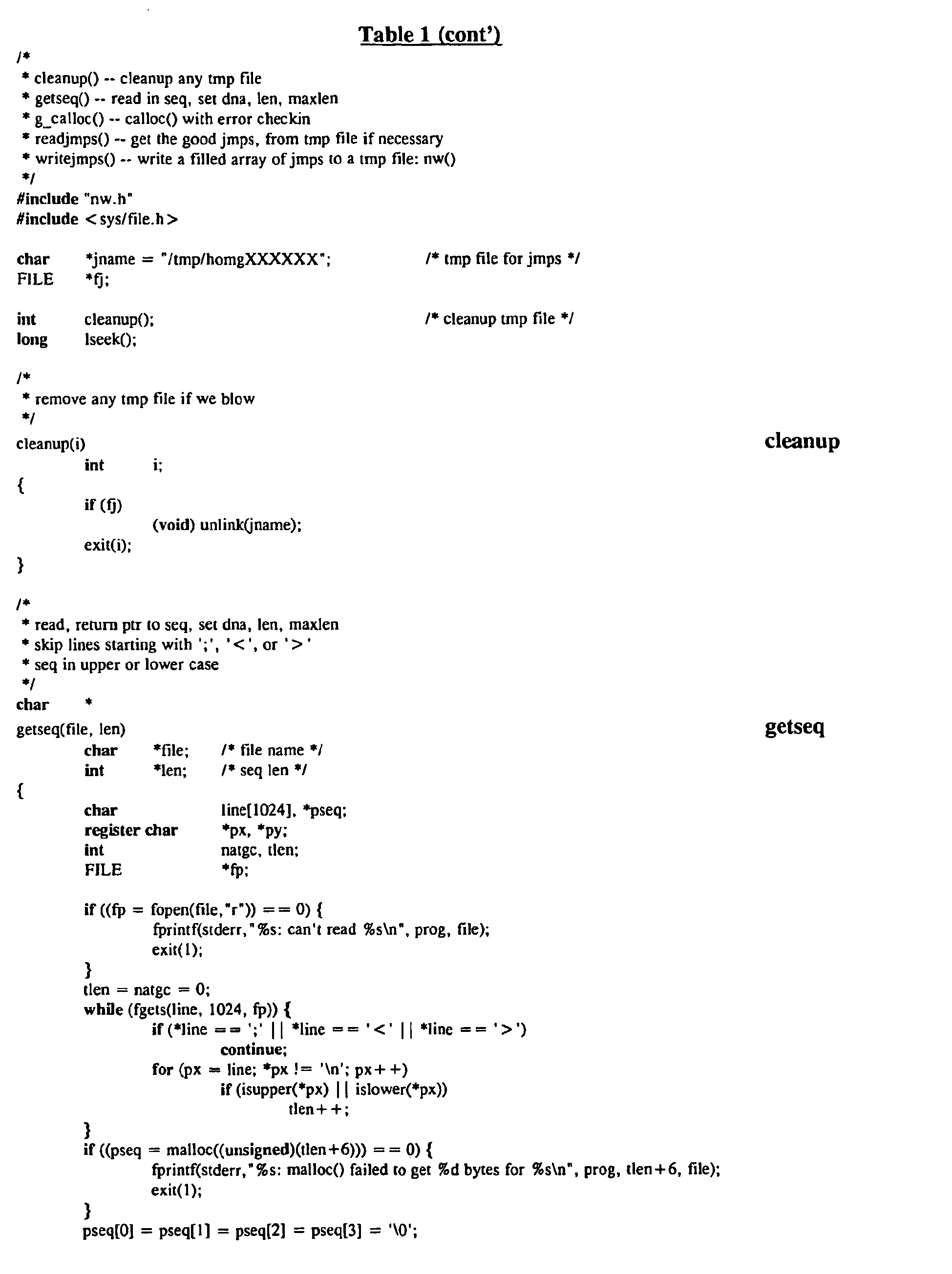

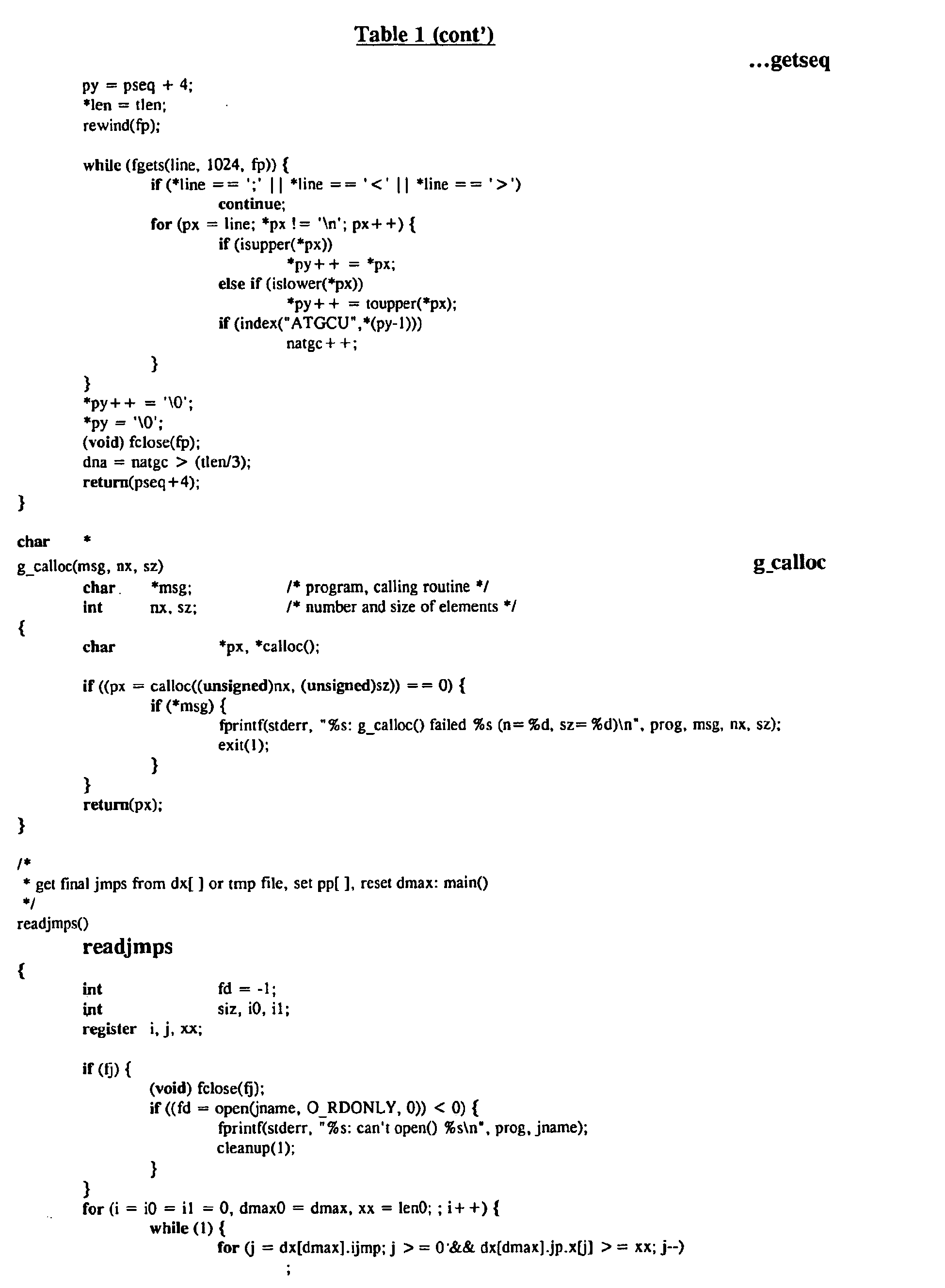

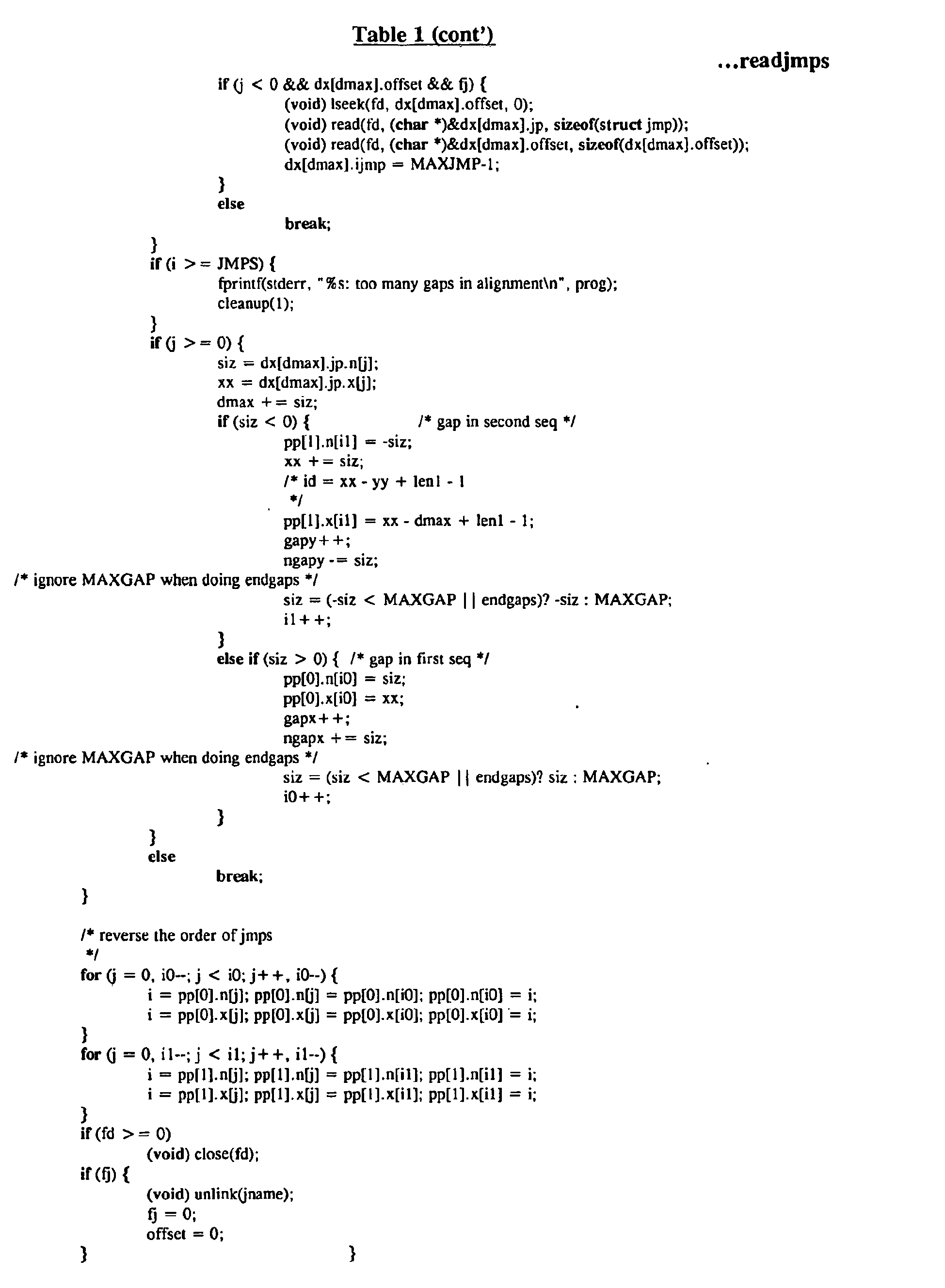

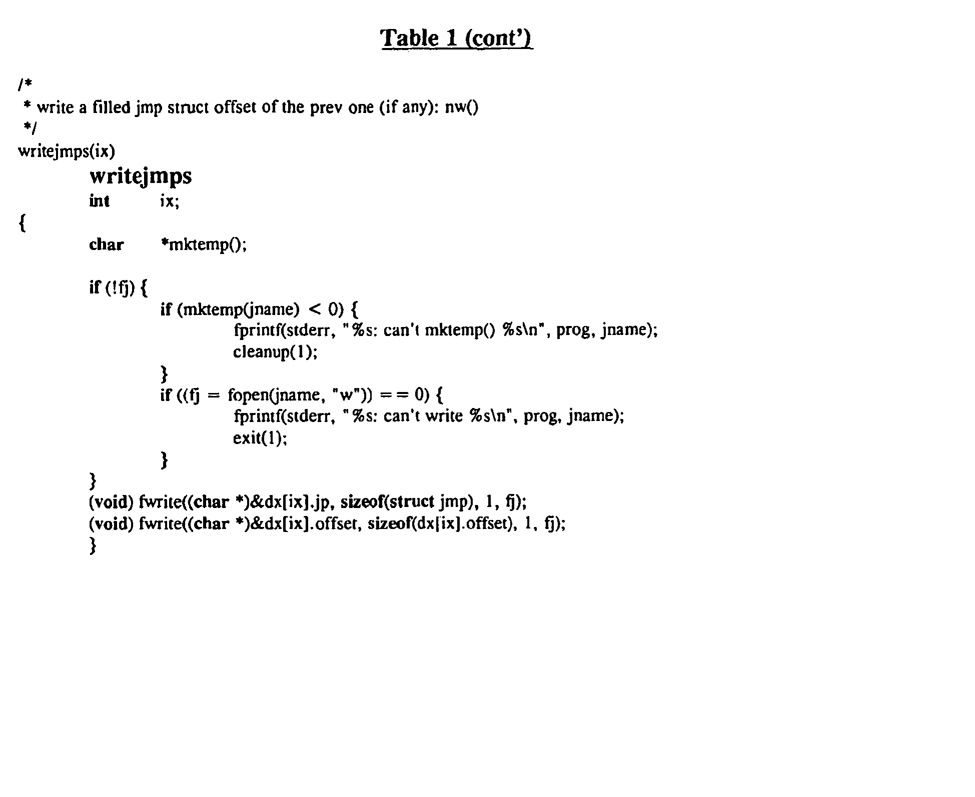

**Table 2**

| | | |
|---|---|---|
| PRO | XXXXXXXXXXXXXXX | (Length = 15 amino acids) |
| Comparison Protein | XXXXXYYYYYYY | (Length = 12 amino acids) |
| % amino acid sequence identity = | | |

(the number of identically matching amino acid residues between the two polypeptide sequences as determined by ALIGN-2) divided by (the total number of amino acid residues of the PRO polypeptide) = 5 divided by 15 = 33.3%

**Table 3**

| | | |
|---|---|---|
| PRO | XXXXXXXXXX | (Length = 10 amino acids) |
| Comparison Protein | XXXXXYYYYYYZZYZ | (Length = 15 amino acids) |
| % amino acid sequence identity = | | |

(the number of identically matching amino acid residues between the two polypeptide sequences as determined by ALIGN-2) divided by (the total number of amino acid residues of the PRO polypeptide) = 5 divided by 10 =50%

**Table 4**

| | | |
|---|---|---|
| PRO-DNA | NNNNNNNNNNNNNN | (Length = 14 nucleotides) |
| Comparison DNA | NNNNNNLLLLLLLLLL | (Length = 16 nucleotides) |
| % nucleic acid sequence identity = | | |

(the number of identically matching nucleotides between the two nucleic acid sequences as determined by ALIGN-2) divided by (the total number of nucleotides of the PRO-DNA nucleic acid sequence) = 6 divided by 14 = 42.9%

**Table 5**

| | | |
|---|---|---|
| PRO-DNA | NNNNNNNNNNNN | (Length = 12 nucleotides) |
| Comparison DNA | NNNNLLLVV | (Length = 9 nucleotides) |
| % nucleic acid sequence identity = | | |

(the number of identically matching nucleotides between the two nucleic acid sequences as determined by ALIGN-2) divided by (the total number of nucleotides of the PRO-DNA nucleic acid sequence) = 4 divided by 12 = 33.3%

### II. Compositions and Methods of the Invention

### A. Full-Length PRO Polypeptides

The present invention provides newly identified and isolated nucleotide sequences encoding polypeptides referred to in the present application as PRO polypeptides. In particular, cDNAs encoding various PRO polypeptides have been identified and isolated, as disclosed in further detail in the Examples below. It is noted that proteins produced in separate expression rounds may be given different PRO numbers but the UNQ number is unique for any given DNA and the encoded protein, and will not be changed. However, for sake of simplicity, in the present specification the protein encoded by the full length native nucleic acid molecules disclosed herein as well as all further native homologues and variants included in the foregoing definition of PRO, will be referred to as "PRO/number", regardless of their origin or mode of preparation.

As disclosed in the Examples below, various cDNA clones have been deposited with the ATCC. The actual nucleotide sequences of those clones can readily be determined by the skilled artisan by sequencing of the deposited clone using routine methods in the art. The predicted amino acid sequence can be determined from the nucleotide sequence using routine skill. For the PRO polypeptides and encoding nucleic acids described herein, Applicants have identified what is believed to be the reading frame best identifiable with the sequence information available at the time.

### B. PRO Polypeptide Variants

In addition to the full-length native sequence PRO polypeptides described herein, it is contemplated that PRO variants can be prepared. PRO variants can be prepared by introducing appropriate nucleotide changes into the PRO DNA, and/or by synthesis of the desired PRO polypeptide. Those skilled in the art will appreciate that amino acid changes may alter post-translational processes of the PRO, such as changing the number or position of glycosylation sites or altering the membrane anchoring characteristics.

Variations in the native full-length sequence PRO or in various domains of the PRO described herein, can be made, for example, using any of the techniques and guidelines for conservative and non-conservative mutations set forth, for instance, in U.S. Patent No. 5,364,934. Variations may be a substitution, deletion or insertion of one or more codons encoding the PRO that results in a change in the amino acid sequence of the PRO as compared with the native sequence PRO. Optionally, the variation is by substitution of at least one amino acid with any other amino acid in one or more of the domains of the PRO. Guidance in determining which amino acid residue may be inserted, substituted or deleted without adversely affecting the desired activity may be found by comparing the sequence of the PRO with that of homologous known protein molecules and minimizing the number of amino acid sequence changes made in regions of high homology. Amino acid substitutions can be the result of replacing one amino acid with another amino acid having similar structural and/or chemical properties, such as the replacement of a leucine with a serine, i.e., conservative amino acid replacements. Insertions or deletions may optionally be in the range of about 1 to 5 amino acids. The variation allowed may be determined by systematically making insertions, deletions or substitutions of amino acids in the sequence and testing the resulting variants for activity exhibited by the full-length or mature native sequence.

PRO polypeptide fragments are provided herein. Such fragments may be truncated at the N-terminus or C-terminus, or may lack internal residues, for example, when compared with a full length native protein. Certain fragments lack amino acid residues that are not essential for a desired biological activity of the PRO polypeptide.

PRO fragments may be prepared by any of a number of conventional techniques. Desired peptide fragments may be chemically synthesized. An alternative approach involves generating PRO fragments by enzymatic digestion, e.g., by treating the protein with an enzyme known to cleave proteins at sites defined by particular amino acid residues, or by digesting the DNA with suitable restriction enzymes and isolating the desired fragment. Yet another suitable technique involves isolating and amplifying a DNA fragment encoding a desired polypeptide fragment, by polymerase chain reaction (PCR). Oligonucleotides that define the desired termini of the DNA fragment are employed at the 5' and 3' primers in the PCR. Preferably, PRO polypeptide fragments share at least one biological and/or immunological activity with the native PRO polypeptide disclosed herein.

In particular embodiments, conservative substitutions of interest are shown in Table 6 under the heading of preferred substitutions. If such substitutions result in a change in biological activity, then more substantial changes, denominated exemplary substitutions in Table 6, or as further described below in reference to amino acid classes, are introduced and the products screened.

**Table 6**

| Original | Exemplary | Preferred |
|---|---|---|
| Residue | Substitutions | Substitutions |
| Ala (A) | val; leu; ile | val |
| Arg (R) | lys; gln; asn | lys |
| Asn (N) | gln; his; lys; arg | gln |
| Asp (D) | glu | glu |
| Cys (C) | ser | ser |
| Gln (Q) | asn | asn |
| Glu (E) | asp | asp |
| Gly (G) | pro; ala | ala |
| His (H) | asn; gln; lys; arg | arg |
| Ile (I) | leu; val; met; ala; phe; norleucine | leu |
| Leu (L) | norleucine; ile; val; met; ala; phe | ile |
| Lys (K) | arg; gln; asn | arg |
| Met (M) | leu; phe; ile | leu |
| Phe (F) | leu; val; ile; ala; tyr | leu |
| Pro (P) | ala | ala |
| Ser (S) | thr | thr |
| Thr (T) | ser | ser |
| Trp (W) | tyr; phe | tyr |
| Tyr (Y) | trp; phe; thr; ser | phe |
| Val (V) | ile; leu; met; phe; ala; norleucine | leu |

Substantial modifications in function or immunological identity of the PRO polypeptide are accomplished by selecting substitutions that differ significantly in their effect on maintaining (a) the structure of the polypeptide backbone in the area of the substitution, for example, as a sheet or helical conformation, (b) the charge or hydrophobicity of the molecule at the target site, or (c) the bulk of the side chain. Naturally occurring residues are divided into groups based on common side-chain properties:
(1) hydrophobic: norleucine, met, ala, val, leu, ile;
(2) neutral hydrophilic: cys, ser, thr;
(3) acidic: asp, glu;
(4) basic: asn, gln, his, lys, arg;
(5) residues that influence chain orientation: gly, pro; and
(6) aromatic: trp, tyr, phe.

Non-conservative substitutions will entail exchanging a member of one of these classes for another class. Such substituted residues also may be introduced into the conservative substitution sites or, more preferably, into the remaining (non-conserved) sites.

The variations can be made using methods known in the art such as oligonucleotide-mediated (site-directed) mutagenesis, alanine scanning, and PCR mutagenesis. Site-directed mutagenesis [Carter et al., Nucl. Acids Res., 13:4331 (1986); Zoller et al., Nucl. Acids Res., 10:6487 (1987)], cassette mutagenesis [Wells et al., Gene, 34:315 (1985)], restriction selection mutagenesis [Wells et al., Philos. Trans. R. Soc. London SerA, 317:415 (1986)] or other known techniques can be performed on the cloned DNA to produce the PRO variant DNA.

Scanning amino acid analysis can also be employed to identify one or more amino acids along a contiguous sequence. Among the preferred scanning amino acids are relatively small, neutral amino acids. Such amino acids include alanine, glycine, serine, and cysteine. Alanine is typically a preferred scanning amino acid among this group because it eliminates the side-chain beyond the beta-carbon and is less likely to alter the main-chain conformation of the variant [Cunningham and Wells, Science, 244: 1081-1085 (1989)]. Alanine is also typically preferred because it is the most common amino acid. Further, it is frequently found in both buried and exposed positions [Creighton, The Proteins, (W.H. Freeman & Co., N.Y.); Chothia, J. Mol. Biol. 150:1 (1976)]. If alanine substitution does not yield adequate amounts of variant, an isoteric amino acid can be used.

### C. Modifications of PRO

Covalent modifications of PRO are included within the scope of this invention. One type of covalent modification includes reacting targeted amino acid residues of a PRO polypeptide with an organic derivatizing agent that is capable of reacting with selected side chains or the N- or C- terminal residues of the PRO. Derivatization with bifunctional agents is useful, for instance, for crosslinking PRO to a water-insoluble support matrix or surface for use in the method for purifying anti-PRO antibodies, and vice-versa. Commonly used crosslinking agents include, e.g., 1,1-bis(diazoacetyl)-2-phenylethane, glutaraldehyde, N-hydroxysuccinimide esters, for example, esters with 4-azidosalicylic acid, homobifunctional imidoesters, including disuccinimidyl esters such as 3,3'-dithiobis(succinimidylpropionate), bifunctional maleimides such as bis-N-maleimido-1,8-octane and agents such as methyl-3-[(p-azidophenyl)dithio]propioimidate.

Other modifications include deamidation of glutaminyl and asparaginyl residues to the corresponding glutamyl and aspartyl residues, respectively, hydroxylation of proline and lysine, phosphorylation of hydroxyl groups of seryl or threonyl residues, methylation of the α-amino groups of lysine, arginine, and histidine side chains [T.E. Creighton, Proteins: Structure and Molecular Properties, W.H. Freeman & Co., San Francisco, pp. 79-86 (1983)], acetylation of the N-terminal amine, and amidation of any C-terminal carboxyl group.

Another type of covalent modification of the PRO polypeptide included within the scope of this invention comprises altering the native glycosylation pattern of the polypeptide. "Altering the native glycosylation pattern" is intended for purposes herein to mean deleting one or more carbohydrate moieties found in native sequence PRO (either by removing the underlying glycosylation site or by deleting the glycosylation by chemical and/or enzymatic means), and/or adding one or more glycosylation sites that are not present in the native sequence PRO. In addition, the phrase includes qualitative changes in the glycosylation of the native proteins, involving a change in the nature and proportions of the various carbohydrate moieties present.

Addition of glycosylation sites to the PRO polypeptide may be accomplished by altering the amino acid sequence. The alteration may be made, for example, by the addition of, or substitution by, one or more serine or threonine residues to the native sequence PRO (for O-linked glycosylation sites). The PRO amino acid sequence may optionally be altered through changes at the DNA level, particularly by mutating the DNA encoding the PRO polypeptide at preselected bases such that codons are generated that will translate into the desired amino acids.

Another means of increasing the number of carbohydrate moieties on the PRO polypeptide is by chemical or enzymatic coupling of glycosides to the polypeptide. Such methods are described in the art, e.g., in WO 87/05330 published 11 September 1987, and in Aplin and Wriston, CRC Crit. Rev. Biochem., pp. 259-306 (1981).

Removal of carbohydrate moieties present on the PRO polypeptide may be accomplished chemically or enzymatically or by mutational substitution of codons encoding for amino acid residues that serve as targets for glycosylation. Chemical deglycosylation techniques are known in the art and described, for instance, by Hakimuddin, et al., Arch. Biochem. Biophys., 259:52 (1987) and by Edge et al., Anal. Biochem., 118:131 (1981). Enzymatic cleavage of carbohydrate moieties on polypeptides can be achieved by the use of a variety of endo- and exo-glycosidases as described by Thotakura et al., Meth. Enzymol., 138:350 (1987).

Another type of covalent modification of PRO comprises linking the PRO polypeptide to one of a variety of nonproteinaceous polymers, e.g., polyethylene glycol (PEG), polypropylene glycol, or polyoxyalkylenes, in the manner set forth in U.S. Patent Nos. 4,640,835; 4,496,689; 4,301,144; 4,670,417; 4,791,192 or 4,179,337.

The PRO of the present invention may also be modified in a way to form a chimeric molecule comprising PRO fused to another, heterologous polypeptide or amino acid sequence.

In one embodiment, such a chimeric molecule comprises a fusion of the PRO with a tag polypeptide which provides an epitope to which an anti-tag antibody can selectively bind. The epitope tag is generally placed at the amino- or carboxyl-terminus of the PRO. The presence of such epitope-tagged forms of the PRO can be detected using an antibody against the tag polypeptide. Also, provision of the epitope tag enables the PRO to be readily purified by affinity purification using an anti-tag antibody or another type of affinity matrix that binds to the epitope tag. Various tag polypeptides and their respective antibodies are well known in the art. Examples include poly-histidine (poly-his) or poly-histidine-glycine (poly-his-gly) tags; the flu HA tag polypeptide and its antibody 12CA5 [Field et al., Mol. Cell. Biol., 8:2159-2165 (1988)]; the c-myc tag and the 8F9, 3C7, 6E10, G4, B7 and 9E10 antibodies thereto [Evan et al., Molecular and Cellular Biology, 5:3610-3616 (1985)]; and the Herpes Simplex virus glycoprotein D (gD) tag and its antibody [Paborsky et al., Protein Engineering, 3(6):547-553 (1990)]. Other tag polypeptides include the Flag-peptide [Hopp et al., BioTechnology, 6:1204-1210 (1988)]; the KT3 epitope peptide [Martin et al., Science, 255:192-194 (1992)]; an alpha-tubulin epitope peptide [Skinner et al., J. Biol. Chem., 266:15163-15166 (1991)]; and the T7 gene 10 protein peptide tag [Lutz-Freyermuth et al., Proc. Natl. Acad. Sci. USA, 87:6393-6397 (1990)].

In an alternative embodiment, the chimeric molecule may comprise a fusion of the PRO with an immunoglobulin or a particular region of an immunoglobulin. For a bivalent form of the chimeric molecule (also referred to as an "immunoadhesin"), such a fusion could be to the Fc region of an IgG molecule. The Ig fusions preferably include the substitution of a soluble (transmembrane domain deleted or inactivated) form of a PRO polypeptide in place of at least one variable region within an Ig molecule. In a particularly preferred embodiment, the immunoglobulin fusion includes the hinge, CH2 and CH3, or the hinge, CH1, CH2 and CH3 regions of an IgG1 molecule. For the production of immunoglobulin fusions see also US Patent No. 5,428,130 issued June 27, 1995.

### D. Preparation of PRO

The description below relates primarily to production of PRO by culturing cells transformed or transfected with a vector containing PRO nucleic acid. It is, of course, contemplated that alternative methods, which are well known in the art, may be employed to prepare PRO. For instance, the PRO sequence, or portions thereof, may be produced by direct peptide synthesis using solid-phase techniques [see, e.g., Stewart et al., Solid-Phase Peptide Synthesis, W.H. Freeman Co., San Francisco, CA (1969); Merrifield, J. Am. Chem. Soc., 85:2149-2154 (1963)]. *In vitro* protein synthesis may be performed using manual techniques or by automation. Automated synthesis may be accomplished, for instance, using an Applied Biosystems Peptide Synthesizer (Foster City, CA) using manufacturer's instructions. Various portions of the PRO may be chemically synthesized separately and combined using chemical or enzymatic methods to produce the full-length PRO.

### 1. Isolation of DNA Encoding PRO

DNA encoding PRO may be obtained from a cDNA library prepared from tissue believed to possess the PRO mRNA and to express it at a detectable level. Accordingly, human PRO DNA can be conveniently obtained from a cDNA library prepared from human tissue, such as described in the Examples. The PRO-encoding gene may also be obtained from a genomic library or by known synthetic procedures (e.g., automated nucleic acid synthesis).

Libraries can be screened with probes (such as antibodies to the PRO or oligonucleotides of at least about 20-80 bases) designed to identify the gene of interest or the protein encoded by it. Screening the cDNA or genomic library with the selected probe may be conducted using standard procedures, such as described in Sambrook et al., Molecular Cloning: A Laboratory Manual (New York: Cold Spring Harbor Laboratory Press, 1989). An alternative means to isolate the gene encoding PRO is to use PCR methodology [Sambrook et al., supra; Dieffenbach et al., PCR Primer: A Laboratory Manual (Cold Spring Harbor Laboratory Press, 1995)].

The Examples below describe techniques for screening a cDNA library. The oligonucleotide sequences selected as probes should be of sufficient length and sufficiently unambiguous that false positives are minimized. The oligonucleotide is preferably labeled such that it can be detected upon hybridization to DNA in the library being screened. Methods of labeling are well known in the art, and include the use of radiolabels like ³²P-labeled ATP, biotinylation or enzyme labeling. Hybridization conditions, including moderate stringency and high stringency, are provided in Sambrook et al., supra.

Sequences identified in such library screening methods can be compared and aligned to other known sequences deposited and available in public databases such as GenBank or other private sequence databases. Sequence identity (at either the amino acid or nucleotide level) within defined regions of the molecule or across the full-length sequence can be determined using methods known in the art and as described herein.

Nucleic acid having protein coding sequence may be obtained by screening selected cDNA or genomic libraries using the deduced amino acid sequence disclosed herein for the first time, and, if necessary, using conventional primer extension procedures as described in Sambrook et al., supra, to detect precursors and processing intermediates of mRNA that may not have been reverse-transcribed into cDNA.

### 2. Selection and Transformation of Host Cells

Host cells are transfected or transformed with expression or cloning vectors described herein for PRO production and cultured in conventional nutrient media modified as appropriate for inducing promoters, selecting transformants, or amplifying the genes encoding the desired sequences. The culture conditions, such as media, temperature, pH and the like, can be selected by the skilled artisan without undue experimentation. In general, principles, protocols, and practical techniques for maximizing the productivity of cell cultures can be found in Mammalian Cell Biotechnology: a Practical Approach, M. Butler, ed. (IRL Press, 1991) and Sambrook et al., supra.

Methods of eukaryotic cell transfection and prokaryotic cell transformation are known to the ordinarily skilled artisan, for example, CaCl₂, CaPO₄, liposome-mediated and electroporation. Depending on the host cell used, transformation is performed using standard techniques appropriate to such cells. The calcium treatment employing calcium chloride, as described in Sambrook et al., supra, or electroporation is generally used for prokaryotes. Infection with *Agrobacterium tumefaciens* is used for transformation of certain plant cells, as described by Shaw et al., Gene, 23:315 (1983) and WO 89/05859 published 29 June 1989. For mammalian cells without such cell walls, the calcium phosphate precipitation method of Graham and van der Eb, Virology, 52:456-457 (1978) can be employed. General aspects of mammalian cell host system transfections have been described in U.S. Patent No. 4,399,216. Transformations into yeast are typically carried out according to the method of Van Solingen et al., J. Bact., 130:946 (1977) and Hsiao et al., Proc. Natl. Acad. Sci. (USA), 76:3829 (1979). However, other methods for introducing DNA into cells, such as by nuclear microinjection, electroporation, bacterial protoplast fusion with intact cells, or polycations, e.g., polybrene, polyornithine, may also be used. For various techniques for transforming mammalian cells, see Keown et al., Methods in Enzymology, 185:527-537 (1990) and Mansour et al., Nature, 336:348-352 (1988).

Suitable host cells for cloning or expressing the DNA in the vectors herein include prokaryote, yeast, or higher eukaryote cells. Suitable prokaryotes include but are not limited to eubacteria, such as Gram-negative or Gram-positive organisms, for example, Enterobacteriaceae such as *E. coli.* Various *E*. *coli* strains are publicly available, such as *E. coli* K12 strain MM294 (ATCC 31,446); *E. coli* X1776 (ATCC 31,537); *E. coli* strain W3110 (ATCC 27,325) and K5 772 (ATCC 53,635). Other suitable prokaryotic host cells include Enterobacteriaceae such as *Escherichia,* e.g., *E. coli, Enterobacter, Erwinia, Klebsiella, Proteus, Salmonella,* e.g., *Salmonella typhimurium, Serratia,* e.g., *Serratia marcescans,* and *Shigella,* as well as *Bacilli* such as *B. subtilis* and *B. licheniformis* (e.g., *B. licheniformis* 41P disclosed in DD 266,710 published 12 April 1989), *Pseudomonas* such as *P. aeruginosa,* and *Streptomyces.* These examples are illustrative rather than limiting. Strain W3110 is one particularly preferred host or parent host because it is a common host strain for recombinant DNA product fermentations. Preferably, the host cell secretes minimal amounts of proteolytic enzymes. For example, strain W3110 may be modified to effect a genetic mutation in the genes encoding proteins endogenous to the host, with examples of such hosts including *E. coli* W3110 strain 1A2, which has the complete genotype *tonA ; E. coli* W3110 strain 9E4, which has the complete genotype *tonA ptr3; E. coli* W3110 strain 27C7 (ATCC 55,244), which has the complete genotype *tonA ptr3 phoA E15 (argF-lac)169 degP ompT kan'; E. coli* W3110 strain 37D6, which has the complete genotype *tonA ptr3 phoA E15* (*argF-lac)169 degP ompT rbs7 ilvG kan'; E. coli* W3110 strain 40B4, which is strain 37D6 with a non-kanamycin resistant *degP* deletion mutation; and an *E. coli* strain having mutant periplasmic protease disclosed in U.S. Patent No. 4,946,783 issued 7 August 1990. Alternatively, *in vitro* methods of cloning, e.g., PCR or other nucleic acid polymerase reactions, are suitable.

In addition to prokaryotes, eukaryotic microbes such as filamentous fungi or yeast are suitable cloning or expression hosts for PRO-encoding vectors. *Saccharomyces cerevisiae* is a commonly used lower eukaryotic host microorganism. Others include *Schizosaccharomyces pombe* (Beach and Nurse, Nature, 290: 140 [1981]; EP 139,383 published 2 May 1985); *Kluyveromyces* hosts (U.S. Patent No. 4,943,529; Fleer et al., Bio/Technology, 9:968-975 (1991)) such as, e.g., *K. lactis* (MW98-8C, CBS683, CBS4574; Louvencourt et al., J. Bacteriol., 154(2):737-742 [1983]), *K. fragilis* (ATCC 12,424), *K. bulgaricus* (ATCC 16,045), *K. wickeramii* (ATCC 24,178), *K. waltii* (ATCC 56,500), *K. drosophilarum* (ATCC 36,906; Van den Berg et al., Bio/Technology 8:135 (1990)), *K. thermotolerans,* and *K. marxianus; yarrowia* (EP 402,226); *Pichia pastoris* (EP 183,070; Sreekrishna et al., J. Basic Microbiol., 28:265-278 [1988]); *Candida; Trichoderma reesia* (EP 244,234); *Neurospora crassa* (Case et al., Proc. Natl. Acad. Sci. USA, 76:5259-5263 [1979]); *Schwanniomyces* such as *Schwanniomyces occidentalis* (EP 394,538 published 31 October 1990); and filamentous fungi such as, e.g., *Neurospora, Penicillium, Tolypocladium* (WO 91/00357 published 10 January 1991), and *Aspergillus* hosts such as *A. nidulans* (Ballance et al., Biochem. Biophys. Res. Commun., 112:284-289 [1983]; Tilburn et al., Gene, 26:205-221 [1983]; Yelton et al., Proc. Natl. Acad. Sci. USA, 81: 1470-1474 [1984]) and *A. niger* (Kelly and Hynes, EMBO J., 4:475-479 [1985]). Methylotropic yeasts are suitable herein and include, but are not limited to, yeast capable of growth on methanol selected from the genera consisting of *Hansenula, Candida, Kloeckera, Pichia, Saccharomyces, Torulopsis,* and *Rhodotorula.* A list of specific species that are exemplary of this class of yeasts may be found in C. Anthony, The Biochemistry of Methylotrophs, 269 (1982).

Suitable host cells for the expression of glycosylated PRO are derived from multicellular organisms. Examples of invertebrate cells include insect cells such as Drosophila S2 and Spodoptera Sf9, as well as plant cells. Examples of useful mammalian host cell lines include Chinese hamster ovary (CHO) and COS cells. More specific examples include monkey kidney CV1 line transformed by SV40 (COS-7, ATCC CRL 1651); human embryonic kidney line (293 or 293 cells subcloned for growth in suspension culture, Graham et al., J. Gen Virol., 36:59 (1977)); Chinese hamster ovary cells/-DHFR (CHO, Urlaub and Chasin, Proc. Natl. Acad. Sci. USA, 77:4216 (1980)); mouse sertoli cells (TM4, Mather, Biol. Reprod., 23:243-251 (1980)); human lung cells (W138, ATCC CCL 75); human liver cells (Hep G2, HB 8065); and mouse mammary tumor (MMT 060562, ATCC CCL51). The selection of the appropriate host cell is deemed to be within the skill in the art.

### 3. Selection and Use of a Replicable Vector

The nucleic acid (e.g., cDNA or genomic DNA) encoding PRO may be inserted into a replicable vector for cloning (amplification of the DNA) or for expression. Various vectors are publicly available. The vector may, for example, be in the form of a plasmid, cosmid, viral particle, or phage. The appropriate nucleic acid sequence may be inserted into the vector by a variety of procedures. In general, DNA is inserted into an appropriate restriction endonuclease site(s) using techniques known in the art. Vector components generally include, but are not limited to, one or more of a signal sequence, an origin of replication, one or more marker genes, an enhancer element, a promoter, and a transcription termination sequence. Construction of suitable vectors containing one or more of these components employs standard ligation techniques which are known to the skilled artisan.

The PRO may be produced recombinantly not only directly, but also as a fusion polypeptide with a heterologous polypeptide, which may be a signal sequence or other polypeptide having a specific cleavage site at the N-terminus of the mature protein or polypeptide. In general, the signal sequence may be a component of the vector, or it may be a part of the PRO-encoding DNA that is inserted into the vector. The signal sequence may be a prokaryotic signal sequence selected, for example, from the group of the alkaline phosphatase, penicillinase, lpp, or heat-stable enterotoxin II leaders. For yeast secretion the signal sequence may be, e.g., the yeast invertase leader, alpha factor leader (including *Saccharomyces* and *Kluyveromyces* α-factor leaders, the latter described in U.S. Patent No. 5,010,182), or acid phosphatase leader, the *C*. *albicans* glucoamylase leader (EP 362,179 published 4 April 1990), or the signal described in WO 90/13646 published 15 November 1990. In mammalian cell expression, mammalian signal sequences may be used to direct secretion of the protein, such as signal sequences from secreted polypeptides of the same or related species, as well as viral secretory leaders.

Both expression and cloning vectors contain a nucleic acid sequence that enables the vector to replicate in one or more selected host cells. Such sequences are well known for a variety of bacteria, yeast, and viruses. The origin of replication from the plasmid pBR322 is suitable for most Gram-negative bacteria, the 2µ plasmid origin is suitable for yeast, and various viral origins (SV40, polyoma, adenovirus, VSV or BPV) are useful for cloning vectors in mammalian cells.

Expression and cloning vectors will typically contain a selection gene, also termed a selectable marker. Typical selection genes encode proteins that (a) confer resistance to antibiotics or other toxins, e.g., ampicillin, neomycin, methotrexate, or tetracycline, (b) complement auxotrophic deficiencies, or (c) supply critical nutrients not available from complex media, e.g., the gene encoding D-alanine racemase for *Bacilli.*

An example of suitable selectable markers for mammalian cells are those that enable the identification of cells competent to take µp the PRO-encoding nucleic acid, such as DHFR or thymidine kinase. An appropriate host cell when wild-type DHFR is employed is the CHO cell line deficient in DHFR activity, prepared and propagated as described by Urlaub et al., Proc. Natl. Acad. Sci. USA, 77:4216 (1980). A suitable selection gene for use in yeast is the *trp*1 gene present in the yeast plasmid YRp7 [Stinchcomb et al., Nature, 282:39 (1979); Kingsman et al., Gene, 7:141 (1979); Tschemper et al., Gene, 10:157 (1980)]. The *trp*1 gene provides a selection marker for a mutant strain of yeast lacking the ability to grow in tryptophan, for example, ATCC No. 44076 or PEP4-1 [Jones, Genetics, 85:12 (1977)].

Expression and cloning vectors usually contain a promoter operably linked to the PRO-encoding nucleic acid sequence to direct mRNA synthesis. Promoters recognized by a variety of potential host cells are well known. Promoters suitable for use with prokaryotic hosts include the β-lactamase and lactose promoter systems [Chang et al., Nature, 275:615 (1978); Goeddel et al., Nature, 281:544 (1979)], alkaline phosphatase, a tryptophan (trp) promoter system [Goeddel, Nucleic Acids Res., 8:4057 (1980); EP 36,776], and hybrid promoters such as the tac promoter [deBoer et al., Proc. Natl. Acad. Sci. USA, 80:21-25 (1983)]. Promoters for use in bacterial systems also will contain a Shine-Dalgarno (S.D.) sequence operably linked to the DNA encoding PRO.

Examples of suitable promoting sequences for use with yeast hosts include the promoters for 3-phosphoglycerate kinase [Hitzeman et al., J. Biol. Chem., 255:2073 (1980)] or other glycolytic enzymes [Hess et al., J. Adv. Enzyme Reg., 7:149 (1968); Holland, Biochemistry, 17:4900 (1978)], such as enolase, glyceraldehyde-3-phosphate dehydrogenase, hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase.

Other yeast promoters, which are inducible promoters having the additional advantage of transcription controlled by growth conditions, are the promoter regions for alcohol dehydrogenase 2, isocytochrome C, acid phosphatase, degradative enzymes associated with nitrogen metabolism, metallothionein, glyceraldehyde-3-phosphate dehydrogenase, and enzymes responsible for maltose and galactose utilization. Suitable vectors and promoters for use in yeast expression are further described in EP 73,657.

PRO transcription from vectors in mammalian host cells is controlled, for example, by promoters obtained from the gcnomes of viruses such as polyoma virus, fowlpox virus (UK 2,211,504 published 5 July 1989), adenovirus (such as Adenovirus 2), bovine papilloma virus, avian sarcoma virus, cytomegalovirus, a retrovirus, hepatitis-B virus and Simian Virus 40 (SV40), from heterologous mammalian promoters, e.g., the actin promoter or an immunoglobulin promoter, and from heat-shock promoters, provided such promoters are compatible with the host cell systems.

Transcription of a DNA encoding the PRO by higher eukaryotes may be increased by inserting an enhancer sequence into the vector. Enhancers are cis-acting elements of DNA, usually about from 10 to 300 bp, that act on a promoter to increase its transcription. Many enhancer sequences are now known from mammalian genes (globin, elastase, albumin, α-fetoprotein, and insulin). Typically, however, one will use an enhancer from a eukaryotic cell virus. Examples include the SV40 enhancer on the late side of the replication origin (bp 100-270), the cytomegalovirus early promoter enhancer, the polyoma enhancer on the late side of the replication origin, and adenovirus enhancers. The enhancer may be spliced into the vector at a position 5' or 3' to the PRO coding sequence, but is preferably located at a site 5' from the promoter.

Expression vectors used in eukaryotic host cells (yeast, fungi, insect, plant, animal, human, or nucleated cells from other multicellular organisms) will also contain sequences necessary for the termination of transcription and for stabilizing the mRNA. Such sequences are commonly available from the 5' and, occasionally 3', untranslated regions of eukaryotic or viral DNAs or cDNAs. These regions contain nucleotide segments transcribed as polyadenylated fragments in the untranslated portion of the mRNA encoding PRO.

Still other methods, vectors, and host cells suitable for adaptation to the synthesis of PRO in recombinant vertebrate cell culture are described in Gething et al., Nature, 293:620-625 (1981); Mantei et al., Nature, 281:40-46 (1979); EP 117,060; and EP 117,058.

### 4. Detecting Gene Amplification/Expression

Gene amplification and/or expression may be measured in a sample directly, for example, by conventional Southern blotting, Northern blotting to quantitate the transcription of mRNA [Thomas, Proc. Natl. Acad. Sci. USA, 77:5201-5205 (1980)], dot blotting (DNA analysis), or *in situ* hybridization, using an appropriately labeled probe, based on the sequences provided herein. Alternatively, antibodies may be employed that can recognize specific duplexes, including DNA duplexes, RNA duplexes, and DNA-RNA hybrid duplexes or DNA-protein duplexes. The antibodies in turn may be labeled and the assay may be carried out where the duplex is bound to a surface, so that upon the formation of duplex on the surface, the presence of antibody bound to the duplex can be detected.

Gene expression, alternatively, may be measured by immunological methods, such as immunohistochemical staining of cells or tissue sections and assay of cell culture or body fluids, to quantitate directly the expression of gene product. Antibodies useful for immunohistochemical staining and/or assay of sample fluids may be either monoclonal or polyclonal, and may be prepared in any mammal. Conveniently, the antibodies may be prepared against a native sequence PRO polypeptide or against a synthetic peptide based on the DNA sequences provided herein or against exogenous sequence fused to PRO DNA and encoding a specific antibody epitope.

### 5. Purification of Polypeptide

Forms of PRO may be recovered from culture medium or from host cell lysates. If membrane-bound, it can be released from the membrane using a suitable detergent solution (e.g. Triton-X 100) or by enzymatic cleavage. Cells employed in expression of PRO can be disrupted by various physical or chemical means, such as freeze-thaw cycling, sonication, mechanical disruption, or cell lysing agents.

It may be desired to purify PRO from recombinant cell proteins or polypeptides. The following procedures are exemplary of suitable purification procedures: by fractionation on an ion-exchange column; ethanol precipitation; reverse phase HPLC; chromatography on silica or on a cation-exchange resin such as DEAE; chromatofocusing; SDS-PAGE; ammonium sulfate precipitation; gel filtration using, for example, Sephadex G-75; protein A Sepharose columns to remove contaminants such as IgG; and metal chelating columns to bind epitope-tagged forms of the PRO. Various methods of protein purification may be employed and such methods are known in the art and described for example in Deutscher, Methods in Enzymology, 182 (1990); Scopes, Protein Purification: Principles and Practice, Springer-Verlag, New York (1982). The purification step(s) selected will depend, for example, on the nature of the production process used and the particular PRO produced.

### E. Tissue Distribution

The location of tissues expressing the PRO can be identified by determining mRNA expression in various human tissues. The location of such genes provides information about which tissues are most likely to be affected by the stimulating and inhibiting activities of the PRO polypeptides. The location of a gene in a specific tissue also provides sample tissue for the activity blocking assays discussed below.

As noted before, gene expression in various tissues may be measured by conventional Southern blotting, Northern blotting to quantitate the transcription of mRNA (Thomas, Proc. Natl. Acad. Sci. USA, 77:5201-5205 [1980]), dot blotting (DNA analysis), or *in situ* hybridization, using an appropriately labeled probe, based on the sequences provided herein. Alternatively, antibodies may be employed that can recognize specific duplexes, including DNA duplexes, RNA duplexes, and DNA-RNA hybrid duplexes or DNA-protein duplexes.

Gene expression in various tissues, alternatively, may be measured by immunological methods, such as immunohistochemical staining of tissue sections and assay of cell culture or body fluids, to quantitate directly the expression of gene product. Antibodies useful for immunohistochemical staining and/or assay of sample fluids may be either monoclonal or polyclonal, and may be prepared in any mammal. Conveniently, the antibodies may be prepared against a native sequence of a PRO polypeptide or against a synthetic peptide based on the DNA sequences encoding the PRO polypeptide or against an exogenous sequence fused to a DNA encoding a PRO polypeptide and encoding a specific antibody epitope. General techniques for generating antibodies, and special protocols for Northern blotting and *in situ* hybridization are provided below.

### F. Antibody Binding Studies

The activity of the PRO polypeptides can be further verified by antibody binding studies, in which the ability of anti-PRO antibodies to inhibit the effect of the PRO polypeptides, respectively, on tissue cells is tested. Exemplary antibodies include polyclonal, monoclonal, humanized, bispecific, and heteroconjugate antibodies, the preparation of which will be described hereinbelow.

Antibody binding studies may be carried out in any known assay method, such as competitive binding assays, direct and indirect sandwich assays, and immunoprecipitation assays. Zola, Monoclonal Antibodies: A Manual of Techniques, pp.147-158 (CRC Press, Inc., 1987).

Competitive binding assays rely on the ability of a labeled standard to compete with the test sample analyte for binding with a limited amount of antibody. The amount of target protein in the test sample is inversely proportional to the amount of standard that becomes bound to the antibodies. To facilitate determining the amount of standard that becomes bound, the antibodies preferably are insolubilized before or after the competition, so that the standard and analyte that are bound to the antibodies may conveniently be separated from the standard and analyte which remain unbound.

Sandwich assays involve the use of two antibodies, each capable of binding to a different immunogenic portion, or epitope, of the protein to be detected. In a sandwich assay, the test sample analyte is bound by a first antibody which is immobilized on a solid support, and thereafter a second antibody binds to the analyte, thus forming an insoluble three-part complex. See, *e.g*., US Pat No. 4,376,110. The second antibody may itself be labeled with a detectable moiety (direct sandwich assays) or may be measured using an anti-immunoglobulin antibody that is labeled with a detectable moiety (indirect sandwich assay). For example, one type of sandwich assay is an ELISA assay, in which case the detectable moiety is an enzyme.

For immunohistochemistry, the tissue sample may be fresh or frozen or may be embedded in paraffin and fixed with a preservative such as formalin, for example.

### G. Cell-Based Assays

Cell-based assays and animal models for immune related diseases can be used to further understand the relationship between the genes and polypeptides identified herein and the development and pathogenesis of immune related disease.

In a different approach, cells of a cell type known to be involved in a particular immune related disease are transfected with the cDNAs described herein, and the ability of these cDNAs to stimulate or inhibit immune function is analyzed. Suitable cells can be transfected with the desired gene, and monitored for immune function activity. Such transfected cell lines can then be used to test the ability of poly- or monoclonal antibodies or antibody compositions to inhibit or stimulate immune function, for example to modulate B-cell proliferation or Ig production. Cells transfected with the coding sequences of the genes identified herein can further be used to identify drug candidates for the treatment of immune related diseases.

In addition, primary cultures derived from transgenic animals (as described below) can be used in the cell-based assays herein, although stable cell lines are preferred. Techniques to derive continuous cell lines from transgenic animals are well known in the art (see, *e.g.*, Small et al., Mol. Cell. Biol. 5: 642-648 [1985]).

A cell based assay for B cells involves incubation of B cells with test polypeptides thought to be inhibitory of IgE production. The amount of inhibition by test polypeptides is compared with IgE production of B cells inhibited by E25 antibody. Human primary PBMCs (1 x 10e6 cell/mL- 1mL final) are isolated and incubated at 37°C. On Day 1, PMBCs (500 ul- 2 x 10e6/mL) in assay medium containing IL-4 [20 ng/mL] and anti-CD40 [100 ng/mL] are combined with 500 ul test polypeptide (2X desired final concentration) into wells. Currently assay is 24 well with a 1 mL volume. Media is PSO4 with 15% horse serum (Intergen, Atlanta GA), 100 units/mL penicillin with 100 mg/mL streptomycin (Gibco, Gaithersburg MD), and 200 mM glutamine On Day 14 cells are centrifuged and supernatant removed for quantitation of IgE. The quantity of IgE is determined by ELISA. A test polypeptide is considered positive if IgE synthesis is decreased by greater than 50% and/or 50% of maximum inhibition by E25. The test polypeptides are run in singlet and the IgE ELISA is run in duplicate for each well.

On the other hand, PRO polypeptides, as well as other compounds of the invention, which are direct inhibitors of B cell proliferation/activation and/or Ig secretion can be directly used to suppress the immune response. These compounds are useful to reduce the degree of the immune response and to treat immune related diseases characterized by a hyperactive, superoptimal, or autoimmune response. The use of compound which suppress Ig production would be expected to reduce inflammation. Such uses would be beneficial in treating conditions associated with excessive inflammation.

Alternatively, compounds, *e.g.*, antibodies, which bind to stimulating PRO polypeptides and block the stimulating effect of these molecules produce a net inhibitory effect and can be used to suppress the B cell mediated immune response by inhibiting B cell proliferation/activation, lymphokine secretion and/or Ig secretion. Blocking the stimulating effect of the polypeptides suppresses the immune response of the mammal.

### H. Animal Models

The results of cell based in vitro assays can be further verified using *in vivo* animal models and assays for B-cell function. A variety of well known animal models can be used to further understand the role of the genes identified herein in the development and pathogenesis of immune related disease, and to test the efficacy of candidate therapeutic agents, including antibodies, and other antagonists of the native polypeptides, including small molecule antagonists. The *in vivo* nature of such models makes them predictive of responses in human patients. Animal models of immune related diseases include both non-recombinant and recombinant (transgenic) animals. Non-recombinant animal models include, for example, rodent, *e.g*., murine models. Such models can be generated by introducing cells into syngeneic mice using standard techniques, *e.g*., subcutaneous injection, tail vein injection, spleen implantation, intraperitoneal implantation, implantation under the renal capsule, *etc.*

An animal model of Systemic Lupus Erythematosus (SLE) was developed specifically for studying this disease. The NZB mouse was the first strain to be described and is the one most like SLE. Female NZB mice develop kidney lesions and hemolytic anemia and produce anti-DNA antibodies, much like SLE in humans. The B cells of these mice are extremely responsive to antigens and cytokines and this abnormal sensitivity has been proposed for the immunologic aberancy in these mice.

Recombinant (transgenic) animal models can be engineered by introducing the coding portion of the genes identified herein into the genome of animals of interest, using standard techniques for producing transgenic animals. Animals that can serve as a target for transgenic manipulation include, without limitation, mice, rats, rabbits, guinea pigs, sheep, goats, pigs, and non-human primates, *e.g*., baboons, chimpanzees and monkeys. Techniques known in the art to introduce a transgene into such animals include pronucleic microinjection (Hoppe and Wanger, U.S. Patent No. 4,873,191); retrovirus-mediated gene transfer into germ lines (*e.g.,* Van der Putten et al., Proc. Natl. Acad. Sci. USA 82, 6148-615 [1985]); gene targeting in embryonic stem cells (Thompson et al., Cell 56, 313-321 [1989]); electroporation of embryos (Lo, Mol. Cel. Biol. 3, 1803-1814 [1983]); sperm-mediated gene transfer (Lavitrano et al., Cell 57, 717-73 [1989]). For review, see, for example, U.S. Patent No. 4,736,866.

For the purpose of the present invention, transgenic animals include those that carry the transgene only in part of their cells ("mosaic animals"). The transgene can be integrated either as a single transgene, or in concatamers, e.g., head-to-head or head-to-tail tandems. Selective introduction of a transgene into a particular cell type is also possible by following, for example, the technique of Lasko et al., Proc. Natl. Acad. Sci. USA 89, 6232-636 (1992).

The expression of the transgene in transgenic animals can be monitored by standard techniques. For example, Southern blot analysis or PCR amplification can be used to verify the integration of the transgene. The level of mRNA expression can then be analyzed using techniques such as *in situ* hybridization, Northern blot analysis, PCR, or immunocytochemistry.

The animals may be further examined for signs of immune disease pathology, for example by histological examination to determine infiltration of immune cells into specific tissues. Blocking experiments can also be performed in which the transgenic animals are treated with the compounds of the invention to determine the extent of the B cell proliferation, stimulation or inhibition of the compounds. In these experiments, blocking antibodies which bind to the PRO polypeptide, prepared as described above, are administered to the animal and the effect on immune function is determined.

Alternatively, "knock out" animals can be constructed which have a defective or altered gene encoding a polypeptide identified herein, as a result of homologous recombination between the endogenous gene encoding the polypeptide and altered genomic DNA encoding the same polypeptide introduced into an embryonic cell of the animal. For example, cDNA encoding a particular polypeptide can be used to clone genomic DNA encoding that polypeptide in accordance with established techniques. A portion of the genomic DNA encoding a particular polypeptide can be deleted or replaced with another gene, such as a gene encoding a selectable marker which can be used to monitor integration. Typically, several kilobases of unaltered flanking DNA (both at the 5' and 3' ends) are included in the vector [see *e.g.,* Thomas and Capecchi, Cell, 51:503 (1987) for a description of homologous recombination vectors]. The vector is introduced into an embryonic stem cell line (*e.g.,* by electroporation) and cells in which the introduced DNA has homologously recombined with the endogenous DNA are selected [see *e.g.,* Li et al., Cell, 69:915 (1992)]. The selected cells are then injected into a blastocyst of an animal (*e.g.,* a mouse or rat) to form aggregation chimeras [see *e.g*., Bradley, in Teratocarcinomas and Embryonic Stem Cells: A Practical Approach, E. J. Robertson, ed. (IRL, Oxford, 1987), pp. 113-152]. A chimeric embryo can then be implanted into a suitable pseudopregnant female foster animal and the embryo brought to term to create a "knock out" animal. Progeny harboring the homologously recombined DNA in their germ cells can be identified by standard techniques and used to breed animals in which all cells of the animal contain the homologously recombined DNA. Knockout animals can be characterized for instance, for their ability to defend against certain pathological conditions and for their development of pathological conditions due to absence of the polypeptide.

### I. Screening Assays for Drug Candidates

Screening assays for drug candidates are designed to identify compounds that bind to or complex with the polypeptides encoded by the genes identified herein or a biologically active fragment thereof, or otherwise interfere with the interaction of the encoded polypeptides with other cellular proteins. Such screening assays will include assays amenable to high-throughput screening of chemical libraries, making them particularly suitable for identifying small molecule drug candidates. Small molecules contemplated include synthetic organic or inorganic compounds, including peptides, preferably soluble peptides, (poly)peptide-immunoglobulin fusions, and, in particular, antibodies including, without limitation, poly- and monoclonal antibodies and antibody fragments, single-chain antibodies, anti-idiotypic antibodies, and chimeric or humanized versions of such antibodies or fragments, as well as human antibodies and antibody fragments. The assays can be performed in a variety of formats, including protein-protein binding assays, biochemical screening assays, immunoassays and cell based assays, which are well characterized in the art. All assays are common in that they call for contacting the drug candidate with a polypeptide encoded by a nucleic acid identified herein under conditions and for a time sufficient to allow these two components to interact.

In binding assays, the interaction is binding and the complex formed can be isolated or detected in the reaction mixture. In a particular embodiment, the polypeptide encoded by the gene identified herein or the drug candidate is immobilized on a solid phase, *e.g*., on a microtiter plate, by covalent or non-covalent attachments. Non-covalent attachment generally is accomplished by coating the solid surface with a solution of the polypeptide and drying. Alternatively, an immobilized antibody, *e.g.,* a monoclonal antibody, specific for the polypeptide to be immobilized can be used to anchor it to a solid surface. The assay is performed by adding the non-immobilized component, which may be labeled by a detectable label, to the immobilized component, *e.g*., the coated surface containing the anchored component. When the reaction is complete, the non-reacted components are removed, *e.g.,* by washing, and complexes anchored on the solid surface are detected. When the originally non-immobilized component carries a detectable label, the detection of label immobilized on the surface indicates that complexing occurred. Where the originally non-immobilized component does not carry a label, complexing can be detected, for example, by using a labelled antibody specifically binding the immobilized complex.

If the candidate compound interacts with but does not bind to a particular protein encoded by a gene identified herein, its interaction with that protein can be assayed by methods well known for detecting protein-protein interactions. Such assays include traditional approaches, such as, cross-linking, co-immunoprecipitation, and co-purification through gradients or chromatographic columns. In addition, protein-protein interactions can be monitored by using a yeast-based genetic system described by Fields and co-workers [Fields and Song, Nature (London) 340, 245-246 (1989); Chien et al., Proc. Natl. Acad. Sci. USA 88, 9578-9582 (1991)] as disclosed by Chevray and Nathans, Proc. Natl. Acad. Sci. USA 89, 5789-5793 (1991). Many transcriptional activators, such as yeast GAL4, consist of two physically discrete modular domains, one acting as the DNA-binding domain, while the other one functioning as the transcription activation domain. The yeast expression system described in the foregoing publications (generally referred to as the "two-hybrid system") takes advantage of this property, and employs two hybrid proteins, one in which the target protein is fused to the DNA-binding domain of GAL4, and another, in which candidate activating proteins are fused to the activation domain. The expression of a GAL1*-lac*Z reporter gene under control of a GAL4-activated promoter depends on reconstitution of GAL4 activity via protein-protein interaction. Colonies containing interacting polypeptides are detected with a chromogenic substrate for β-galactosidase. A complete kit (MATCHMAKER^{™}) for identifying protein-protein interactions between two specific proteins using the two-hybrid technique is commercially available from Clontech. This system can also be extended to map protein domains involved in specific protein interactions as well as to pinpoint amino acid residues that are crucial for these interactions.

In order to find compounds that interfere with the interaction of a gene identified herein and other intra- or extracellular components can be tested, a reaction mixture is usually prepared containing the product of the gene and the intra- or extracellular component under conditions and for a time allowing for the interaction and binding of the two products. To test the ability of a test compound to inhibit binding, the reaction is run in the absence and in the presence of the test compound. In addition, a placebo may be added to a third reaction mixture, to serve as positive control. The binding (complex formation) between the test compound and the intra- or extracellular component present in the mixture is monitored as described above. The formation of a complex in the control reaction(s) but not in the reaction mixture containing the test compound indicates that the test compound interferes with the interaction of the test compound and its reaction partner.

### J. Compositions and Methods for the Treatment of Immune Related Diseases

The compositions useful in the treatment of immune related diseases include, without limitation, proteins, antibodies, small organic molecules, peptides, phosphopeptides, antisense and ribozyme molecules, triple helix molecules, *etc.* that inhibit or stimulate immune function, for example, B cell proliferation/activation, lymphokine release, or Ig production.

For example, antisense RNA and RNA molecules act to directly block the translation of mRNA by hybridizing to targeted mRNA and preventing protein translation. When antisense DNA is used, oligodeoxyribonucleotides derived from the translation initiation site, *e.g*., between about -10 and + 10 positions of the target gene nucleotide sequence, are preferred.

Ribozymes are enzymatic RNA molecules capable of catalyzing the specific cleavage of RNA. Ribozymes act by sequence-specific hybridization to the complementary target RNA, followed by endonucleolytic cleavage. Specific ribozyme cleavage sites within a potential RNA target can be identified by known techniques. For further details see, *e.g*., Rossi, Current Biology 4, 469-471 (1994), and PCT publication No. WO 97/33551 (published September 18, 1997).

Nucleic acid molecules in triple helix formation used to inhibit transcription should be single-stranded and composed of deoxynucleotides. The base composition of these oligonucleotides is designed such that it promotes triple helix formation via Hoogsteen base pairing rules, which generally require sizeable stretches of purines or pyrimidines on one strand of a duplex. For further details see, *e.g.,* PCT publication No. WO 97/33551, *supra.*

These molecules can be identified by any or any combination of the screening assays discussed above and/or by any other screening techniques well known for those skilled in the art.

### K. Anti-PRO Antibodies

The present invention further provides anti-PRO antibodies. Exemplary antibodies include polyclonal, monoclonal, humanized, bispecific, and heteroconjugate antibodies.

### 1. Polyclonal Antibodies

The anti-PRO antibodies may comprise polyclonal antibodies. Methods of preparing polyclonal antibodies are known to the skilled artisan. Polyclonal antibodies can be raised in a mammal, for example, by one or more injections of an immunizing agent and, if desired, an adjuvant. Typically, the immunizing agent and/or adjuvant will be injected in the mammal by multiple subcutaneous or intraperitoneal injections. The immunizing agent may include the PRO polypeptide or a fusion protein thereof. It may be useful to conjugate the immunizing agent to a protein known to be immunogenic in the mammal being immunized. Examples of such immunogenic proteins include but are not limited to keyhole limpet hemocyanin, serum albumin, bovine thyroglobulin, and soybean trypsin inhibitor. Examples of adjuvants which may be employed include Freund's complete adjuvant and MPL-TDM adjuvant (monophosphoryl Lipid A, synthetic trehalose dicorynomycolate). The immunization protocol may be selected by one skilled in the art without undue experimentation.

### 2. Monoclonal Antibodies

The anti-PRO antibodies may, alternatively, be monoclonal antibodies. Monoclonal antibodies may be prepared using hybridoma methods, such as those described by Kohler and Milstein, Nature, 256:495 (1975). In a hybridoma method, a mouse, hamster, or other appropriate host animal, is typically immunized with an immunizing agent to elicit lymphocytes that produce or are capable of producing antibodies that will specifically bind to the immunizing agent. Alternatively, the lymphocytes may be immunized *in vitro.*

The immunizing agent will typically include the PRO polypeptide or a fusion protein thereof. Generally, either peripheral blood lymphocytes ("PBLs") are used if cells of human origin are desired, or spleen cells or lymph node cells are used if non-human mammalian sources are desired. The lymphocytes are then fused with an immortalized cell line using a suitable fusing agent, such as polyethylene glycol, to form a hybridoma cell [Goding, Monoclonal Antibodies: Principles and Practice, Academic Press, (1986) pp. 59-103]. Immortalized cell lines are usually transformed mammalian cells, particularly myeloma cells of rodent, bovine and human origin. Usually, rat or mouse myeloma cell lines are employed. The hybridoma cells may be cultured in a suitable culture medium that preferably contains one or more substances that inhibit the growth or survival of the unfused, immortalized cells. For example, if the parental cells lack the enzyme hypoxanthine guanine phosphoribosyl transferase (HGPRT or HPRT), the culture medium for the hybridomas typically will include hypoxanthine, aminopterin, and thymidine ("HAT medium"), which substances prevent the growth of HGPRT-deficient cells.

Preferred immortalized cell lines are those that fuse efficiently, support stable high level expression of antibody by the selected antibody-producing cells, and are sensitive to a medium such as HAT medium. More preferred immortalized cell lines are murine myeloma lines, which can be obtained, for instance, from the Salk Institute Cell Distribution Center, San Diego, California and the American Type Culture Collection, Manassas, Virginia. Human myeloma and mouse-human heteromyeloma cell lines also have been described for the production of human monoclonal antibodies [Kozbor, J. Immunol., 133:3001 (1984); Brodeur et al., Monoclonal Antibody Production Techniques and Applications, Marcel Dekker, Inc., New York, (1987) pp. 51-63].

The culture medium in which the hybridoma cells are cultured can then be assayed for the presence of monoclonal antibodies directed against PRO. Preferably, the binding specificity of monoclonal antibodies produced by the hybridoma cells is determined by immunoprecipitation or by an *in vitro* binding assay, such as radioimmunoassay (RIA) or enzyme-linked immunoabsorbent assay (ELISA). Such techniques and assays are known in the art. The binding affinity of the monoclonal antibody can, for example, be determined by the Scatchard analysis of Munson and Pollard, Anal. Biochem., 107:220 (1980).

After the desired hybridoma cells are identified, the clones may be subcloned by limiting dilution procedures and grown by standard methods [Goding, supra]. Suitable culture media for this purpose include, for example, Dulbecco's Modified Eagle's Medium and RPMI-1640 medium. Alternatively, the hybridoma cells may be grown *in vivo* as ascites in a mammal.

The monoclonal antibodies secreted by the subclones may be isolated or purified from the culture medium or ascites fluid by conventional immunoglobulin purification procedures such as, for example, protein A-Sepharose, hydroxylapatite chromatography, gel electrophoresis, dialysis, or affinity chromatography.

The monoclonal antibodies may also be made by recombinant DNA methods, such as those described in U.S. Patent No. 4,816,567. DNA encoding the monoclonal antibodies of the invention can be readily isolated and sequenced using conventional procedures (e.g., by using oligonucleotide probes that are capable of binding specifically to genes encoding the heavy and light chains of murine antibodies). The hybridoma cells of the invention serve as a preferred source of such DNA. Once isolated, the DNA may be placed into expression vectors, which are then transfected into host cells such as simian COS cells, Chinese hamster ovary (CHO) cells, or myeloma cells that do not otherwise produce immunoglobulin protein, to obtain the synthesis of monoclonal antibodies in the recombinant host cells. The DNA also may be modified, for example, by substituting the coding sequence for human heavy and light chain constant domains in place of the homologous murine sequences [U.S. Patent No. 4,816,567; Morrison et al., supra] or by covalently joining to the immunoglobulin coding sequence all or part of the coding sequence for a non-immunoglobulin polypeptide. Such a non-immunoglobulin polypeptide can be substituted for the constant domains of an antibody of the invention, or can be substituted for the variable domains of one antigen-combining site of an antibody of the invention to create a chimeric bivalent antibody.

The antibodies may be monovalent antibodies. Methods for preparing monovalent antibodies are well known in the art. For example, one method involves recombinant expression of immunoglobulin light chain and modified heavy chain. The heavy chain is truncated generally at any point in the Fc region so as to prevent heavy chain crosslinking. Alternatively, the relevant cysteine residues are substituted with another amino acid residue or are deleted so as to prevent crosslinking.

*In vitro* methods are also suitable for preparing monovalent antibodies. Digestion of antibodies to produce fragments thereof, particularly, Fab fragments, can be accomplished using routine techniques known in the art.

### 3. Human and Humanized Antibodies

The anti-PRO antibodies of the invention may further comprise humanized antibodies or human antibodies. Humanized forms of non-human (e.g., murine) antibodies are chimeric immunoglobulins, immunoglobulin chains or fragments thereof (such as Fv, Fab, Fab', F(ab')₂ or other antigen-binding subsequences of antibodies) which contain minimal sequence derived from non-human immunoglobulin. Humanized antibodies include human immunoglobulins (recipient antibody) in which residues from a complementary determining region (CDR) of the recipient are replaced by residues from a CDR of a non-human species (donor antibody) such as mouse, rat or rabbit having the desired specificity, affinity and capacity. In some instances, Fv framework residues of the human immunoglobulin are replaced by corresponding non-human residues. Humanized antibodies may also comprise residues which are found neither in the recipient antibody nor in the imported CDR or framework sequences. In general, the humanized antibody will comprise substantially all of at least one, and typically two, variable domains, in which all or substantially all of the CDR regions correspond to those of a non-human immunoglobulin and all or substantially all of the FR regions are those of a human immunoglobulin consensus sequence. The humanized antibody optimally also will comprise at least a portion of an immunoglobulin constant region (Fc), typically that of a human immunoglobulin [Jones et al., Nature, 321:522-525 (1986); Riechmann et al., Nature, 332:323-329 (1988); and Presta, Curr. Op. Struct. Biol., 2:593-596 (1992)].

Methods for humanizing non-human antibodies are well known in the art. Generally, a humanized antibody has one or more amino acid residues introduced into it from a source which is non-human. These non-human amino acid residues are often referred to as "import" residues, which are typically taken from an "import" variable domain. Humanization can be essentially performed following the method of Winter and co-workers [Jones et al., Nature, 321:522-525 (1986); Riechmann et al., Nature, 332:323-327 (1988); Verhoeyen et al., Science, 239:1534-1536 (1988)], by substituting rodent CDRs or CDR sequences for the corresponding sequences of a human antibody. Accordingly, such "humanized" antibodies are chimeric antibodies (U.S. Patent No. 4,816,567), wherein substantially less than an intact human variable domain has been substituted by the corresponding sequence from a non-human species. In practice, humanized antibodies are typically human antibodies in which some CDR residues and possibly some FR residues are substituted by residues from analogous sites in rodent antibodies.

Human antibodies can also be produced using various techniques known in the art, including phage display libraries [Hoogenboom and Winter, J. Mol. Biol., 227:381 (1991); Marks et al., J. Mol. Biol., 222:581 (1991)]. The techniques of Cole et al. and Boerner et al. are also available for the preparation of human monoclonal antibodies (Cole et al., Monoclonal Antibodies and Cancer Therapy, Alan R. Liss, p. 77 (1985) and Boerner et al., J. Immunol., 147(1):86-95 (1991)]. Similarly, human antibodies can be made by introducing of human immunoglobulin loci into transgenic animals, e.g., mice in which the endogenous immunoglobulin genes have been partially or completely inactivated. Upon challenge, human antibody production is observed, which closely resembles that seen in humans in all respects, including gene rearrangement, assembly, and antibody repertoire. This approach is described, for example, in U.S. Patent Nos. 5,545,807; 5,545,806; 5,569,825; 5,625,126; 5,633,425; 5,661,016, and in the following scientific publications: Marks et al., Bio/Technology 10, 779-783 (1992); Lonberg et al., Nature 368 856-859 (1994); Morrison, Nature 368, 812-13 (1994); Fishwild et al., Nature Biotechnology 14, 845-51 (1996); Neuberger, Nature Biotechnology 14, 826 (1996); Lonberg and Huszar, Intern. Rev. Immunol. 13 65-93 (1995).

The antibodies may also be affinity matured using known selection and/or mutagenesis methods as described above. Preferred affinity matured antibodies have an affinity which is five times, more preferably 10 times, even more preferably 20 or 30 times greater than the starting antibody (generally murine, humanized or human) from which the matured antibody is prepared.

### 4. Bispecific Antibodies

Bispecific antibodies are monoclonal, preferably human or humanized, antibodies that have binding specificities for at least two different antigens. In the present case, one of the binding specificities is for the PRO, the other one is for any other antigen, and preferably for a cell-surface protein or receptor or receptor subunit.

Methods for making bispecific antibodies are known in the art. Traditionally, the recombinant production of bispecific antibodies is based on the co-expression of two immunoglobulin heavy-chain/light-chain pairs, where the two heavy chains have different specificities [Milstein and Cuello, Nature, 305:537-539 (1983)]. Because of the random assortment of immunoglobulin heavy and light chains, these hybridomas (quadromas) produce a potential mixture of ten different antibody molecules, of which only one has the correct bispecific structure. The purification of the correct molecule is usually accomplished by affinity chromatography steps. Similar procedures are disclosed in WO 93/08829, published 13 May 1993, and in Traunecker et al., EMBO J., 10:3655-3659 (1991).

Antibody variable domains with the desired binding specificities (antibody-antigen combining sites) can be fused to immunoglobulin constant domain sequences. The fusion preferably is with an immunoglobulin heavy-chain constant domain, comprising at least part of the hinge, CH2, and CH3 regions. It is preferred to have the first heavy-chain constant region (CH1) containing the site necessary for light-chain binding present in at least one of the fusions. DNAs encoding the immunoglobulin heavy-chain fusions and, if desired, the immunoglobulin light chain, are inserted into separate expression vectors, and are co-transfected into a suitable host organism. For further details of generating bispecific antibodies see, for example, Suresh et al., Methods in Enzymology, 121:210 (1986).

According to another approach described in WO 96/27011, the interface between a pair of antibody molecules can be engineered to maximize the percentage of heterodimers which are recovered from recombinant cell culture. The preferred interface comprises at least a part of the CH3 region of an antibody constant domain. In this method, one or more small amino acid side chains from the interface of the first antibody molecule are replaced with larger side chains (e.g. tyrosine or tryptophan). Compensatory "cavities" of identical or similar size to the large side chain(s) are created on the interface of the second antibody molecule by replacing large amino acid side chains with smaller ones (e.g. alanine or threonine). This provides a mechanism for increasing the yield of the heterodimer over other unwanted end-products such as homodimers.

Bispecific antibodies can be prepared as full length antibodies or antibody fragments (e.g. F(ab')₂ bispecific antibodies). Techniques for generating bispecific antibodies from antibody fragments have been described in the literature. For example, bispecific antibodies can be prepared can be prepared using chemical linkage. Brennan et al., Science 229:81 (1985) describe a procedure wherein intact antibodies are proteolytically cleaved to generate F(ab')₂ fragments. These fragments are reduced in the presence of the dithiol complexing agent sodium arsenite to stabilize vicinal dithiols and prevent intermolecular disulfide formation. The Fab' fragments generated are then converted to thionitrobenzoate (TNB) derivatives. One of the Fab'-TNB derivatives is then reconverted to the Fab'-thiol by reduction with mercaptoethylamine and is mixed with an equimolar amount of the other Fab'-TNB derivative to form the bispecific antibody. The bispecific antibodies produced can be used as agents for the selective immobilization of enzymes.

Fab' fragments may be directly recovered from *E. coli* and chemically coupled to form bispecific antibodies. Shalaby et al., J. Exp. Med. 175:217-225 (1992) describe the production of a fully humanized bispecific antibody F(ab')₂ molecule. Each Fab' fragment was separately secreted from *E. coli* and subjected to directed chemical coupling *in vitro* to form the bispecific antibody. The bispecific antibody thus formed was able to bind to cells overexpressing the ErbB2 receptor and normal human T cells, as well as trigger the lytic activity of human cytotoxic lymphocytes against human breast tumor targets.

Various technique for making and isolating bispecific antibody fragments directly from recombinant cell culture have also been described. For example, bispecific antibodies have been produced using leucine zippers. Kostelny et al., J. Immunol. 148(5):1547-1553 (1992). The leucine zipper peptides from the Fos and Jun proteins were linked to the Fab' portions of two different antibodies by gene fusion. The antibody homodimers were reduced at the hinge region to form monomers and then re-oxidized to form the antibody heterodimers. This method can also be utilized for the production of antibody homodimers. The "diabody" technology described by Hollinger et al., Proc. Natl. Acad. Sci. USA 90:6444-6448 (1993) has provided an alternative mechanism for making bispecific antibody fragments. The fragments comprise a heavy-chain variable domain (V_{H}) connected to a light-chain variable domain (V_{L}) by a linker which is too short to allow pairing between the two domains on the same chain. Accordingly, the V_{H} and V_{L} domains of one fragment are forced to pair with the complementary V_{L} and V_{H} domains of another fragment, thereby forming two antigen-binding sites. Another strategy for making bispecific antibody fragments by the use of single-chain Fv (sFv) dimers has also been reported. See, Gruber et al., J. Immunol. 152:5368 (1994).

Antibodies with more than two valencies are contemplated. For example, trispecific antibodies can be prepared. Tutt et al., J. Immunol. 147:60 (1991).

Exemplary bispecific antibodies may bind to two different epitopes on a given PRO polypeptide herein. Alternatively, an anti-PRO polypeptide arm may be combined with an arm which binds to a triggering molecule on a leukocyte such as a T-cell receptor molecule (e.g. CD2, CD3, CD28, or B7), or Fc receptors for IgG (FcγR), such as FcγRI (CD64), FcγRII (CD32) and FcγRIII (CD16) so as to focus cellular defense mechanisms to the cell expressing the particular PRO polypeptide. Bispecific antibodies may also be used to localize cytotoxic agents to cells which express a particular PRO polypeptide. These antibodies possess a PRO-binding arm and an arm which binds a cytotoxic agent or a radionuclide chelator, such as EOTUBE, DPTA, DOTA, or TETA. Another bispecific antibody of interest binds the PRO polypeptide and further binds tissue factor (TF).

### 5. Heteroconiugate Antibodies

Heteroconjugate antibodies are also within the scope of the present invention. Heteroconjugate antibodies are composed of two covalently joined antibodies. Such antibodies have, for example, been proposed to target immune system cells to unwanted cells [U.S. Patent No. 4,676,980], and for treatment of HIV infection [WO 91/00360; WO 92/200373; EP 03089]. It is contemplated that the antibodies may be prepared *in vitro* using known methods in synthetic protein chemistry, including those involving crosslinking agents. For example, immunotoxins may be constructed using a disulfide exchange reaction or by forming a thioether bond. Examples of suitable reagents for this purpose include iminothiolate and methyl-4-mercaptobutyrimidate and those disclosed, for example, in U.S. Patent No. 4,676,980.

### 6. Effector Function Engineering

It may be desirable to modify the antibody of the invention with respect to effector function, so as to enhance, *e.g*., the effectiveness of the antibody in treating cancer. For example, cysteine residue(s) may be introduced into the Fc region, thereby allowing interchain disulfide bond formation in this region. The homodimeric antibody thus generated may have improved internalization capability and/or increased complement-mediated cell killing and antibody-dependent cellular cytotoxicity (ADCC). See Caron et al., J. Exp Med., 176: 1191-1195 (1992) and Shopes, J. Immunol., 148: 2918-2922 (1992). Homodimeric antibodies with enhanced anti-tumor activity may also be prepared using heterobifunctional cross-linkers as described in Wolff et al. Cancer Research, 53: 2560-2565 (1993). Alternatively, an antibody can be engineered that has dual Fc regions and may thereby have enhanced complement lysis and ADCC capabilities. See Stevenson et al., Anti-Cancer Drug Design, 3: 219-230 (1989).

### 7. Immunoconjugates

The invention also pertains to immunoconjugates comprising an antibody conjugated to a cytotoxic agent such as a chemotherapeutic agent, toxin (*e.g.*, an enzymatically active toxin of bacterial, fungal, plant, or animal origin, or fragments thereof), or a radioactive isotope (*i.e.*, a radioconjugate).

Chemotherapeutic agents useful in the generation of such immunoconjugates have been described above. Enzymatically active toxins and fragments thereof that can be used include diphtheria A chain, nonbinding active fragments of diphtheria toxin, exotoxin A chain (from *Pseudomonas aeruginosa*), ricin A chain, abrin A chain, modeccin A chain, alpha-sarcin, *Aleurites fordii* proteins, dianthin proteins, *Phytolaca americana* proteins (PAPI, PAPII, and PAP-S), momordica charantia inhibitor, curcin, crotin, sapaonaria officinalis inhibitor, gelonin, mitogellin, restrictocin, phenomycin, enomycin, and the tricothecenes. A variety of radionuclides are available for the production of radioconjugated antibodies. Examples include ²¹²Bi, ¹³¹I, ¹³¹In, ⁹⁰Y, and ¹⁸⁶Re.

Conjugates of the antibody and cytotoxic agent are made using a variety of bifunctional protein-coupling agents such as N-succinimidyl-3-(2-pyridyldithiol) propionate (SPDP), iminothiolane (IT), bifunctional derivatives of imidoesters (such as dimethyl adipimidate HCL), active esters (such as disuccinimidyl suberate), aldehydes (such as glutareldehyde), bis-azido compounds (such as bis (p-azidobenzoyl) hexanediamine), bis-diazonium derivatives (such as bis-(p-diazoniumbenzoyl)-ethylenediamine), diisocyanates (such as tolyene 2,6-diisocyanate), and bis-active fluorine compounds (such as 1,5-difluoro-2,4-dinitrobenzene). For example, a ricin immunotoxin can be prepared as described in Vitetta et al., Science, 238: 1098 (1987). Carbon-14-labeled 1-isothiocyanatobenzyl-3-methyldiethylene triaminepentaacetic acid (MX-DTPA) is an exemplary chelating agent for conjugation of radionucleotide to the antibody. See WO94/11026.

In another embodiment, the antibody may be conjugated to a "receptor" (such streptavidin) for utilization in tumor pretargeting wherein the antibody-receptor conjugate is administered to the patient, followed by removal of unbound conjugate from the circulation using a clearing agent and then administration of a "ligand" (*e.g.*, avidin) that is conjugated to a cytotoxic agent (*e.g.*, a radionucleotide).

### 8. Immunoliposomes

The antibodies disclosed herein may also be formulated as immunoliposomes. Liposomes containing the antibody are prepared by methods known in the art, such as described in Epstein et al., Proc. Natl. Acad. Sci. USA 82: 3688 (1985); Hwang et al., Proc. Natl Acad. Sci. USA, 77: 4030 (1980); and U.S. Pat. Nos. 4,485,045 and 4,544,545. Liposomes with enhanced circulation time are disclosed in U.S. Patent No. 5,013,556.

Particularly useful liposomes can be generated by the reverse-phase evaporation method with a lipid composition comprising phosphatidylcholine, cholesterol, and PEG-derivatized phosphatidylethanolamine (PEG-PE). Liposomes are extruded through filters of defined pore size to yield liposomes with the desired diameter. Fab' fragments of the antibody of the present invention can be conjugated to the liposomes as described in Martin et al ., J. Biol. Chem., 257: 286-288 (1982) via a disulfide-interchange reaction. A chemotherapeutic agent (such as Doxorubicin) is optionally contained within the liposome. See Gabizon et al., J. National Cancer Inst., 81(19): 1484 (1989).

### L. Pharmaceutical Compositions

The active PRO molecules of the invention (*e.g.*, PRO polypeptides, anti-PRO antibodies, and/or variants of each) as well as other molecules identified by the screening assays disclosed above, can be administered for the treatment of immune related diseases, in the form of pharmaceutical compositions.

Therapeutic formulations of the active PRO molecule, preferably a polypeptide or antibody of the invention, are prepared for storage by mixing the active molecule having the desired degree of purity with optional pharmaceutically acceptable carriers, excipients or stabilizers (Remington's Pharmaceutical Sciences 16th edition, Osol, A. Ed. [1980]), in the form of lyophilized formulations or aqueous solutions. Acceptable carriers, excipients, or stabilizers are nontoxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g., Zn-protein complexes); and/or non-ionic surfactants such as TWEEN^{™}, PLURONICS^{™} or polyethylene glycol (PEG).

Compounds identified by the screening assays disclosed herein can be formulated in an analogous manner, using standard techniques well known in the art.

Lipofections or liposomes can also be used to deliver the PRO molecule into cells. Where antibody fragments are used, the smallest inhibitory fragment which specifically binds to the binding domain of the target protein is preferred. For example, based upon the variable region sequences of an antibody, peptide molecules can be designed which retain the ability to bind the target protein sequence. Such peptides can be synthesized chemically and/or produced by recombinant DNA technology (see, *e.g.*, Marasco et al., Proc. Natl. Acad. Sci. USA 90, 7889-7893 [1993]).

The formulation herein may also contain more than one active compound as necessary for the particular indication being treated, preferably those with complementary activities that do not adversely affect each other. Alternatively, or in addition, the composition may comprise a cytotoxic agent, cytokine or growth inhibitory agent. Such molecules are suitably present in combination in amounts that are effective for the purpose intended.

The active PRO molecules may also be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nano-particles and nanocapsules) or in macroemulsions. Such techniques are disclosed in Remington's Pharmaceutical Sciences 16th edition, Osol, A. Ed. (1980).

The formulations to be used for *in vivo* administration must be sterile. This is readily accomplished by filtration through sterile filtration membranes.

Sustained-release preparations or the PRO molecules may be prepared. Suitable examples of sustained-release preparations include semipermeable matrices of solid hydrophobic polymers containing the antibody, which matrices are in the form of shaped articles, *e.g.*, films, or microcapsules. Examples of sustained-release matrices include polyesters, hydrogels (for example, poly(2-hydroxyethylmethacrylate), or poly(vinylalcohol)), polylactides (U.S. Pat. No. 3,773,919), copolymers of L-glutamic acid and γ-ethyl-L-glutamate, non-degradable ethylene-vinyl acetate, degradable lactic acid-glycolic acid copolymers such as the LUPRON DEPOT^{™} (injectable microspheres composed of lactic acid-glycolic acid copolymer and leuprolide acetate), and poly-D-(-)-3-hydroxybutyric acid. While polymers such as ethylene-vinyl acetate and lactic acid-glycolic acid enable release of molecules for over 100 days, certain hydrogels release proteins for shorter time periods. When encapsulated antibodies remain in the body for a long time, they may denature or aggregate as a result of exposure to moisture at 37°C, resulting in a loss of biological activity and possible changes in immunogenicity. Rational strategies can be devised for stabilization depending on the mechanism involved. For example, if the aggregation mechanism is discovered to be intermolecular S-S bond formation through thio-disulfide interchange, stabilization may be achieved by modifying sulfhydryl residues, lyophilizing from acidic solutions, controlling moisture content, using appropriate additives, and developing specific polymer matrix compositions.

### M. Methods of Treatment

It is contemplated that the polypeptides, antibodies and other active compounds of the present invention may be used to treat various immune related diseases and conditions, such as B cell mediated diseases, including those characterized by stimulation of B-cell proliferation, inhibition of B-cell proliferation, increased or decreased Ig production or the inhibition thereof.

Exemplary conditions or disorders to be treated with the polypeptides, antibodies and other compounds of the invention, include, but are not limited to: systemic lupus erythematosis, X-linked infantile hypogammaglobulinemia, polysaccaride antigen unresponsiveness, selective IgA deficiency, selective IgM deficiency, selective deficiency of IgG subclasses, immunodeficiency with hyper Ig-M, transient hypogammaglobulinemia of infancy, Burkitt's lymphoma, Intermediate lymphoma, follicular lymphoma, typeII hypersensitivity, rheumatoid arthritis, autoimmune mediated hemolytic anemia, myesthenia gravis, hypoadrenocorticism, glomerulonephritis and ankylosing spondylitis.

In systemic lupus erythematosus (SLE), the central mediator of disease is the production of auto-reactive antibodies to self proteins/tissues and the subsequent generation of immune-mediated inflammation. Antibodies either directly or indirectly mediate tissue injury. Multiple organs and systems that are affected clinically include kidney, lung, musculoskeletal system, mucocutaneous, eye, central nervous system, cardiovascular system, gastrointestinal tract, bone marrow and blood.

In patients with X-linked infantile hypogammaglobulinemia, the B cells have a deficient kinase which leads to a lack of differentiation from the pre-B cell stage. The consequences of this is that these cells do not secrete immunoglobulin. Children with this disease usually show no symptoms until 6 months of age, an age which corresponds to the loss of maternal antibodies. Symptoms consist of pneumonia, meningitis, dermatitis with some instances of arthritis and malabsorption. Treatment at this time involves the use of intravenous gamma globulin replacement therapy.

Other diseases in which intervention of the immune and/or inflammatory response have benefit are infectious disease including but not limited to viral infection (including but not limited to Epstein-Barr virus) which stimulate the proliferation/lg secretion of B-cells can be utilized therapeutically to enhance the immune response to infectious agents, diseases of immunodeficiency (molecules/derivatives/agonists) which stimulate B-cell proliferation/Ig secretion can be utilized therapeutically to enhance the immune response for conditions of inherited, acquired, infectious induced (as in HIV infection), or iatrogenic (i.e., as from chemotherapy) immunodeficiency, and neoplasia.

B-cell leukemias can be treated by antibodies against surface proteins. This is illustrated in a regimen using antibodies to CD9 or CD10 which are often expressed at high levels in B-cell leukemias. Bone marrow is removed from patients with this type of leukemia and is treated with toxin-conjugated anti-CD9/anti-CD10, while the patient is treated with high doses of chemotherapy or radiation therapy. The treated marrow now devoid of leukemic cells, is reintroduced into the patient to repopulate the hematopoeitic lineage.

Additionally, inhibition of molecules with proinflammatory properties may have therapeutic benefit in reperfusion injury; stroke; myocardial infarction; atherosclerosis; acute lung injury; hemorrhagic shock; burn; sepsis/septic shock; acute tubular necrosis; endometriosis; degenerative joint disease and pancreatis.

The compounds of the present invention, *e.g.*, polypeptides or antibodies, are administered to a mammal, preferably a human, in accord with known methods, such as intravenous administration as a bolus or by continuous infusion over a period of time, by intramuscular, intraperitoneal, intracerobrospinal, subcutaneous, intra-articular, intrasynovial, intrathecal, oral, topical, or inhalation (intranasal, intrapulmonary) routes. Intravenous or inhaled administration of polypeptides and antibodies is preferred.

In immunoadjuvant therapy, other therapeutic regimens, such administration of an anti-cancer agent, may be combined with the administration of the proteins, antibodies or compounds of the instant invention. For example, the patient to be treated with a the immunoadjuvant of the invention may also receive an anti-cancer agent (chemotherapeutic agent) or radiation therapy. Preparation and dosing schedules for such chemotherapeutic agents may be used according to manufacturers' instructions or as determined empirically by the skilled practitioner. Preparation and dosing schedules for such chemotherapy are also described in Chemotherapy Service Ed., M.C. Perry, Williams & Wilkins, Baltimore, MD (1992). The chemotherapeutic agent may precede, or follow administration of the immunoadjuvant or may be given simultaneously therewith. Additionally, an anti-estrogen compound such as tamoxifen or an anti-progesterone such as onapristone (see, EP 616812) may be given in dosages known for such molecules.

It may be desirable to also administer antibodies against other immune disease associated or tumor associated antigens, such as antibodies which bind to CD20, CD11a, CD18, ErbB2, EGFR, ErbB3, ErbB4, or vascular endothelial factor (VEGF). Alternatively, or in addition, two or more antibodies binding the same or two or more different antigens disclosed herein may be coadministered to the patient. Sometimes, it may be beneficial to also administer one or more cytokines to the patient. In one embodiment, the PRO polypeptides are coadministered with a growth inhibitory agent. For example, the growth inhibitory agent may be administered first, followed by a PRO polypeptide. However, simultaneous administration or administration first is also contemplated. Suitable dosages for the growth inhibitory agent are those presently used and may be lowered due to the combined action (synergy) of the growth inhibitory agent and the PRO polypeptide.

For the treatment or reduction in the severity of immune related disease, the appropriate dosage of an a compound of the invention will depend on the type of disease to be treated, as defined above, the severity and course of the disease, whether the agent is administered for preventive or therapeutic purposes, previous therapy, the patient's clinical history and response to the compound, and the discretion of the attending physician. The compound is suitably administered to the patient at one time or over a series of treatments.

For example, depending on the type and severity of the disease, about 1 µg/kg to 15 mg/kg (*e.g.*, 0.1-20 mg/kg) of polypeptide or antibody is an initial candidate dosage for administration to the patient, whether, for example, by one or more separate administrations, or by continuous infusion. A typical daily dosage might range from about 1 µg/kg to 100 mg/kg or more, depending on the factors mentioned above. For repeated administrations over several days or longer, depending on the condition, the treatment is sustained until a desired suppression of disease symptoms occurs. However, other dosage regimens may be useful. The progress of this therapy is easily monitored by conventional techniques and assays.

### N. Articles of Manufacture

In another embodiment of the invention, an article of manufacture containing materials (*e.g.*, comprising a PRO molecule) useful for the diagnosis or treatment of the disorders described above is provided. The article of manufacture comprises a container and an instruction. Suitable containers include, for example, bottles, vials, syringes, and test tubes. The containers may be formed from a variety of materials such as glass or plastic. The container holds a composition which is effective for diagnosing or treating the condition and may have a sterile access port (for example the container may be an intravenous solution bag or a vial having a stopper pierceable by a hypodermic injection needle). The active agent in the composition is usually a polypeptide or an antibody of the invention. An instruction or label on, or associated with, the container indicates that the composition is used for diagnosing or treating the condition of choice. The article of manufacture may further comprise a second container comprising a pharmaceutically-acceptable buffer, such as phosphate-buffered saline, Ringer's solution and dextrose solution. It may further include other materials desirable from a commercial and user standpoint, including other buffers, diluents, filters, needles, syringes, and package inserts with instructions for use.

### O. Diagnosis and Prognosis of Immune Related Disease

Cell surface proteins, such as proteins which are overexpressed in certain immune related diseases, are excellent targets for drug candidates or disease treatment. The same proteins along with secreted proteins encoded by the genes amplified in immune related disease states find additional use in the diagnosis and prognosis of these diseases. For example, antibodies directed against the protein products of genes amplified in multiple sclerosis, rheumatoid arthritis, or another immune related disease, can be used as diagnostics or prognostics.

For example, antibodies, including antibody fragments, can be used to qualitatively or quantitatively detect the expression of proteins encoded by amplified or overexpressed genes ("marker gene products"). The antibody preferably is equipped with a detectable, *e.g.*, fluorescent label, and binding can be monitored by light microscopy, flow cytometry, fluorimetry, or other techniques known in the art. These techniques are particularly suitable, if the overexpressed gene encodes a cell surface protein Such binding assays are performed essentially as described above.

*In situ* detection of antibody binding to the marker gene products can be performed, for example, by immunofluorescence or immunoelectron microscopy. For this purpose, a histological specimen is removed from the patient, and a labeled antibody is applied to it, preferably by overlaying the antibody on a biological sample. This procedure also allows for determining the distribution of the marker gene product in the tissue examined. It will be apparent for those skilled in the art that a wide variety of histological methods are readily available for *in situ* detection.

The following examples are offered for illustrative purposes only, and are not intended to limit the scope of the present invention in any way.

All patent and literature references cited in the present specification are hereby incorporated by reference in their entirety.

### EXAMPLES

Commercially available reagents referred to in the examples were used according to manufacturer's instructions unless otherwise indicated. The source of those cells identified in the following examples, and throughout the specification, by ATCC accession numbers is the American Type Culture Collection, Manassas, VA.

### EXAMPLE 1: Microarray analysis of stimulated B-cells

Nucleic acid microarrays, often containing thousands of gene sequences, are useful for identifying differentially expressed genes in diseased tissues as compared to their normal counterparts. Using nucleic acid microarrays, test and control mRNA samples from test and control tissue samples are reverse transcribed and labeled to generate cDNA probes. The cDNA probes are then hybridized to an array of nucleic acids immobilized on a solid support. The array is configured such that the sequence and position of each member of the array is known. For example, a selection of genes known to be expressed in certain disease states may be arrayed on a solid support. Hybridization of a labeled probe with a particular array member indicates that the sample from which the probe was derived expresses that gene. If the hybridization signal of a probe from a test (in this example, stimulated B cells) sample is greater than hybridization signal of a probe from a control (in this instance, non-stimulated B cells) sample, the gene or genes overexpressed in the test tissue are identified. The implication of this result is that an overexpressed protein in a test tissue is useful not only as a diagnostic marker for the presence of the disease condition, but also as a therapeutic target for treatment of the disease condition.

The methodology of hybridization of nucleic acids and microarray technology is well known in the art. In one example, the specific preparation of nucleic acids for hybridization and probes, slides, and hybridization conditions are all detailed in PCT Patent Application Serial No. PCT/US01/10482, filed on March 30, 2001 and which is herein incorporated by reference.

In this experiment, primary B cells were isolated from peripheral blood provided by 3 normal male donors. B cells were isolated by negative selection using the B Cell Isolation Kit with the MACS™ magnetic cell sorting system (Miltenyi Biotec, Auburn CA). The cell purity was determined by fluorescence antibody staining with anti-CD19 vs isotype antibody control and subsequent FACS analysis to determine purity. The purity of the B cell population was above 90% for each donor.

The isolated cells were suspended in RPMI1640 media supplemented with 10% FBS, 2 mM L glutamine, 55 mM 2-ME, 100 units/mL of Penicillin, 100 mg/mL of streptomycin. Cells were cultured at a density of 3 x 10⁵ cells/mL in 5 mL/well in 6 well FALCON™ polystyrene tissue culture plates. Cells were cultured for 23 hours at 37°C either in the presence and absence of anti-CD40 (10mg/mL) and IL-4 (100 ng/mL). The immune competence of the isolated B cells to respond to stimulation by anti-CD40/IL-4 was determined by induction of expression of the cell surface protein, CD69. The increase in expression of CD69 was monitored at a 0 timepoint and 23 hours after culture with anti-CD40/IL-4, using fluorescence staining with anti-CD69 antibodies.

Total RNA was extracted from the cultured B cells at the 0 timepoint and at 23 hours with and without the anti-CD40/IL-4 stimulation using the Qiagen Rneasy Maxi Kit™. The RNA was extracted from columns treated with DNAse I as per Qiagen protocol and eluted using DEPC treated water. The extracted RNA was run on Affimax (Affymetrix Inc. Santa Clara, CA) U95A chips. Non-stimulated cells harvested at the 0 timepoint were subjected to the same analysis. Genes were compared whose expression was upregulated at the 23 hour timepoint in stimulated vs. non-stimulated cells. These genes were also compared to a panel of normal tissues. A normal "universal" tissue control sample was prepared by pooling non-cancerous, human tissues including liver, kidney, and lung. Microarray hybridization experiments using the universal control samples generated a linear plot in a 2-color analysis. The slope of the line generated in a 2-color analysis was then used to normalize the ratios of (test:control detection) within each experiment. The normalized ratios from various experiments were then compared and used to identify clustering of gene expression. Thus, the universal control sample not only allowed effective relative gene expression determinations in a simple 2-sample comparison, it also allowed multi-sample comparisons across several experiments.

Below are the results of these experiments, demonstrating that various PRO polypeptides of the present invention are significantly overexpressed in isolated B cells stimulated by anti-CD40/IL-4 as compared to isolated, non-stimulated B cells. As described above, these data demonstrate that the PRO polypeptides of the present invention are useful not only as diagnostic markers for the presence of one or more immune disorders, but also serve as therapeutic targets for the treatment of those immune disorders.

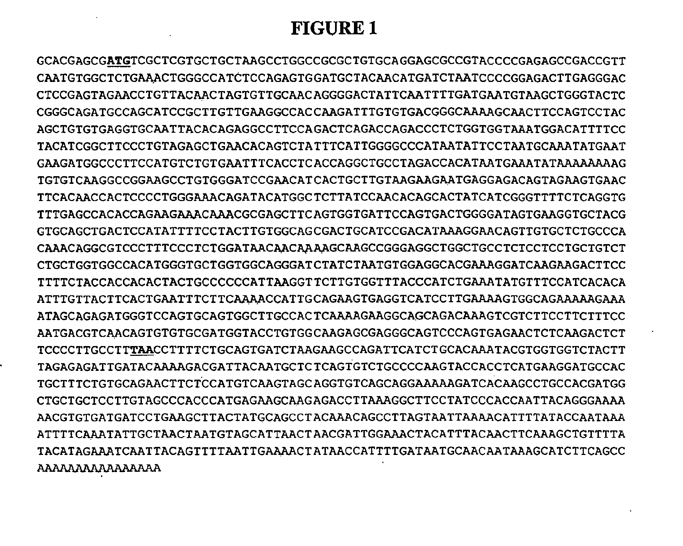

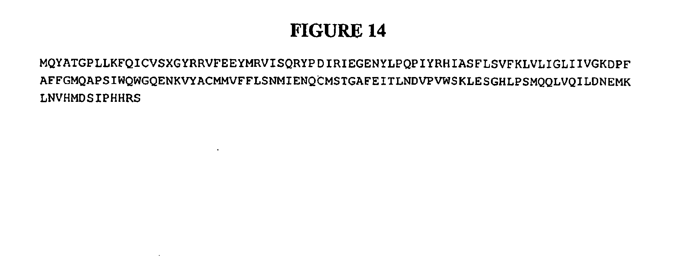

Figures 1-28 are upregulated upon stimulation with anti-CD40/IL-4.

### EXAMPLE 2: Use of PRO as a hybridization probe

The following method describes use of a nucleotide sequence encoding PRO as a hybridization probe.

DNA comprising the coding sequence of full-length or mature PRO as disclosed herein is employed as a probe to screen for homologous DNAs (such as those encoding naturally-occurring variants of PRO) in human tissue cDNA libraries or human tissue genomic libraries.

Hybridization and washing of filters containing either library DNAs is performed under the following high stringency conditions. Hybridization of radiolabeled PRO-derived probe to the filters is performed in a solution of 50% formamide, 5x SSC, 0.1% SDS, 0.1% sodium pyrophosphate, 50 mM sodium phosphate, pH 6.8, 2x Denhardt's solution, and 10% dextran sulfate at 42°C for 20 hours. Washing of the filters is performed in an aqueous solution of 0.1 x SSC and 0.1 % SDS at 42°C.

DNAs having a desired sequence identity with the DNA encoding full-length native sequence PRO can then be identified using standard techniques known in the art.

### EXAMPLE 3: Expression of PRO in E. coli

This example illustrates preparation of an unglycosylated form of PRO by recombinant expression in *E. coli.*

The DNA sequence encoding PRO is initially amplified using selected PCR primers. The primers should contain restriction enzyme sites which correspond to the restriction enzyme sites on the selected expression vector. A variety of expression vectors may be employed. An example of a suitable vector is pBR322 (derived from *E. coli*; see Bolivar et al., Gene, 2:95 (1977)) which contains genes for ampicillin and tetracycline resistance. The vector is digested with restriction enzyme and dephosphorylated. The PCR amplified sequences are then ligated into the vector. The vector will preferably include sequences which encode for an antibiotic resistance gene, a trp promoter, a polyhis leader (including the first six STII codons, polyhis sequence, and enterokinase cleavage site), the PRO coding region, lambda transcriptional terminator, and an argU gene.

The ligation mixture is then used to transform a selected *E. coli* strain using the methods described in Sambrook et al., supra. Transformants are identified by their ability to grow on LB plates and antibiotic resistant colonies are then selected. Plasmid DNA can be isolated and confirmed by restriction analysis and DNA sequencing.

Selected clones can be grown overnight in liquid culture medium such as LB broth supplemented with antibiotics. The overnight culture may subsequently be used to inoculate a larger scale culture. The cells are then grown to a desired optical density, during which the expression promoter is turned on.

After culturing the cells for several more hours, the cells can be harvested by centrifugation. The cell pellet obtained by the centrifugation can be solubilized using various agents known in the art, and the solubilized PRO protein can then be purified using a metal chelating column under conditions that allow tight binding of the protein.

PRO may be expressed in *E. coli* in a poly-His tagged form, using the following procedure. The DNA encoding PRO is initially amplified using selected PCR primers. The primers will contain restriction enzyme sites which correspond to the restriction enzyme sites on the selected expression vector, and other useful sequences providing for efficient and reliable translation initiation, rapid purification on a metal chelation column, and proteolytic removal with enterokinase. The PCR-amplified, poly-His tagged sequences are then ligated into an expression vector, which is used to transform an *E. coli* host based on strain 52 (W3110 fuhA(tonA) lon galE rpoHts(htpRts) clpP(lacIq). Transformants are first grown in LB containing 50 mg/ml carbenicillin at 30°C with shaking until an O.D.600 of 3-5 is reached. Cultures are then diluted 50-100 fold into CRAP media (prepared by mixing 3.57 g (NH₄)₂SO₄, 0.71 g sodium citrate•2H2O, 1.07 g KCl, 5 .36 g Difco yeast extract, 5.36 g Sheffield hycase SF in 500 mL water, as well as 110 mM MPOS, pH 7.3, 0.55% (w/v) glucose and 7 mM MgSO₄) and grown for approximately 20-30 hours at 30°C with shaking. Samples are removed to verify expression by SDS-PAGE analysis, and the bulk culture is centrifuged to pellet the cells. Cell pellets are frozen until purification and refolding.

*E. coli* paste from 0.5 to 1 L fermentations (6-10 g pellets) is resuspended in 10 volumes (w/v) in 7 M guanidine, 20 mM Tris, pH 8 buffer. Solid sodium sulfite and sodium tetrathionate is added to make final concentrations of 0.1M and 0.02 M, respectively, and the solution is stirred overnight at 4°C. This step results in a denatured protein with all cysteine residues blocked by sulfitolization. The solution is centrifuged at 40,000 rpm in a Beckman Ultracentifuge for 30 min. The supernatant is diluted with 3-5 volumes of metal chelate column buffer (6 M guanidine, 20 mM Tris, pH 7.4) and filtered through 0.22 micron filters to clarify. The clarified extract is loaded onto a 5 ml Qiagen Ni-NTA metal chelate column equilibrated in the metal chelate column buffer. The column is washed with additional buffer containing 50 mM imidazole (Calbiochem, Utrol grade), pH 7.4. The protein is eluted with buffer containing 250 mM imidazole. Fractions containing the desired protein are pooled and stored at 4°C. Protein concentration is estimated by its absorbance at 280 nm using the calculated extinction coefficient based on its amino acid sequence.

The proteins are refolded by diluting the sample slowly into freshly prepared refolding buffer consisting of: 20 mM Tris, pH 8.6, 0.3 M NaCl, 2.5 M urea, 5 mM cysteine, 20 mM glycine and 1 mM EDTA. Refolding volumes are chosen so that the final protein concentration is between 50 to 100 micrograms/ml. The refolding solution is stirred gently at 4°C for 12-36 hours. The refolding reaction is quenched by the addition of TFA to a final concentration of 0.4% (pH of approximately 3). Before further purification of the protein, the solution is filtered through a 0.22 micron filter and acetonitrile is added to 2-10% final concentration. The refolded protein is chromatographed on a Poros R1/H reversed phase column using a mobile buffer of 0.1 % TFA with elution with a gradient of acetonitrile from 10 to 80%. Aliquots of fractions with A280 absorbance are analyzed on SDS polyacrylamide gels and fractions containing homogeneous refolded protein are pooled. Generally, the properly refolded species of most proteins are eluted at the lowest concentrations of acetonitrile since those species are the most compact with their hydrophobic interiors shielded from interaction with the reversed phase resin. Aggregated species are usually eluted at higher acetonitrile concentrations. In addition to resolving misfolded forms of proteins from the desired form, the reversed phase step also removes endotoxin from the samples.

Fractions containing the desired folded PRO polypeptide are pooled and the acetonitrile removed using a gentle stream of nitrogen directed at the solution. Proteins are formulated into 20 mM Hepes, pH 6.8 with 0.14 M sodium chloride and 4% mannitol by dialysis or by gel filtration using G25 Superfine (Pharmacia) resins equilibrated in the formulation buffer and sterile filtered.

Many of the PRO polypeptides disclosed herein were successfully expressed as described above.

### EXAMPLE 4: Expression of PRO in mammalian cells

This example illustrates preparation of a potentially glycosylated form of PRO by recombinant expression in mammalian cells.

The vector, pRK5 (see EP 307,247, published March 15, 1989), is employed as the expression vector. Optionally, the PRO DNA is ligated into pRK5 with selected restriction enzymes to allow insertion of the PRO DNA using ligation methods such as described in Sambrook et al., supra. The resulting vector is called pRK5-PRO.

In one embodiment, the selected host cells may be 293 cells. Human 293 cells (ATCC CCL 1573) are grown to confluence in tissue culture plates in medium such as DMEM supplemented with fetal calf serum and optionally, nutrient components and/or antibiotics. About 10 µg pRK5-PRO DNA is mixed with about 1 µg DNA encoding the VA RNA gene [Thimmappaya et al., Cell, 31:543 (1982)] and dissolved in 500 µl of 1 mM Tris-HCl, 0.1 mM EDTA, 0.227 M CaCl₂. To this mixture is added, dropwise, 500 µl of 50 mM HEPES (pH 7.35), 280 mM NaCl, 1.5 mM NaPO₄, and a precipitate is allowed to form for 10 minutes at 25°C. The precipitate is suspended and added to the 293 cells and allowed to settle for about four hours at 37°C. The culture medium is aspirated off and 2 ml of 20% glycerol in PBS is added for 30 seconds. The 293 cells are then washed with serum free medium, fresh medium is added and the cells are incubated for about 5 days.

Approximately 24 hours after the transfections, the culture medium is removed and replaced with culture medium (alone) or culture medium containing 200 µCi/ml ³⁵S-cysteine and 200 µCi/ml ³⁵S-methionine. After a 12 hour incubation, the conditioned medium is collected, concentrated on a spin filter, and loaded onto a 15% SDS gel. The processed gel may be dried and exposed to film for a selected period of time to reveal the presence of PRO polypeptide. The cultures containing transfected cells may undergo further incubation (in serum free medium) and the medium is tested in selected bioassays.

In an alternative technique, PRO may be introduced into 293 cells transiently using the dextran sulfate method described by Somparyrac et al., Proc. Natl. Acad. Sci., 12:7575 (1981). 293 cells are grown to maximal density in a spinner flask and 700 µg pRK5-PRO DNA is added. The cells are first concentrated from the spinner flask by centrifugation and washed with PBS. The DNA-dextran precipitate is incubated on the cell pellet for four hours. The cells are treated with 20% glycerol for 90 seconds, washed with tissue culture medium, and re-introduced into the spinner flask containing tissue culture medium, 5 µg/ml bovine insulin and 0.1 µg/ml bovine transferrin. After about four days, the conditioned media is centrifuged and filtered to remove cells and debris. The sample containing expressed PRO can then be concentrated and purified by any selected method, such as dialysis and/or column chromatography.

In another embodiment, PRO can be expressed in CHO cells. The pRK5-PRO can be transfected into CHO cells using known reagents such as CaPO₄ or DEAE-dextran. As described above, the cell cultures can be incubated, and the medium replaced with culture medium (alone) or medium containing a radiolabel such as ³⁵S-methionine. After determining the presence of PRO polypeptide, the culture medium may be replaced with serum free medium. Preferably, the cultures are incubated for about 6 days, and then the conditioned medium is harvested. The medium containing the expressed PRO can then be concentrated and purified by any selected method.

Epitope-tagged PRO may also be expressed in host CHO cells. The PRO may be subcloned out of the pRK5 vector. The subclone insert can undergo PCR to fuse in frame with a selected epitope tag such as a poly-his tag into a Baculovirus expression vector. The poly-his tagged PRO insert can then be subcloned into a SV40 promoter/enhancer containing vector containing a selection marker such as DHFR for selection of stable clones. Finally, the CHO cells can be transfected (as described above) with the SV40 promoter/enhancer containing vector. Labeling may be performed, as described above, to verify expression. The culture medium containing the expressed poly-His tagged PRO can then be concentrated and purified by any selected method, such as by Ni²⁺-chelate affinity chromatography.

PRO may also be expressed in CHO and/or COS cells by a transient expression procedure or in CHO cells by another stable expression procedure.

Stable expression in CHO cells is performed using the following procedure. The proteins are expressed as an IgG construct (immunoadhesin), in which the coding sequences for the soluble forms (e.g. extracellular domains) of the respective proteins are fused to an IgG1 constant region sequence containing the hinge, CH2 and CH2 domains and/or is a poly-His tagged form.

Following PCR amplification, the respective DNAs are subcloned in a CHO expression vector using standard techniques as described in Ausubel et al., Current Protocols of Molecular Biology, Unit 3.16, John Wiley and Sons (1997). CHO expression vectors are constructed to have compatible restriction sites 5' and 3' of the DNA of interest to allow the convenient shuttling of cDNA's. The vector used expression in CHO cells is as described in Lucas et al., Nucl. Acids Res. 24:9 (1774-1779 (1996), and uses the SV40 early promoter/enhancer to drive expression of the cDNA of interest and dihydrofolate reductase (DHFR). DHFR expression permits selection for stable maintenance of the plasmid following transfection.

Twelve micrograms of the desired plasmid DNA is introduced into approximately 10 million CHO cells using commercially available transfection reagents Superfect^{•} (Quiagen), Dosper^{•} or Fugene^{•} (Boehringer Mannheim). The cells are grown as described in Lucas et al., supra. Approximately 3 x 10⁻⁷ cells are frozen in an ampule for further growth and production as described below.

The ampules containing the plasmid DNA are thawed by placement into water bath and mixed by vortexing. The contents are pipetted into a centrifuge tube containing 10 mL of media and centrifuged at 1000 rpm for 5 minutes. The supernatant is aspirated and the cells are resuspended in 10 mL of selective media (0.2 µm filtered PS20 with 5% 0.2 µm diafiltered fetal bovine serum). The cells are then aliquoted into a 100 mL spinner containing 90 mL of selective media. After 1-2 days, the cells are transferred into a 250 mL spinner filled with 150 mL selective growth medium and incubated at 37°C. After another 2-3 days, 250 mL, 500 mL and 2000 mL spinners are seeded with 3 x 10⁵ cells/mL. The cell media is exchanged with fresh media by centrifugation and resuspension in production medium. Although any suitable CHO media may be employed, a production medium described in U.S. Patent No. 5,122,469, issued June 16, 1992 may actually be used. A 3L production spinner is seeded at 1.2 x 10⁶ cells/mL. On day 0, pH is determined. On day 1, the spinner is sampled and sparging with filtered air is commenced. On day 2, the spinner is sampled, the temperature shifted to 33°C, and 30 mL of 500 g/L glucose and 0.6 mL of 10% antifoam (e.g., 35% polydimethylsiloxane emulsion, Dow Corning 365 Medical Grade Emulsion) taken. Throughout the production, the pH is adjusted as necessary to keep it at around 7.2. After 10 days, or until the viability dropped below 70%, the cell culture is harvested by centrifugation and filtering through a 0.22 µm filter. The filtrate was either stored at 4°C or immediately loaded onto columns for purification.

For the poly-His tagged constructs, the proteins are purified using a Ni-NTA column (Qiagen). Before purification, imidazole is added to the conditioned media to a concentration of 5 mM. The conditioned media is pumped onto a 6 ml Ni-NTA column equilibrated in 20 mM Hepes, pH 7.4, buffer containing 0.3 M NaCl and 5 mM imidazole at a flow rate of 4-5 ml/min. at 4°C. After loading, the column is washed with additional equilibration buffer and the protein eluted with equilibration buffer containing 0.25 M imidazole. The highly purified protein is subsequently desalted into a storage buffer containing 10 mM Hepes, 0.14 M NaCl and 4% mannitol, pH 6.8, with a 25 ml G25 Superfine (Pharmacia) column and stored at -80°C.

Immunoadhesin (Fc-containing) constructs are purified from the conditioned media as follows. The conditioned medium is pumped onto a 5 ml Protein A column (Pharmacia) which had been equilibrated in 20 mM Na phosphate buffer, pH 6.8. After loading, the column is washed extensively with equilibration buffer before elution with 100 mM citric acid, pH 3.5. The eluted protein is immediately neutralized by collecting 1 ml fractions into tubes containing 275 µl of 1 M Tris buffer, pH 9. The highly purified protein is subsequently desalted into storage buffer as described above for the poly-His tagged proteins. The homogeneity is assessed by SDS polyacrylamide gels and by N-terminal amino acid sequencing by Edman degradation.

Many of the PRO polypeptides disclosed herein were successfully expressed as described above.

### EXAMPLE 5: Expression of PRO in Yeast

The following method describes recombinant expression of PRO in yeast.

First, yeast expression vectors are constructed for intracellular production or secretion of PRO from the ADH2/GAPDH promoter. DNA encoding PRO and the promoter is inserted into suitable restriction enzyme sites in the selected plasmid to direct intracellular expression of PRO. For secretion, DNA encoding PRO can be cloned into the selected plasmid, together with DNA encoding the ADH2/GAPDH promoter, a native PRO signal peptide or other mammalian signal peptide, or, for example, a yeast alpha-factor or invertase secretory signal/leader sequence, and linker sequences (if needed) for expression of PRO.

Yeast cells, such as yeast strain AB110, can then be transformed with the expression plasmids described above and cultured in selected fermentation media. The transformed yeast supernatants can be analyzed by precipitation with 10% trichloroacetic acid and separation by SDS-PAGE, followed by staining of the gels with Coomassie Blue stain.

Recombinant PRO can subsequently be isolated and purified by removing the yeast cells from the fermentation medium by centrifugation and then concentrating the medium using selected cartridge filters. The concentrate containing PRO may further be purified using selected column chromatography resins.

Many of the PRO polypeptides disclosed herein were successfully expressed as described above.

### EXAMPLE 6: Expression of PRO in Baculovirus-Infected Insect Cells

The following method describes recombinant expression of PRO in Baculovirus-infected insect cells.

The sequence coding for PRO is fused upstream of an epitope tag contained within a baculovirus expression vector. Such epitope tags include poly-his tags and immunoglobulin tags (like Fc regions of IgG). A variety of plasmids may be employed, including plasmids derived from commercially available plasmids such as pVL1393 (Novagen). Briefly, the sequence encoding PRO or the desired portion of the coding sequence of PRO such as the sequence encoding the extracellular domain of a transmembrane protein or the sequence encoding the mature protein if the protein is extracellular is amplified by PCR with primers complementary to the 5' and 3' regions. The 5' primer may incorporate flanking (selected) restriction enzyme sites. The product is then digested with those selected restriction enzymes and subcloned into the expression vector.

Recombinant baculovirus is generated by co-transfecting the above plasmid and BaculoGold™ virus DNA (Pharmingen) into *Spodoptera frugiperda* ("Sf9") cells (ATCC CRL 1711) using lipofectin (commercially available from GIBCO-BRL). After 4 - 5 days of incubation at 28°C, the released viruses are harvested and used for further amplifications. Viral infection and protein expression are performed as described by O'Reilley et al., Baculovirus expression vectors: A Laboratory Manual, Oxford: Oxford University Press (1994).

Expressed poly-his tagged PRO can then be purified, for example, by Ni²⁺-chelate affinity chromatography as follows. Extracts are prepared from recombinant virus-infected Sf9 cells as described by Rupert et al., Nature, 362:175-179 (1993). Briefly, Sf9 cells are washed, resuspended in sonication buffer (25 mL Hepes, pH 7.9; 12.5 mM MgCl₂; 0.1 mM EDTA; 10% glycerol; 0.1 % NP-40; 0.4 M KCl), and sonicated twice for 20 seconds on ice. The sonicates are cleared by centrifugation, and the supernatant is diluted 50-fold in loading buffer (50 mM phosphate, 300 mM NaCl, 10% glycerol, pH 7.8) and filtered through a 0.45 µm filter. A Ni²⁺-NTA agarose column (commercially available from Qiagen) is prepared with a bed volume of 5 mL, washed with 25 mL of water and equilibrated with 25 mL of loading buffer. The filtered cell extract is loaded onto the column at 0.5 mL per minute. The column is washed to baseline A₂₈₀ with loading buffer, at which point fraction collection is started. Next, the column is washed with a secondary wash buffer (50 mM phosphate; 300 mM NaCl, 10% glycerol, pH 6.0), which elutes nonspecifically bound protein. After reaching A₂₈₀ baseline again, the column is developed with a 0 to 500 mM Imidazole gradient in the secondary wash buffer. One mL fractions are collected and analyzed by SDS-PAGE and silver staining or Western blot with Ni²⁺-NTA-conjugated to alkaline phosphatase (Qiagen). Fractions containing the eluted His₁₀-tagged PRO are pooled and dialyzed against loading buffer.

Alternatively, purification of the IgG tagged (or Fc tagged) PRO can be performed using known chromatography techniques, including for instance, Protein A or protein G column chromatography.

Many of the PRO polypeptides disclosed herein were successfully expressed as described above.

### EXAMPLE 7: Preparation of Antibodies that Bind PRO

This example illustrates preparation of monoclonal antibodies which can specifically bind PRO.

Techniques for producing the monoclonal antibodies are known in the art and are described, for instance, in Goding, supra. Immunogens that may be employed include purified PRO, fusion proteins containing PRO, and cells expressing recombinant PRO on the cell surface. Selection of the immunogen can be made by the skilled artisan without undue experimentation.

Mice, such as Balb/c, are immunized with the PRO immunogen emulsified in complete Freund's adjuvant and injected subcutaneously or intraperitoneally in an amount from 1-100 micrograms. Alternatively, the immunogen is emulsified in MPL-TDM adjuvant (Ribi Immunochemical Research, Hamilton, MT) and injected into the animal's hind foot pads. The immunized mice are then boosted 10 to 12 days later with additional immunogen emulsified in the selected adjuvant. Thereafter, for several weeks, the mice may also be boosted with additional immunization injections. Serum samples may be periodically obtained from the mice by retro-orbital bleeding for testing in ELISA assays to detect anti-PRO antibodies.

After a suitable antibody titer has been detected, the animals "positive" for antibodies can be injected with a final intravenous injection of PRO. Three to four days later, the mice are sacrificed and the spleen cells are harvested. The spleen cells are then fused (using 35% polyethylene glycol) to a selected murine myeloma cell line such as P3X63AgU.1, available from ATCC, No. CRL 1597. The fusions generate hybridoma cells which can then be plated in 96 well tissue culture plates containing HAT (hypoxanthine, aminopterin, and thymidine) medium to inhibit proliferation of non-fused cells, myeloma hybrids, and spleen cell hybrids.

The hybridoma cells will be screened in an ELISA for reactivity against PRO. Determination of "positive" hybridoma cells secreting the desired monoclonal antibodies against PRO is within the skill in the art.

The positive hybridoma cells can be injected intraperitoneally into syngeneic Balb/c mice to produce ascites containing the anti-PRO monoclonal antibodies. Alternatively, the hybridoma cells can be grown in tissue culture flasks or roller bottles. Purification of the monoclonal antibodies produced in the ascites can be accomplished using ammonium sulfate precipitation, followed by gel exclusion chromatography. Alternatively, affinity chromatography based upon binding of antibody to protein A or protein G can be employed.

### EXAMPLE 8: Purification of PRO Polypeptides Using Specific Antibodies

Native or recombinant PRO polypeptides may be purified by a variety of standard techniques in the art of protein purification. For example, pro-PRO polypeptide, mature PRO polypeptide, or pre-PRO polypeptide is purified by immunoaffinity chromatography using antibodies specific for the PRO polypeptide of interest. In general, an immunoaffinity column is constructed by covalently coupling the anti-PRO polypeptide antibody to an activated chromatographic resin.

Polyclonal immunoglobulins are prepared from immune sera either by precipitation with ammonium sulfate or by purification on immobilized Protein A (Pharmacia LKB Biotechnology, Piscataway, N.J.). Likewise, monoclonal antibodies are prepared from mouse ascites fluid by ammonium sulfate precipitation or chromatography on immobilized Protein A. Partially purified immunoglobulin is covalently attached to a chromatographic resin such as CnBr-activated SEPHAROSE^{™} (Pharmacia LKB Biotechnology). The antibody is coupled to the resin, the resin is blocked, and the derivative resin is washed according to the manufacturer's instructions.

Such an immunoaffinity column is utilized in the purification of PRO polypeptide by preparing a fraction from cells containing PRO polypeptide in a soluble form. This preparation is derived by solubilization of the whole cell or of a subcellular fraction obtained via differential centrifugation by the addition of detergent or by other methods well known in the art. Alternatively, soluble PRO polypeptide containing a signal sequence may be secreted in useful quantity into the medium in which the cells are grown.

A soluble PRO polypeptide-containing preparation is passed over the immunoaffinity column, and the column is washed under conditions that allow the preferential absorbance of PRO polypeptide (*e.g.*, high ionic strength buffers in the presence of detergent). Then, the column is eluted under conditions that disrupt antibody/PRO polypeptide binding (*e.g.*, a low pH buffer such as approximately pH 2-3, or a high concentration of a chaotrope such as urea or thiocyanate ion), and PRO polypeptide is collected.

### EXAMPLE 9: Drug Screening

This invention is particularly useful for screening compounds by using PRO polypeptides or binding fragment thereof in any of a variety of drug screening techniques. The PRO polypeptide or fragment employed in such a test may either be free in solution, affixed to a solid support, borne on a cell surface, or located intracellularly. One method of drug screening utilizes eukaryotic or prokaryotic host cells which are stably transformed with recombinant nucleic acids expressing the PRO polypeptide or fragment. Drugs are screened against such transformed cells in competitive binding assays. Such cells, either in viable or fixed form, can be used for standard binding assays. One may measure, for example, the formation of complexes between PRO polypeptide or a fragment and the agent being tested. Alternatively, one can examine the diminution in complex formation between the PRO polypeptide and its target cell or target receptors caused by the agent being tested.

Thus, the present invention provides methods of screening for drugs or any other agents which can affect a PRO polypeptide-associated disease or disorder. These methods comprise contacting such an agent with an PRO polypeptide or fragment thereof and assaying (I) for the presence of a complex between the agent and the PRO polypeptide or fragment, or (ii) for the presence of a complex between the PRO polypeptide or fragment and the cell, by methods well known in the art. In such competitive binding assays, the PRO polypeptide or fragment is typically labeled. After suitable incubation, free PRO polypeptide or fragment is separated from that present in bound form, and the amount of free or uncomplexed label is a measure of the ability of the particular agent to bind to PRO polypeptide or to interfere with the PRO polypeptide/cell complex.

Another technique for drug screening provides high throughput screening for compounds having suitable binding affinity to a polypeptide and is described in detail in WO 84/03564, published on September 13, 1984. Briefly stated, large numbers of different small peptide test compounds are synthesized on a solid substrate, such as plastic pins or some other surface. As applied to a PRO polypeptide, the peptide test compounds are reacted with PRO polypeptide and washed. Bound PRO polypeptide is detected by methods well known in the art. Purified PRO polypeptide can also be coated directly onto plates for use in the aforementioned drug screening techniques. In addition, non-neutralizing antibodies can be used to capture the peptide and immobilize it on the solid support.

This invention also contemplates the use of competitive drug screening assays in which neutralizing antibodies capable of binding PRO polypeptide specifically compete with a test compound for binding to PRO polypeptide or fragments thereof. In this manner, the antibodies can be used to detect the presence of any peptide which shares one or more antigenic determinants with PRO polypeptide.

### EXAMPLE 10: Rational Drug Design

The goal of rational drug design is to produce structural analogs of biologically active polypeptide of interest (*i.e.*, a PRO polypeptide) or of small molecules with which they interact, *e.g.*, agonists, antagonists, or inhibitors. Any of these examples can be used to fashion drugs which are more active or stable forms of the PRO polypeptide or which enhance or interfere with the function of the PRO polypeptide *in vivo* (*c.f.*, Hodgson, Bio/Technology, 9: 19-21 (1991)).

In one approach, the three-dimensional structure of the PRO polypeptide, or of a PRO polypeptide-inhibitor complex, is determined by x-ray crystallography, by computer modeling or, most typically, by a combination of the two approaches. Both the shape and charges of the PRO polypeptide must be ascertained to elucidate the structure and to determine active site(s) of the molecule. Less often, useful information regarding the structure of the PRO polypeptide may be gained by modeling based on the structure of homologous proteins. In both cases, relevant structural information is used to design analogous PRO polypeptide-like molecules or to identify efficient inhibitors. Useful examples of rational drug design may include molecules which have improved activity or stability as shown by Braxton and Wells, Biochemistry, 31:7796-7801 (1992) or which act as inhibitors, agonists, or antagonists of native peptides as shown by Athauda et al., J. Biochem., 113:742-746 (1993).

It is also possible to isolate a target-specific antibody, selected by functional assay, as described above, and then to solve its crystal structure. This approach, in principle, yields a pharmacore upon which subsequent drug design can be based. It is possible to bypass protein crystallography altogether by generating anti-idiotypic antibodies (anti-ids) to a functional, pharmacologically active antibody. As a mirror image of a mirror image, the binding site of the anti-ids would be expected to be an analog of the original receptor. The anti-id could then be used to identify and isolate peptides from banks of chemically or biologically produced peptides. The isolated peptides would then act as the pharmacore.

By virtue of the present invention, sufficient amounts of the PRO polypeptide may be made available to perform such analytical studies as X-ray crystallography. In addition, knowledge of the PRO polypeptide amino acid sequence provided herein will provide guidance to those employing computer modeling techniques in place of or in addition to x-ray crystallography.

The foregoing written specification is considered to be sufficient to enable one skilled in the art to practice the invention. The present invention is not to be limited in scope by the construct deposited, since the deposited embodiment is intended as a single illustration of certain aspects of the invention and any constructs that are functionally equivalent are within the scope of this invention. The deposit of material herein does not constitute an admission that the written description herein contained is inadequate to enable the practice of any aspect of the invention, including the best mode thereof, nor is it to be construed as limiting the scope of the claims to the specific illustrations that it represents. Indeed, various modifications of the invention in addition to those shown and described herein will become apparent to those skilled in the art from the foregoing description and fall within the scope of the appended claims.

### Statements of Invention

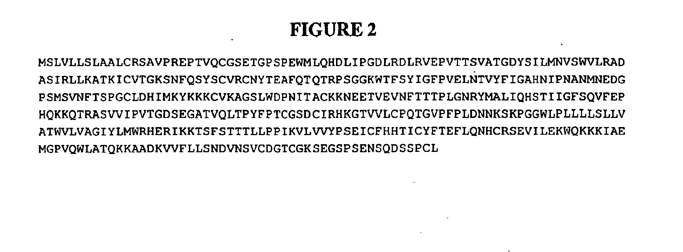

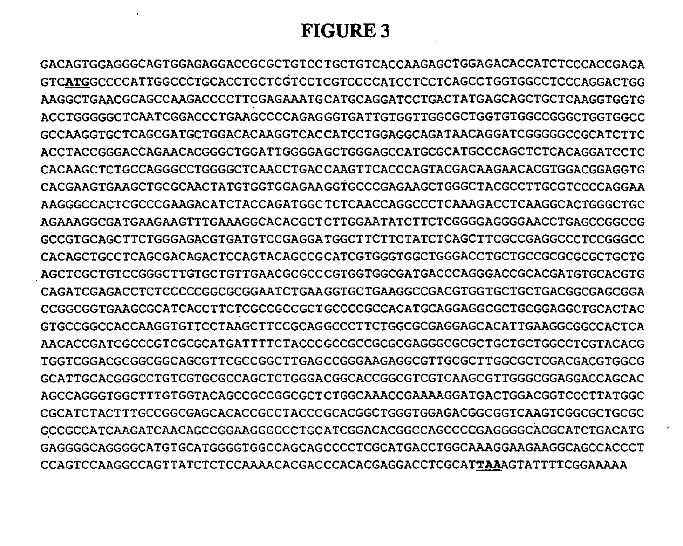

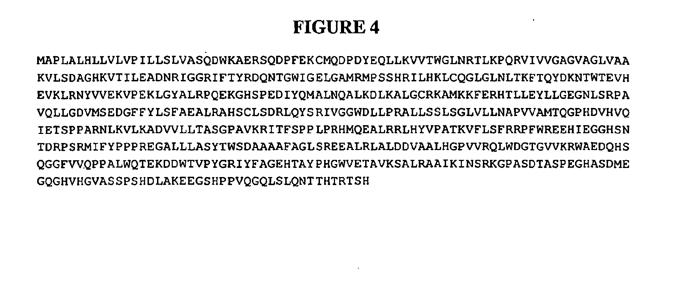

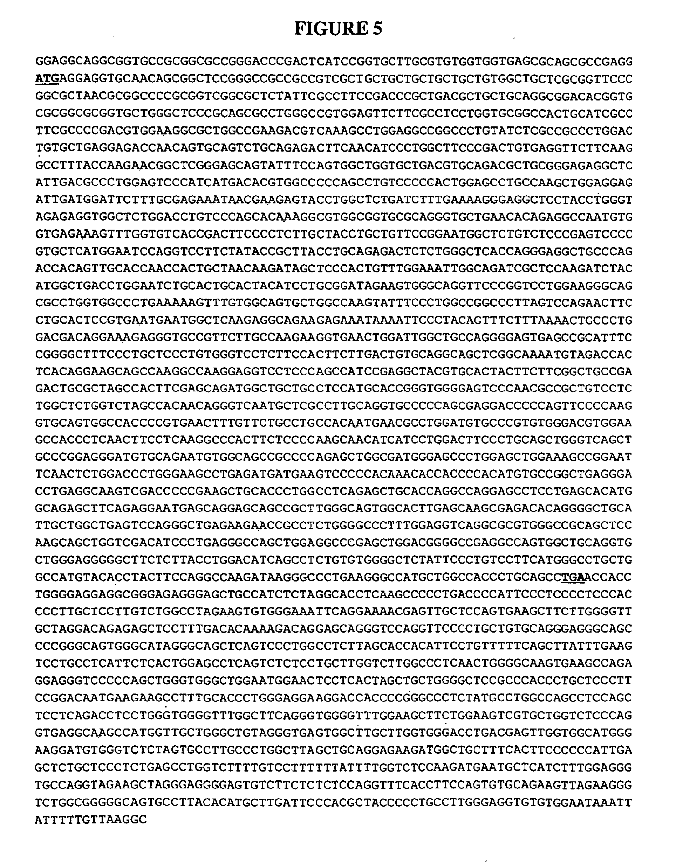

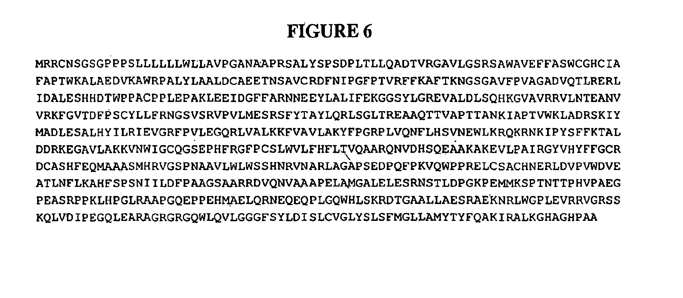

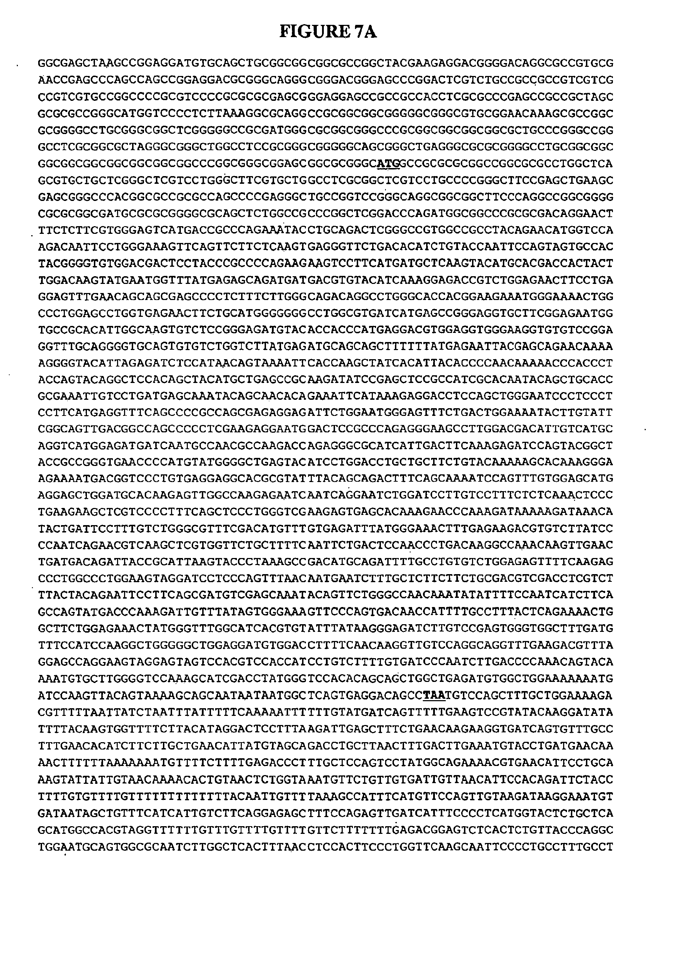

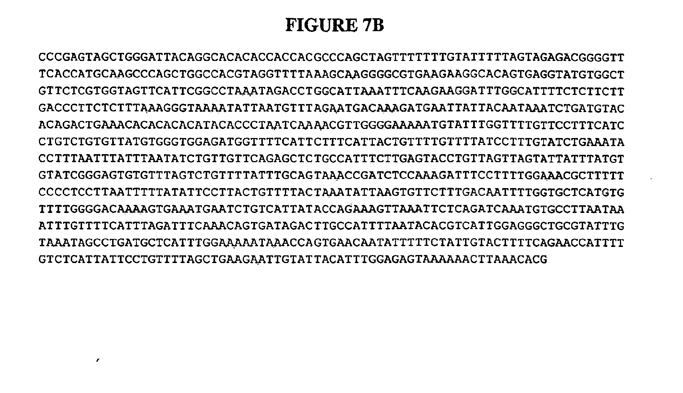

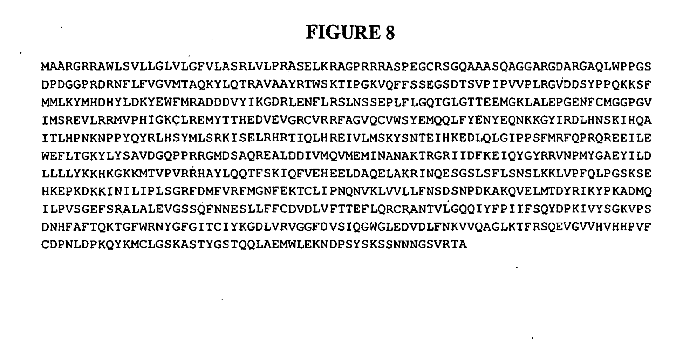

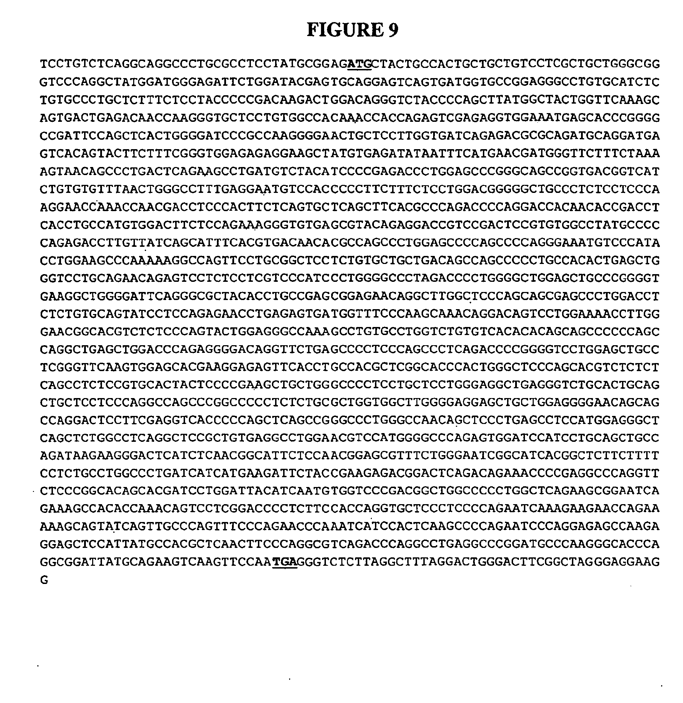

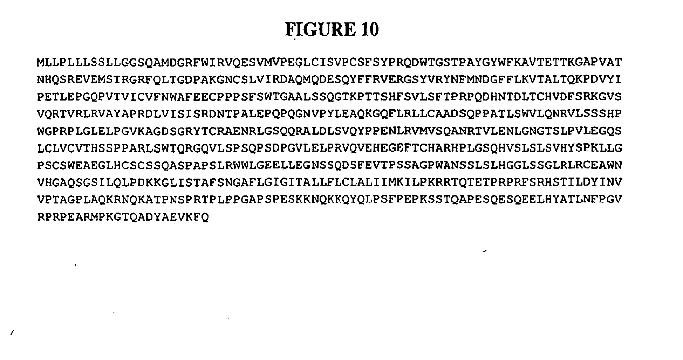

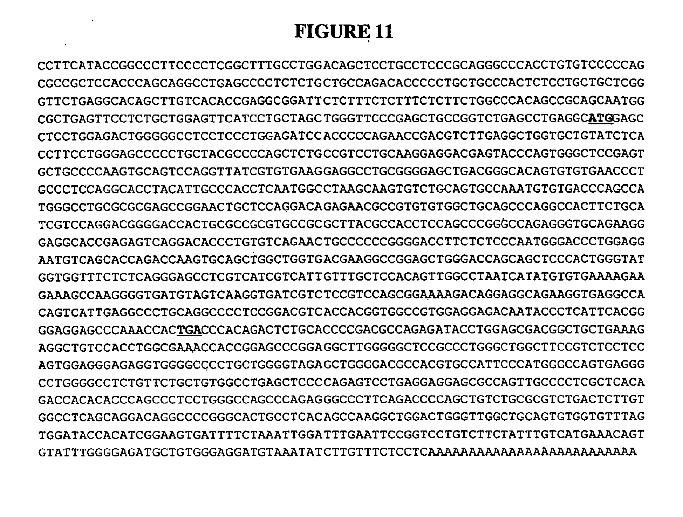

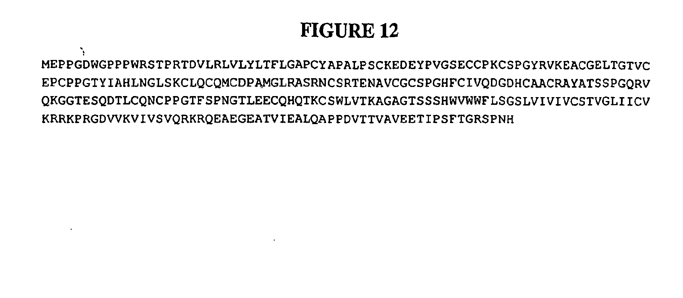

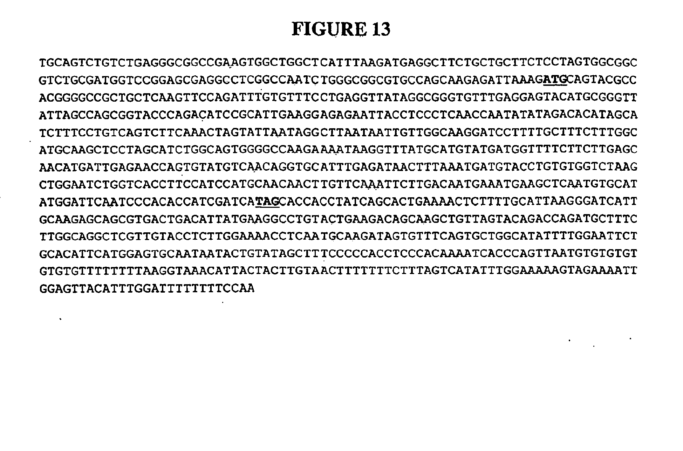

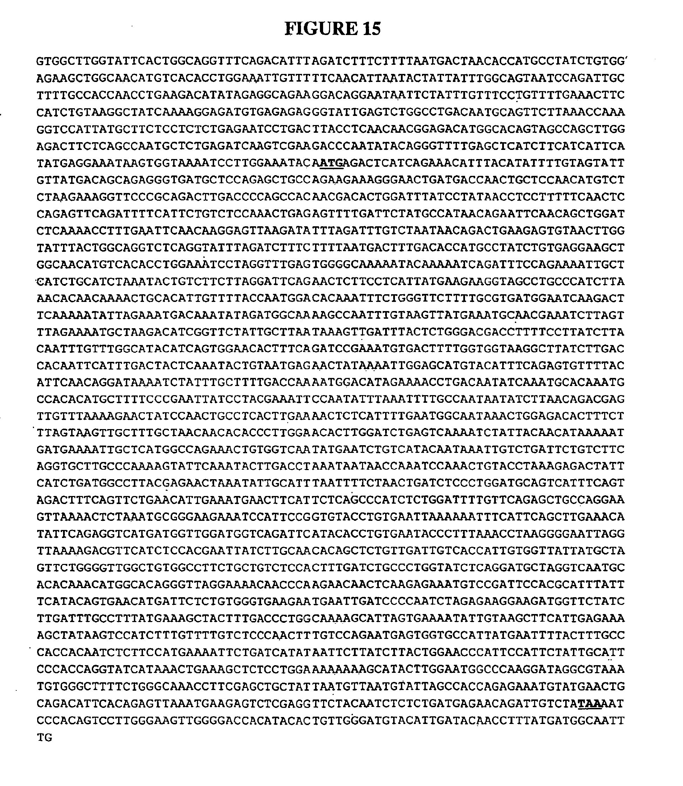

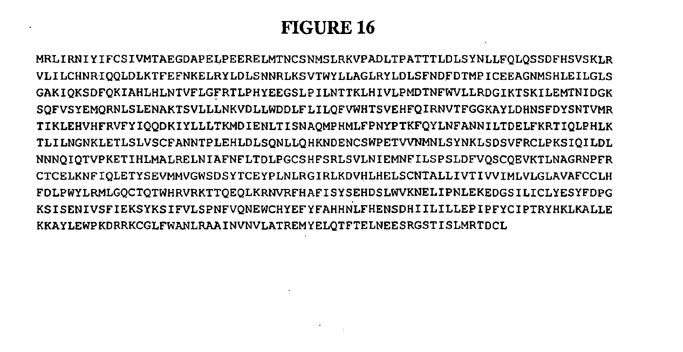

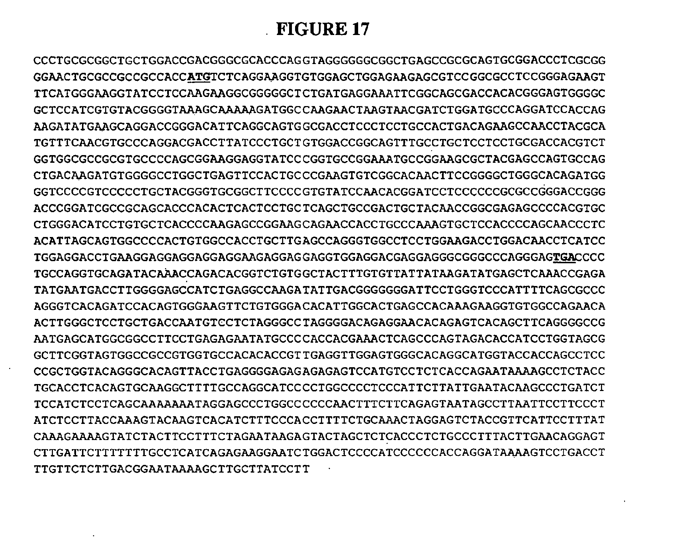

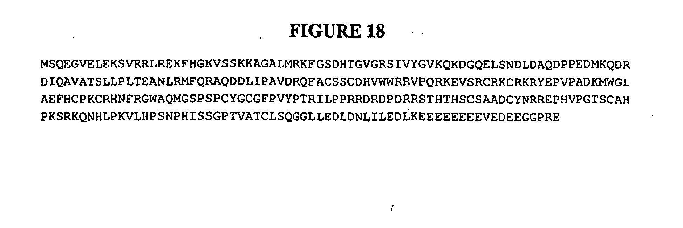

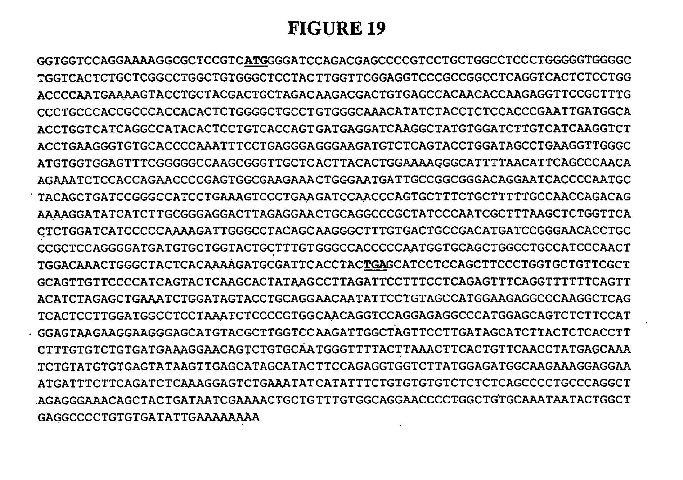

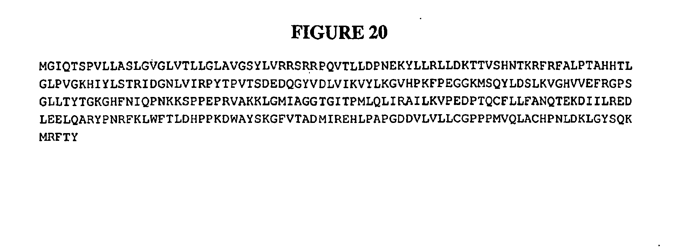

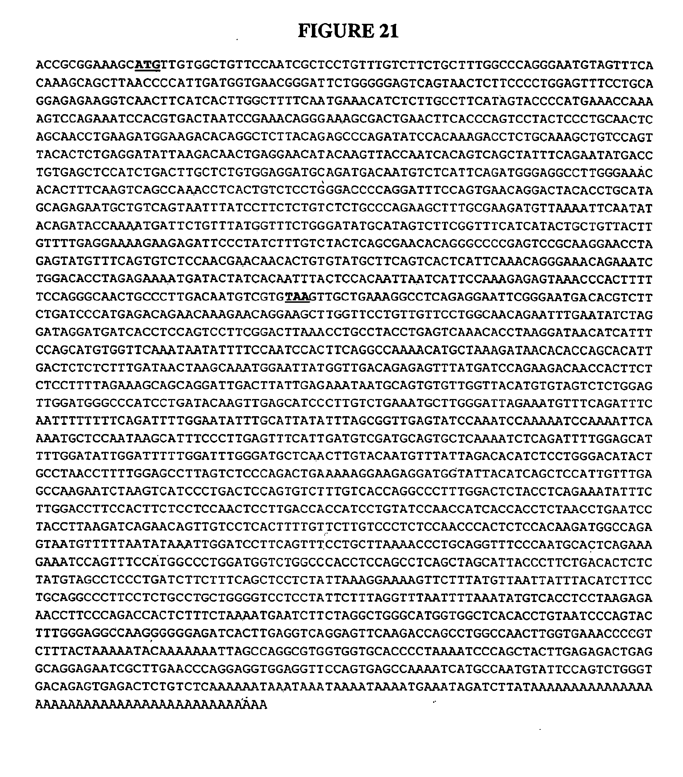

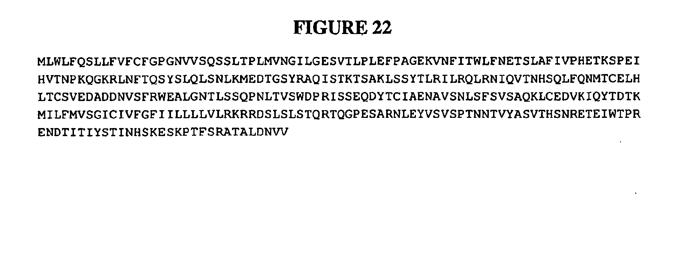

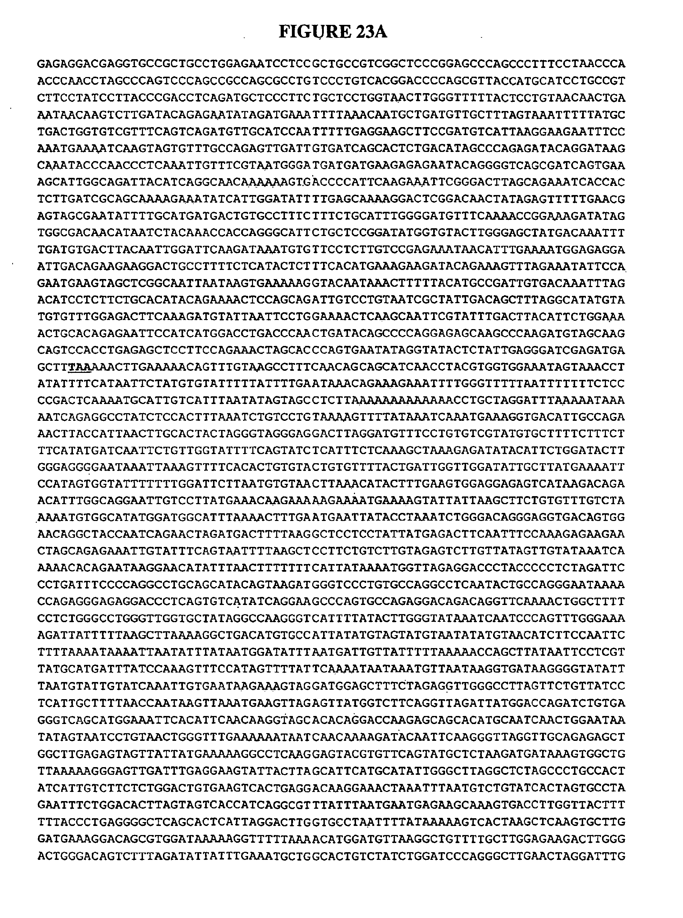

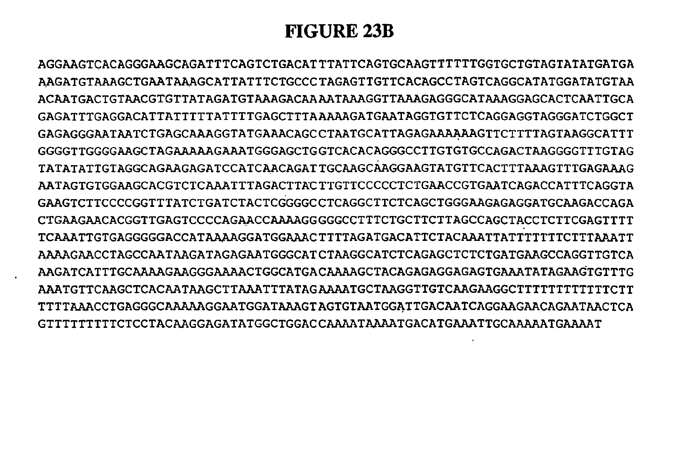

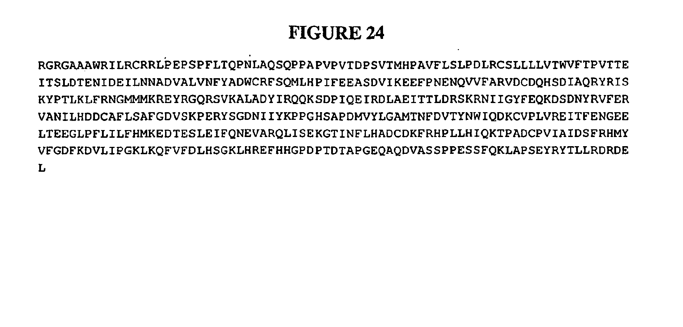

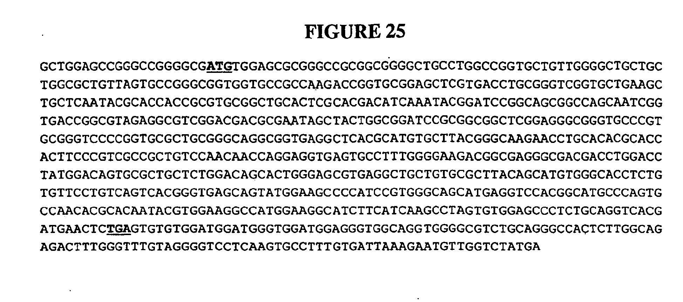

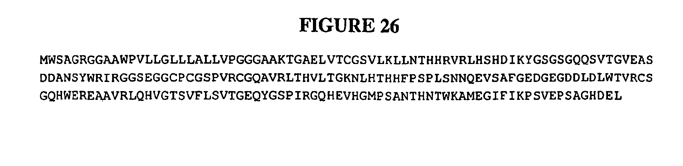

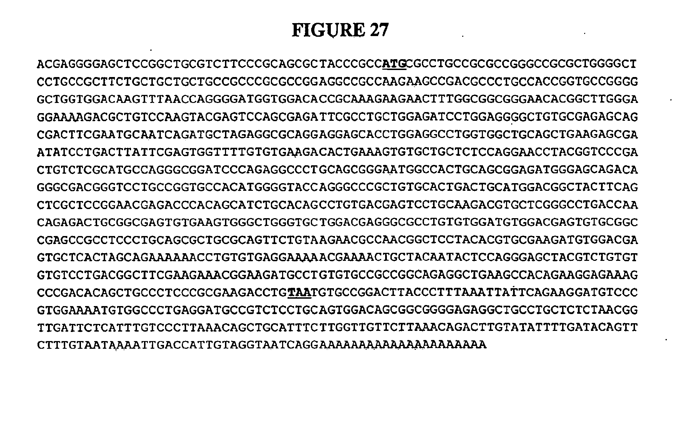

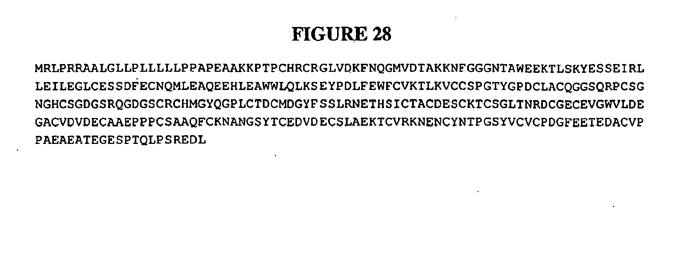

1. Isolated nucleic acid having at least 80% nucleic acid sequence identity to:
   (a) a nucleotide sequence encoding the polypeptide shown in Figure 2 (SEQ ID NO:2), Figure 4 (SEQ ID NO:4), Figure 6 (SEQ ID NO:6), Figure 8 (SEQ ID NO:8), Figure 10 (SEQ ID NO:10), Figure 12 (SEQ ID NO: 12), Figure 8 (SEQ ID NO: 14), Figure 8 (SEQ ID NO:14), Figure 16 (SEQ ID NO:16), Figure 18 (SEQ ID NO: 18), Figure 20 (SEQ ID NO:20), Figure 22 (SEQ ID NO:22), Figure 24 (SEQ ID NO:24), Figure 26 (SEQ ID NO:26) or Figure 28 (SEQ ID NO:28).
2. Isolated nucleic acid having at least 80% nucleic acid sequence identity to a nucleotide sequence selected from the group consisting of the nucleotide sequence shown in Figure 1 (SEQ ID NO:1), Figure 3 (SEQ ID NO:3), Figure 5 (SEQ ID NO:5), Figure 7A-B (SEQ ID NO:7), Figure 9 (SEQ ID NO:9), Figure 11 (SEQ ID NO:11), Figure 11 (SEQ ID NO:11), Figure 11 (SEQ ID NO:11), Figure 13 (SEQ ID NO:13), Figure 15 (SEQ ID NO:15), Figure 17 (SEQ ID NO:17), Figure 19 (SEQ ID NO:19), Figure 21 (SEQ ID NO:21), Figure 23A-B (SEQ ID NO:23), Figure 25 (SEQ ID NO:25) and Figure 27 (SEQ ID NO:27).
3. Isolated nucleic acid having at least 80% nucleic acid sequence identity to a nucleotide sequence selected from the group consisting of the full-length coding sequence of the nucleotide sequence shown in Figure 1 (SEQ ID NO:1), Figure 3 (SEQ ID NO:3), Figure 5 (SEQ ID NO:5), Figure 7A-B (SEQ ID NO:7), Figure 9 (SEQ ID NO:9), Figure 11 (SEQ ID NO:11), Figure 11 (SEQ ID NO:11), Figure 11 (SEQ ID NO:11), Figure 13 (SEQ ID NO:13), Figure 15A-B (SEQ ID NO:15), Figure 17 (SEQ ID NO:17), Figure 19 (SEQ ID NO:19), Figure 21 (SEQ ID NO:21), Figure 23A-B (SEQ ID NO:23), Figure 25 (SEQ ID NO:25) and Figure 27 (SEQ ID NO:27).
5. A vector comprising the nucleic acid of 1.
6. The vector of 5 operably linked to control sequences recognized by a host cell transformed with the vector.
7. A host cell comprising the vector of 5.
8. The host cell of 7, wherein said cell is a CHO cell, an *E.coli* cell or a yeast cell.
9. A process for producing a PRO polypeptide comprising culturing the host cell of 7 under conditions suitable for expression of said PRO polypeptide and recovering said PRO polypeptide from the cell culture.
10. An isolated polypeptide having at least 80% amino acid sequence identity to:
   (a) an amino acid sequence of the polypeptide shown in Figure 2 (SEQ ID NO:2), Figure 4 (SEQ ID NO:4), Figure 6 (SEQ ID NO:6), Figure 8 (SEQ ID NO:8), Figure 10 (SEQ ID NO:10), Figure 12 (SEQ ID NO:12), Figure 8 (SEQ ID NO:14), Figure 8 (SEQ ID NO:14), Figure 16 (SEQ ID NO:16), Figure 18 (SEQ ID NO:18), Figure 20 (SEQ ID NO:20), Figure 22 (SEQ ID NO:22), Figure 24 (SEQ ID NO:24), Figure 26 (SEQ ID NO:26) or Figure 28 (SEQ ID NO:28).
12. A chimeric molecule comprising a polypeptide according to 10 fused to a heterologous amino acid sequence.
13. The chimeric molecule of 12, wherein said heterologous amino acid sequence is an epitope tag sequence or an Fc region of an immunoglobulin.
14. An antibody which specifically binds to a polypeptide according tc 10.
15. The antibody of 14, wherein said antibody is a monoclonal antibody, a humanized antibody or a single-chain antibody.
16. A composition of matter comprising (a) a polypeptide of 10, (b) an agonist of said polypeptide, (c) an antagonist of said polypeptide, or (d) an antibody that binds to said polypeptide, in combination with a carrier.
17. The composition of matter of 16, wherein said carrier is a pharmaceutically acceptable carrier.
18. The composition of matter of 16 comprising a therapeutically effective amount of (a), (b), (c) or (d).
19. An article of manufacture, comprising:
   a container;
   a label on said container; and
   a composition of matter comprising (a) a polypeptide of 10, (b) an agonist of said polypeptide, (c) an antagonist of said polypeptide, or (d) an antibody that binds to said polypeptide, contained within said container, wherein label on said container indicates that said composition of matter can be used for treating an immune related disease.
20. A method of treating an immune related disorder in a mammal in need thereof comprising administering to said mammal a therapeutically effective amount of (a) a polypeptide of 10, (b) an agonist of said polypeptide, (c) an antagonist of said polypeptide, or (d) an antibody that binds to said polypeptide.
21. The method of 20, wherein the immune related disorder is; systemic lupus erythematosis, X-linked infantile hypogammaglobulinemia, polysaccaride antigen unresponsiveness, selective IgA deficiency, selective IgM deficiency, selective deficiency of IgG subclasses, immunodeficiency with hyper Ig-M, transient hypogammaglobulinemia of infancy, Burkitt's lymphoma, Intermediate lymphoma, follicular lymphoma, typeII hypersensitivity, rheumatoid arthritis, autoimmune mediated hemolytic anemia, myesthenia gravis, hypoadrenocorticism, glomerulonephritis and ankylosing spondylitis.
22. A method for determining the presence of a PRO polypeptide in a sample suspected of containing said polypeptide, said method comprising exposing said sample to an anti-PRO71061, anti-PRO1265, anti-PRO6013, anti-PRO71042, anti-PRO71236, anti-PRO3813, anti-PRO71237, anti-PRO38838, anti-PRO71238, anti-PRO71239, anti-PRO71240, anti-PR071241, anti-PR071242 or anti-PRO710 antibody and determining binding of said antibody to a component of said sample.
23. A method of diagnosing an immune related disease in a mammal, said method comprising detecting the level of expression of a gene encoding PRO71061, PRO1265, PRO6013, PRO71042, PRO71236, PRO3813, PRO71237, PRO38838, PRO71238, PRO71239, PRO71240, PRO71241, PRO71242 or PRO71044 polypeptide (a) in a test sample of tissue cells obtained from the mammal, and (b) in a control sample of known normal tissue cells of the same cell type, wherein a higher or lower level of expression of said gene in the test sample as compared to the control sample is indicative of the presence of an immune related disease in the mammal from which the test tissue cells were obtained.
24. A method of diagnosing an immune related disease in a mammal, said method comprising (a) contacting an anti-PRO71061, anti-PRO1265, anti-PRO6013, anti-PRO71042, anti-PRO71236, anti-PRO3813, anti-PRO71237, anti-PRO38838, anti-PRO71238, anti-PRO71239, anti-PRO71240, anti-PRO71241, anti-PRO71242 or anti-PRO71044 antibody with a test sample of tissue cells obtained from said mammal and (b) detecting the formation of a complex between the antibody and the polypeptide in the test sample, wherein formation of said complex is indicative of the presence of an immune related disease in the mammal from which the test tissue cells were obtained.
25. A method of identifying a compound that inhibits the activity of PRO71061, PRO1265, PRO6013, PRO71042, PRO71236, PRO3813, PRO71237, PRO38838, PRO71238, PRO71239, PRO71240, PRO71241, PRO71242 or PRO71044 polypeptide, said method comprising contacting cells which normally respond to said polypeptide with (a) said polypeptide and (b) a candidate compound, and determining the lack responsiveness by said cell to (a).
26. A method of identifying a compound that inhibits the expression of a gene encoding a PRO71061, PRO1265, PRO6013, PRO71042, PRO71236, PRO3813, PRO71237, PRO38838, PRO71238, PRO71239, PRO71240, PRO71241, PRO71242 or PRO71044 polypeptide, said method comprising contacting cells which normally express said polypeptide with a candidate compound, and determining the lack of expression said gene.
27. The method of 26, wherein said candidate compound is an antisense nucleic acid.
28. A method of identifying a compound that mimics the activity of a PRO71061, PRO1265, PRO6013, PRO71042, PRO71236, PRO3813, PRO71237, PRO38838, PRO71238, PRO71239, PRO71240, PRO71241, PRO71242 or PRO71044 polypeptide, said method comprising contacting cells which normally respond to said polypeptide with a candidate compound, and determining the responsiveness by said cell to said candidate compound.
31. A method of stimulating the immune response in a mammal, said method comprising administering to said mammal an effective amount of a PRO71061, PRO1265, PRO6013, PRO71042, PRO71236, PRO3813, PRO71237, PRO38838, PRO71238, PRO71239, PRO71240, PRO71241, PRO71242 or PRO71044 polypeptide antagonist, wherein said immune response is stimulated.
32. A method of diagnosing a B-cell mediated immune response in a mammal, said method comprising detecting the level of expression of a gene encoding PRO71061, PRO1265, PRO6013, PRO71042, PRO71236, PRO3813, PRO71237, PRO38838, PRO71238, PRO71239, PRO71240, PRO71241, PRO71242 or PRO71044 polypeptide (a) in a test sample of tissue cells obtained from the mammal, and (b) in a control sample of known normal tissue cells of the same cell type, wherein a higher or lower level of expression of said gene in the test sample as compared to the control sample is indicative of the presence of an inflammatory immune response in the mammal from which the test tissue cells were obtained.
33. A method of diagnosing a B cell mediated disease in a mammal, said method comprising detecting the level of expression of a gene encoding PR071061, PRO1265, PRO6013, PRO71042, PRO71236, PRO3813, PRO71237, PRO38838, PRO71238, PRO71239, PRO71240, PRO71241, PRO71242 or PRO71044 polypeptide (a) in a test sample of tissue cells obtained from the mammal, and (b) in a control sample of known normal tissue cells of the same cell type, wherein a higher or lower level of expression of said gene in the test sample as compared to the control sample is indicative of the presence of an inflammatory immune response in the mammal from which the test tissue cells were obtained.

## Claims

1. A method of diagnosing B cell mediated disease in a mammal, said method comprising:
detecting the level of expression of a gene encoding PR071061 polypeptide, wherein the gene has at least 80% nucleic acid sequence identity with the nucleic acid sequence of SEQ ID NO: 1 (a) in a test sample of tissue cells obtained from the mammal, and (b) in a control sample of known normal tissue cells of the same cell type, wherein a higher or lower level of expression of said gene in the test sample as compared to the control sample is indicative of the presence of B cell mediated disease in the mammal from which the test tissue cells were obtained; or
(a) contacting an anti-PR071061 antibody with a test sample of tissue cells obtained from said mammal, wherein said antibody specifically binds a PRO71061 polypeptide having at least 80% amino acid sequence identity with the amino acid sequence of SEQ ID NO: 2, and (b) detecting the formation of a complex between the antibody and the polypeptide in the test sample, wherein formation of said complex is indicative of the presence of B cell mediated disease in the mammal from which the test tissue cells were obtained.

2. The method of claim 1, wherein the gene encoding PR071061 has the nucleic acid sequence of SEQ ID NO: 1.

3. The method of claim 1 or 2, wherein the antibody specifically binds to a PR071061 polypeptide with the amino acid sequence shown in SEQ ID NO: 2.

4. The method of any one of claims 1 to 3, wherein a larger quantity of complexes formed in the test sample indicates the presence or absence of an immune disease in the mammal from which the test tissue cells were obtained.

5. The method of any one of claims 1 to 4, wherein the antibody carries a detectable label.

6. The method of any one of claim 1 to 5, wherein complex formation is monitored by light microscopy, flow cytometry, or fluorimetry.

7. The method of any one of claims 1 to 6, wherein the B cell mediated disease is any one of: systemic lupus erythematosis, X-linked infantile hypogammaglobulinemia, polysaccharide antigen unresponsiveness, selective IgA deficiency, selective IgM deficiency, selective deficiency of IgG subclasses, immunodeficiency with hyper Ig-M, transient hypogammaglobulinemia of infancy, Burkitt's lymphoma, Intermediate lymphoma, follicular lymphoma, type II hypersensitivity, rheumatoid arthritis, autoimmune mediated hemolytic anemia, myesthenia gravis, glomerulonephritis and ankylosing spondylitis.

8. A method of diagnosing a B-cell mediated immune response in a mammal, said method comprising detecting the level of expression of a gene encoding PR07I061 polypeptide, wherein the gene has at least 80% nucleic acid sequence identity with the nucleic acid sequence of SEQ ID NO: 1,
(a) in a test sample of tissue cells obtained from the mammal, and
(b) in a control sample of known normal tissue cells of the same cell type,
wherein a higher or lower level of expression of said gene in the test sample as compared to the control sample is indicative of the presence of a B-cell mediated immune response in the mammal from which the test tissue cells were obtained.

9. The method of claim 8, wherein the gene encoding PR071061 has the nucleic acid sequence of SEQ ID NO: 1.


Sequence Listing

<110> GENENTECH, INC.

<120> COMPOSITIONS AND METHODS FOR THE TREATMENT OF IMMUNE
      RELATED DISEASES

<130> SMW/FP6746523

<140> EP 11151848.6
<141> 2003-07-08

<150> EP 10004913.9
<151> 2003-07-08

<150> EP 03763223.9
<151> 2003-07-08

<150> PCT/US03/21083
<151> 2003-07-08

<150> US 60/394,485
<151> 2002-07-08

<160> 28

<210> 1
<211> 1816
<212> DNA
<213> Homo sapien

<400> 1
 gcacgagcga tgtcgctcgt gctgctaagc ctggccgcgc tgtgcaggag 50

 cgccgtaccc cgagagccga ccgttcaatg tggctctgaa actgggccat 100

 ctccagagtg gatgctacaa catgatctaa tccccggaga cttgagggac 150

 ctccgagtag aacctgttac aactagtgtt gcaacagggg actattcaat 200

 tttgatgaat gtaagctggg tactccgggc agatgccagc atccgcttgt 250

 tgaaggccac caagatttgt gtgacgggca aaagcaactt ccagtcctac 300

 agctgtgtga ggtgcaatta cacagaggcc ttccagactc agaccagacc 350

 ctctggtggt aaatggacat tttcctacat cggcttccct gtagagctga 400

 acacagtcta tttcattggg gcccataata ttcctaatgc aaatatgaat 450

 gaagatggcc cttccatgtc tgtgaatttc acctcaccag gctgcctaga 500

 ccacataatg aaatataaaa aaaagtgtgt caaggccgga agcctgtggg 550

 atccgaacat cactgcttgt aagaagaatg aggagacagt agaagtgaac 600

 ttcacaacca ctcccctggg aaacagatac atggctctta tccaacacag 650

 cactatcatc gggttttctc aggtgtttga gccacaccag aagaaacaaa 700

 cgcgagcttc agtggtgatt ccagtgactg gggatagtga aggtgctacg 750

 gtgcagctga ctccatattt tcctacttgt ggcagcgact gcatccgaca 800

 taaaggaaca gttgtgctct gcccacaaac aggcgtccct ttccctctgg 850

 ataacaacaa aagcaagccg ggaggctggc tgcctctcct cctgctgtct 900

ctgctggtgg ccacatgggt gctggtggca gggatctatc taatgtggag 950

gcacgaaagg atcaagaaga cttccttttc taccaccaca ctactgcccc 1000

ccattaaggt tcttgtggtt tacccatctg aaatatgttt ccatcacaca 1050

atttgttact tcactgaatt tcttcaaaac cattgcagaa gtgaggtcat 1100

ccttgaaaag tggcagaaaa agaaaatagc agagatgggt ccagtgcagt 1150

ggcttgccac tcaaaagaag gcagcagaca aagtcgtctt ccttctttcc 1200

aatgacgtca acagtgtgtg cgatggtacc tgtggcaaga gcgagggcag 1250

tcccagtgag aactctcaag actcttcccc ttgcctttaa ccttttctgc 1300

agtgatctaa gaagccagat tcatctgcac aaatacgtgg tggtctactt 1350

tagagagatt gatacaaaag acgattacaa tgctctcagt gtctgcccca 1400

agtaccacct catgaaggat gccactgctt tctgtgcaga acttctccat 1450

gtcaagtagc aggtgtcagc aggaaaaaga tcacaagcct gccacgatgg 1500

ctgctgctcc ttgtagccca cccatgagaa gcaagagacc ttaaaggctt 1550

cctatcccac caattacagg gaaaaaacgt gtgatgatcc tgaagcttac 1600

tatgcagcct acaaacagcc ttagtaatta aaacatttta taccaataaa 1650

attttcaaat attgctaact aatgtagcat taactaacga ttggaaacta 1700

catttacaac ttcaaagctg ttttatacat agaaatcaat tacagtttta 1750

attgaaaact ataaccattt tgataatgca acaataaagc atcttcagcc 1800

aaaaaaaaaa aaaaaa 1816

<210> 2
<211> 426
<212> PRT
<213> Homo sapien

<400> 2
Met Ser Leu Val Leu Leu Ser Leu Ala Ala Leu Cys Arg Ser Ala
1               5                   10                  15

Val Pro Arg Glu Pro Thr Val Gln Cys Gly Ser Glu Thr Gly Pro
                20                  25                  30

Ser Pro Glu Trp Met Leu Gln His Asp Leu Ile Pro Gly Asp Leu
                35                  40                  45

Arg Asp Leu Arg Val Glu Pro Val Thr Thr Ser Val Ala Thr Gly
                50                  55                  60

Asp Tyr Ser Ile Leu Met Asn Val Ser Trp Val Leu Arg Ala Asp
                65                  70                  75

Ala Ser Ile Arg Leu Leu Lys Ala Thr Lys Ile Cys Val Thr Gly
                80                  85                  90

Lys Ser Asn Phe Gln Ser Tyr Ser Cys Val Arg Cys Asn Tyr Thr
                95                  100                 105

Glu Ala Phe Gln Thr Gln Thr Arg Pro Ser Gly Gly Lys Trp Thr
                110                 115                 120

Phe Ser Tyr Ile Gly Phe Pro Val Glu Leu Asn Thr Val Tyr Phe
                125                 130                 135

Ile Gly Ala His Asn Ile Pro Asn Ala Asn Met Asn Glu Asp Gly
                140                 145                 150

Pro Ser Met Ser Val Asn Phe Thr Ser Pro Gly Cys Leu Asp His
                155                 160                 165

Ile Met Lys Tyr Lys Lys Lys Cys Val Lys Ala Gly Ser Leu Trp
                170                 175                 180

Asp Pro Asn Ile Thr Ala Cys Lys Lys Asn Glu Glu Thr Val Glu
                185                 190                 195

Val Asn Phe Thr Thr Thr Pro Leu Gly Asn Arg Tyr Met Ala Leu
                200                 205                 210

Ile Gln His Ser Thr Ile Ile Gly Phe Ser Gln Val Phe Glu Pro
                215                 220                 225

His Gln Lys Lys Gln Thr Arg Ala Ser Val Val Ile Pro Val Thr
                230                 235                 240

Gly Asp Ser Glu Gly Ala Thr Val Gln Leu Thr Pro Tyr Phe Pro
                245                 250                 255

Thr Cys Gly Ser Asp Cys Ile Arg His Lys Gly Thr Val Val Leu
                260                 265                 270

Cys Pro Gln Thr Gly Val Pro Phe Pro Leu Asp Asn Asn Lys Ser
                275                 280                 285

Lys Pro Gly Gly Trp Leu Pro Leu Leu Leu Leu Ser Leu Leu Val
                290                 295                 300

Ala Thr Trp Val Leu Val Ala Gly Ile Tyr Leu Met Trp Arg His
                305                 310                 315

Glu Arg Ile Lys Lys Thr Ser Phe Ser Thr Thr Thr Leu Leu Pro
                320                 325                 330

Pro Ile Lys Val Leu Val Val Tyr Pro Ser Glu Ile Cys Phe His
                335                 340                 345

His Thr Ile Cys Tyr Phe Thr Glu Phe Leu Gln Asn His Cys Arg
                350                 355                 360

Ser Glu Val Ile Leu Glu Lys Trp Gln Lys Lys Lys Ile Ala Glu
                365                 370                 375

Met Gly Pro Val Gln Trp Leu Ala Thr Gln Lys Lys Ala Ala Asp
                380                 385                 390

Lys Val Val Phe Leu Leu Ser Asn Asp Val Asn Ser Val Cys Asp
                395                 400                 405

Gly Thr Cys Gly Lys Ser Glu Gly Ser Pro Ser Glu Asn Ser Gln
                410                 415                 420

Asp Ser Ser Pro Cys Leu
                425

<210> 3
<211> 1798
<212> DNA
<213> Homo sapien

<400> 3
gacagtggag ggcagtggag aggaccgcgc tgtcctgctg tcaccaagag 50
ctggagacac catctcccac cgagagtcat ggccccattg gccctgcacc 100
tcctcgtcct cgtccccatc ctcctcagcc tggtggcctc ccaggactgg 150
aaggctgaac gcagccaaga ccccttcgag aaatgcatgc aggatcctga 200
ctatgagcag ctgctcaagg tggtgacctg ggggctcaat cggaccctga 250
agccccagag ggtgattgtg gttggcgctg gtgtggccgg gctggtggcc 300
gccaaggtgc tcagcgatgc tggacacaag gtcaccatcc tggaggcaga 350
taacaggatc gggggccgca tcttcaccta ccgggaccag aacacgggct 400
ggattgggga gctgggagcc atgcgcatgc ccagctctca caggatcctc 450
cacaagctct gccagggcct ggggctcaac ctgaccaagt tcacccagta 500
cgacaagaac acgtggacgg aggtgcacga agtgaagctg cgcaactatg 550
tggtggagaa ggtgcccgag aagctgggct acgccttgcg tccccaggaa 600
aagggccact cgcccgaaga catctaccag atggctctca accaggccct 650
caaagacctc aaggcactgg gctgcagaaa ggcgatgaag aagtttgaaa 700
ggcacacgct cttggaatat cttctcgggg aggggaacct gagccggccg 750
gccgtgcagc ttctgggaga cgtgatgtcc gaggatggct tcttctatct 800
cagcttcgcc gaggccctcc gggcccacag ctgcctcagc gacagactcc 850
agtacagccg catcgtgggt ggctgggacc tgctgccgcg cgcgctgctg 900
agctcgctgt ccgggcttgt gctgttgaac gcgcccgtgg tggcgatgac 950
ccagggaccg cacgatgtgc acgtgcagat cgagacctct cccccggcgc 1000
ggaatctgaa ggtgctgaag gccgacgtgg tgctgctgac ggcgagcgga 1050
ccggcggtga agcgcatcac cttctcgccg ccgctgcccc gccacatgca 1100
ggaggcgctg cggaggctgc actacgtgcc ggccaccaag gtgttcctaa 1150
gcttccgcag gcccttctgg cgcgaggagc acattgaagg cggccactca 1200
aacaccgatc gcccgtcgcg catgattttc tacccgccgc cgcgcgaggg 1250
cgcgctgctg ctggcctcgt acacgtggtc ggacgcggcg gcagcgttcg 1300
ccggcttgag ccgggaagag gcgttgcgct tggcgctcga cgacgtggcg 1350
gcattgcacg ggcctgtcgt gcgccagctc tgggacggca ccggcgtcgt 1400
caagcgttgg gcggaggacc agcacagcca gggtggcttt gtggtacagc 1450
cgccggcgct ctggcaaacc gaaaaggatg actggacggt cccttatggc 1500
cgcatctact ttgccggcga gcacaccgcc tacccgcacg gctgggtgga 1550
gacggcggtc aagtcggcgc tgcgcgccgc catcaagatc aacagccgga 1600
aggggcctgc atcggacacg gccagccccg aggggcacgc atctgacatg 1650
gaggggcagg ggcatgtgca tggggtggcc agcagcccct cgcatgacct 1700

ggcaaaggaa gaaggcagcc accctccagt ccaaggccag ttatctctcc 1750

aaaacacgac ccacacgagg acctcgcatt aaagtatttt cggaaaaa 1798

<210> 4
<211> 567
<212> PRT
<213> Homo sapien

<400> 4
Met Ala Pro Leu Ala Leu His Leu Leu Val Leu Val Pro Ile Leu
1               5                   10                  15

Leu Ser Leu Val Ala Ser Gln Asp Trp Lys Ala Glu Arg Ser Gln
                20                  25                  30

Asp Pro Phe Glu Lys Cys Met Gln Asp Pro Asp Tyr Glu Gln Leu
                35                  40                  45

Leu Lys Val Val Thr Trp Gly Leu Asn Arg Thr Leu Lys Pro Gln
                50                  55                  60

Arg Val Ile Val Val Gly Ala Gly Val Ala Gly Leu Val Ala Ala
                65                  70                  75

Lys Val Leu Ser Asp Ala Gly His Lys Val Thr Ile Leu Glu Ala
                80                  85                  90

Asp Asn Arg Ile Gly Gly Arg Ile Phe Thr Tyr Arg Asp Gln Asn
                95                  100                 105

Thr Gly Trp Ile Gly Glu Leu Gly Ala Met Arg Met Pro Ser Ser
                110                 115                 120

His Arg Ile Leu His Lys Leu Cys Gln Gly Leu Gly Leu Asn Leu
                125                 130                 135

Thr Lys Phe Thr Gln Tyr Asp Lys Asn Thr Trp Thr Glu Val His
                140                 145                 150

Glu Val Lys Leu Arg Asn Tyr Val Val Glu Lys Val Pro Glu Lys
                155                 160                 165

Leu Gly Tyr Ala Leu Arg Pro Gln Glu Lys Gly His Ser Pro Glu
                170                 175                 180

Asp Ile Tyr Gln Met Ala Leu Asn Gln Ala Leu Lys Asp Leu Lys
                185                 190                 195

Ala Leu Gly Cys Arg Lys Ala Met Lys Lys Phe Glu Arg His Thr
                200                 205                 210

Leu Leu Glu Tyr Leu Leu Gly Glu Gly Asn Leu Ser Arg Pro Ala
                215                 220                 225

Val Gln Leu Leu Gly Asp Val Met Ser Glu Asp Gly Phe Phe Tyr
                230                 235                 240

Leu Ser Phe Ala Glu Ala Leu Arg Ala His Ser Cys Leu Ser Asp
                245                 250                 255

Arg Leu Gln Tyr Ser Arg Ile Val Gly Gly Trp Asp Leu Leu Pro
                260                 265                 270

Arg Ala Leu Leu Ser Ser Leu Ser Gly Leu Val Leu Leu Asn Ala
                275                 280                 285

Pro Val Val Ala Met Thr Gln Gly Pro His Asp Val His Val Gln
                290                 295                 300

Ile Glu Thr Ser Pro Pro Ala Arg Asn Leu Lys Val Leu Lys Ala
                305                 310                 315

Asp Val Val Leu Leu Thr Ala Ser Gly Pro Ala Val Lys Arg Ile
                320                 325                 330

Thr Phe Ser Pro Pro Leu Pro Arg His Met Gln Glu Ala Leu Arg
                335                 340                 345

Arg Leu His Tyr Val Pro Ala Thr Lys Val Phe Leu Ser Phe Arg
                350                 355                 360

Arg Pro Phe Trp Arg Glu Glu His Ile Glu Gly Gly His Ser Asn
                365                 370                 375

Thr Asp Arg Pro Ser Arg Met Ile Phe Tyr Pro Pro Pro Arg Glu
                380                 385                 390

Gly Ala Leu Leu Leu Ala Ser Tyr Thr Trp Ser Asp Ala Ala Ala
                395                 400                 405

Ala Phe Ala Gly Leu Ser Arg Glu Glu Ala Leu Arg Leu Ala Leu
                410                 415                 420

Asp Asp Val Ala Ala Leu His Gly Pro Val Val Arg Gln Leu Trp
                425                 430                 435

Asp Gly Thr Gly Val Val Lys Arg Trp Ala Glu Asp Gln His Ser
                440                 445                 450

Gln Gly Gly Phe Val Val Gln Pro Pro Ala Leu Trp Gln Thr Glu
                455                 460                 465

Lys Asp Asp Trp Thr Val Pro Tyr Gly Arg Ile Tyr Phe Ala Gly
                470                 475                 480

Glu His Thr Ala Tyr Pro His Gly Trp Val Glu Thr Ala Val Lys
                485                 490                 495

Ser Ala Leu Arg Ala Ala Ile Lys Ile Asn Ser Arg Lys Gly Pro
                500                 505                 510

Ala Ser Asp Thr Ala Ser Pro Glu Gly His Ala Ser Asp Met Glu
                515                 520                 525

Gly Gln Gly His Val His Gly Val Ala Ser Ser Pro Ser His Asp
                530                 535                 540

Leu Ala Lys Glu Glu Gly Ser His Pro Pro Val Gln Gly Gln Leu
                545                 550                 555

Ser Leu Gln Asn Thr Thr His Thr Arg Thr Ser His
                560                 565

<210> 5
<211> 3314
<212> DNA
<213> Homo sapien

<400> 5
ggaggcaggc ggtgccgcgg cgccgggacc cgactcatcc ggtgcttgcg 50

tgtggtggtg agcgcagcgc cgaggatgag gaggtgcaac agcggctccg 100

ggccgccgcc gtcgctgctg ctgctgctgc tgtggctgct cgcggttccc 150

ggcgctaacg cggccccgcg gtcggcgctc tattcgcctt ccgacccgct 200
gacgctgctg caggcggaca cggtgcgcgg cgcggtgctg ggctcccgca 250
gcgcctgggc cgtggagttc ttcgcctcct ggtgcggcca ctgcatcgcc 300
ttcgccccga cgtggaaggc gctggccgaa gacgtcaaag cctggaggcc 350
ggccctgtat ctcgccgccc tggactgtgc tgaggagacc aacagtgcag 400
tctgcagaga cttcaacatc cctggcttcc cgactgtgag gttcttcaag 450
gcctttacca agaacggctc gggagcagta tttccagtgg ctggtgctga 500
cgtgcagacg ctgcgggaga ggctcattga cgccctggag tcccatcatg 550
acacgtggcc cccagcctgt cccccactgg agcctgccaa gctggaggag 600
attgatggat tctttgcgag aaataacgaa gagtacctgg ctctgatctt 650
tgaaaaggga ggctcctacc tgggtagaga ggtggctctg gacctgtccc 700
agcacaaagg cgtggcggtg cgcagggtgc tgaacacaga ggccaatgtg 750
gtgagaaagt ttggtgtcac cgacttcccc tcttgctacc tgctgttccg 800
gaatggctct gtctcccgag tccccgtgct catggaatcc aggtccttct 850
ataccgctta cctgcagaga ctctctgggc tcaccaggga ggctgcccag 900
accacagttg caccaaccac tgctaacaag atagctccca ctgtttggaa 950
attggcagat cgctccaaga tctacatggc tgacctggaa tctgcactgc 1000
actacatcct gcggatagaa gtgggcaggt tcccggtcct ggaagggcag 1050
cgcctggtgg ccctgaaaaa gtttgtggca gtgctggcca agtatttccc 1100
tggccggccc ttagtccaga acttcctgca ctccgtgaat gaatggctca 1150
agaggcagaa gagaaataaa attccctaca gtttctttaa aactgccctg 1200
gacgacagga aagagggtgc cgttcttgcc aagaaggtga actggattgg 1250
ctgccagggg agtgagccgc atttccgggg ctttccctgc tccctgtggg 1300
tcctcttcca cttcttgact gtgcaggcag ctcggcaaaa tgtagaccac 1350
tcacaggaag cagccaaggc caaggaggtc ctcccagcca tccgaggcta 1400
cgtgcactac ttcttcggct gccgagactg cgctagccac ttcgagcaga 1450
tggctgctgc ctccatgcac cgggtgggga gtcccaacgc cgctgtcctc 1500
tggctctggt ctagccacaa cagggtcaat gctcgccttg caggtgcccc 1550
cagcgaggac ccccagttcc ccaaggtgca gtggccaccc cgtgaacttt 1600
gttctgcctg ccacaatgaa cgcctggatg tgcccgtgtg ggacgtggaa 1650
gccaccctca acttcctcaa ggcccacttc tccccaagca acatcatcct 1700
ggacttccct gcagctgggt cagctgcccg gagggatgtg cagaatgtgg 1750
cagccgcccc agagctggcg atgggagccc tggagctgga aagccggaat 1800
tcaactctgg accctgggaa gcctgagatg atgaagtccc ccacaaacac 1850

caccccacat gtgccggctg agggacctga ggcaagtcga cccccgaagc 1900

tgcaccctgg cctcagagct gcaccaggcc aggagcctcc tgagcacatg 1950

gcagagcttc agaggaatga gcaggagcag ccgcttgggc agtggcactt 2000

gagcaagcga gacacagggg ctgcattgct ggctgagtcc agggctgaga 2050

agaaccgcct ctggggccct ttggaggtca ggcgcgtggg ccgcagctcc 2100

aagcagctgg tcgacatccc tgagggccag ctggaggccc gagctggacg 2150

gggccgaggc cagtggctgc aggtgctggg agggggcttc tcttacctgg 2200

acatcagcct ctgtgtgggg ctctattccc tgtccttcat gggcctgctg 2250

gccatgtaca cctacttcca ggccaagata agggccctga agggccatgc 2300

tggccaccct gcagcctgaa ccacctgggg aggaggcggg agagggagct 2350

gccatctcta ggcacctcaa gccccctgac cccattccct cccctcccac 2400

cccttgctcc ttgtctggcc tagaagtgtg ggaaattcag gaaaacgagt 2450

tgctccagtg aagcttcttg gggttgctag gacagagagc tcctttgaca 2500

caaaagacag gagcagggtc caggttcccc tgctgtgcag ggagggcagc 2550

cccgggcagt gggcataggg cagctcagtc cctggcctct tagcaccaca 2600

ttcctgtttt tcagcttatt tgaagtcctg cctcattctc actggagcct 2650

cagtctctcc tgcttggtct tggccctcaa ctggggcaag tgaagccaga 2700

ggagggtccc ccagctgggt gggctggaat ggaactcctc actagctgct 2750

ggggctccgc ccaccctgct cccttccgga caatgaagaa gcctttgcac 2800

cctgggagga aggaccaccc cgggccctct atgcctggcc agcctccagc 2850

tcctcagacc tcctgggtgg ggtttggctt cagggtgggg tttggaagct 2900

tctggaagtc gtgctggtct cccaggtgag gcaagccatg gttgctgggc 2950

tgtagggtga gtggcttgct tggtgggacc tgacgagttg gtggcatggg 3000

aaggatgtgg gtctctagtg ccttgccctg gcttagctgc aggagaagat 3050

ggctgctttc acttcccccc attgagctct gctccctctg agcctggtct 3100

tttgtccttt tttattttgg tctccaagat gaatgctcat ctttggaggg 3150

tgccaggtag aagctaggga ggggagtgtc ttctctctcc aggtttcacc 3200

ttccagtgtg cagaagttag aagggtctgg cgggggcagt gccttacaca 3250

tgcttgattc ccacgctacc ccctgccttg ggaggtgtgt ggaataaatt 3300

atttttgtta aggc 3314

<210> 6
<211> 747
<212> PRT
<213> Homo sapien

<400> 6
Met Arg Arg Cys Asn Ser Gly Ser Gly Pro Pro Pro Ser Leu Leu

1               5                   10                  15

Leu Leu Leu Leu Trp Leu Leu Ala Val Pro Gly Ala Asn Ala Ala
                20                  25                  30

Pro Arg Ser Ala Leu Tyr Ser Pro Ser Asp Pro Leu Thr Leu Leu
                35                  40                  45

Gln Ala Asp Thr Val Arg Gly Ala Val Leu Gly Ser Arg Ser Ala
                50                  55                  60

Trp Ala Val Glu Phe Phe Ala Ser Trp Cys Gly His Cys Ile Ala
                65                  70                  75

Phe Ala Pro Thr Trp Lys Ala Leu Ala Glu Asp Val Lys Ala Trp
                80                  85                  90

Arg Pro Ala Leu Tyr Leu Ala Ala Leu Asp Cys Ala Glu Glu Thr
                95                  100                 105

Asn Ser Ala Val Cys Arg Asp Phe Asn Ile Pro Gly Phe Pro Thr
                110                 115                 120

Val Arg Phe Phe Lys Ala Phe Thr Lys Asn Gly Ser Gly Ala Val
                125                 130                 135

Phe Pro Val Ala Gly Ala Asp Val Gln Thr Leu Arg Glu Arg Leu
                140                 145                 150

Ile Asp Ala Leu Glu Ser His His Asp Thr Trp Pro Pro Ala Cys
                155                 160                 165

Pro Pro Leu Glu Pro Ala Lys Leu Glu Glu Ile Asp Gly Phe Phe
                170                 175                 180

Ala Arg Asn Asn Glu Glu Tyr Leu Ala Leu Ile Phe Glu Lys Gly
                185                 190                 195

Gly Ser Tyr Leu Gly Arg Glu Val Ala Leu Asp Leu Ser Gln His
                200                 205                 210

Lys Gly Val Ala Val Arg Arg Val Leu Asn Thr Glu Ala Asn Val
                215                 220                 225

Val Arg Lys Phe Gly Val Thr Asp Phe Pro Ser Cys Tyr Leu Leu
                230                 235                 240

Phe Arg Asn Gly Ser Val Ser Arg Val Pro Val Leu Met Glu Ser
                245                 250                 255

Arg Ser Phe Tyr Thr Ala Tyr Leu Gln Arg Leu Ser Gly Leu Thr
                260                 265                 270

Arg Glu Ala Ala Gln Thr Thr Val Ala Pro Thr Thr Ala Asn Lys
                275                 280                 285

Ile Ala Pro Thr Val Trp Lys Leu Ala Asp Arg Ser Lys Ile Tyr
                290                 295                 300

Met Ala Asp Leu Glu Ser Ala Leu His Tyr Ile Leu Arg Ile Glu
                305                 310                 315

Val Gly Arg Phe Pro Val Leu Glu Gly Gln Arg Leu Val Ala Leu
                320                 325                 330

Lys Lys Phe Val Ala Val Leu Ala Lys Tyr Phe Pro Gly Arg Pro
                335                 340                 345

Leu Val Gln Asn Phe Leu His Ser Val Asn Glu Trp Leu Lys Arg
                350                 355                 360

Gln Lys Arg Asn Lys Ile Pro Tyr Ser Phe Phe Lys Thr Ala Leu
                365                 370                 375

Asp Asp Arg Lys Glu Gly Ala Val Leu Ala Lys Lys Val Asn Trp
                380                 385                 390

Ile Gly Cys Gln Gly Ser Glu Pro His Phe Arg Gly Phe Pro Cys
                395                 400                 405

Ser Leu Trp Val Leu Phe His Phe Leu Thr Val Gln Ala Ala Arg
                410                 415                 420

Gln Asn Val Asp His Ser Gln Glu Ala Ala Lys Ala Lys Glu Val
                425                 430                 435

Leu Pro Ala Ile Arg Gly Tyr Val His Tyr Phe Phe Gly Cys Arg
                440                 445                 450

Asp Cys Ala Ser His Phe Glu Gln Met Ala Ala Ala Ser Met His
                455                 460                 465

Arg Val Gly Ser Pro Asn Ala Ala Val Leu Trp Leu Trp Ser Ser
                470                 475                 480

His Asn Arg Val Asn Ala Arg Leu Ala Gly Ala Pro Ser Glu Asp
                485                 490                 495

Pro Gln Phe Pro Lys Val Gln Trp Pro Pro Arg Glu Leu Cys Ser
                500                 505                 510

Ala Cys His Asn Glu Arg Leu Asp Val Pro Val Trp Asp Val Glu
                515                 520                 525

Ala Thr Leu Asn Phe Leu Lys Ala His Phe Ser Pro Ser Asn Ile
                530                 535                 540

Ile Leu Asp Phe Pro Ala Ala Gly Ser Ala Ala Arg Arg Asp Val
                545                 550                 555

Gln Asn Val Ala Ala Ala Pro Glu Leu Ala Met Gly Ala Leu Glu
                560                 565                 570

Leu Glu Ser Arg Asn Ser Thr Leu Asp Pro Gly Lys Pro Glu Met
                575                 580                 585

Met Lys Ser Pro Thr Asn Thr Thr Pro His Val Pro Ala Glu Gly
                590                 595                 600

Pro Glu Ala Ser Arg Pro Pro Lys Leu His Pro Gly Leu Arg Ala
                605                 610                 615

Ala Pro Gly Gln Glu Pro Pro Glu His Met Ala Glu Leu Gln Arg
                620                 625                 630

Asn Glu Gln Glu Gln Pro Leu Gly Gln Trp His Leu Ser Lys Arg
                635                 640                 645

Asp Thr Gly Ala Ala Leu Leu Ala Glu Ser Arg Ala Glu Lys Asn
                650                 655                 660

Arg Leu Trp Gly Pro Leu Glu Val Arg Arg Val Gly Arg Ser Ser
                665                 670                 675

Lys Gln Leu Val Asp Ile Pro Glu Gly Gln Leu Glu Ala Arg Ala
                680                 685                 690

Gly Arg Gly Arg Gly Gln Trp Leu Gln Val Leu Gly Gly Gly Phe
                695                 700                 705

Ser Tyr Leu Asp Ile Ser Leu Cys Val Gly Leu Tyr Ser Leu Ser
                710                 715                 720

Phe Met Gly Leu Leu Ala Met Tyr Thr Tyr Phe Gln Ala Lys Ile
                725                 730                 735

Arg Ala Leu Lys Gly His Ala Gly His Pro Ala Ala
                740                 745

<210> 7
<211> 4565
<212> DNA
<213> Homo sapien

<400> 7
ggcgagctaa gccggaggat gtgcagctgc ggcggcggcg ccggctacga 50

agaggacggg gacaggcgcc gtgcgaaccg agcccagcca gccggaggac 100

gcgggcaggg cgggacggga gcccggactc gtctgccgcc gccgtcgtcg 150

ccgtcgtgcc ggccccgcgt ccccgcgcgc gagcgggagg agccgccgcc 200

acctcgcgcc cgagccgccg ctagcgcgcg ccgggcatgg tcccctctta 250

aaggcgcagg ccgcggcggc gggggcgggc gtgcggaaca aagcgccggc 300

gcggggcctg cgggcggctc gggggccgcg atgggcgcgg cgggcccgcg 350

gcggcggcgg cgctgcccgg gccgggcctc gcggcgctag ggcgggctgg 400

cctccgcggg cgggggcagc gggctgaggg cgcgcggggc ctgcggcggc 450

ggcggcggcg gcggcggcgg cccggcgggc ggagcggcgc gggcatggcc 500

gcgcgcggcc ggcgcgcctg gctcagcgtg ctgctcgggc tcgtcctggg 550

cttcgtgctg gcctcgcggc tcgtcctgcc ccgggcttcc gagctgaagc 600

gagcgggccc acggcgccgc gccagccccg agggctgccg gtccgggcag 650

gcggcggctt cccaggccgg cggggcgcgc ggcgatgcgc gcggggcgca 700

gctctggccg cccggctcgg acccagatgg cggcccgcgc gacaggaact 750

ttctcttcgt gggagtcatg accgcccaga aatacctgca gactcgggcc 800

gtggccgcct acagaacatg gtccaagaca attcctggga aagttcagtt 850

cttctcaagt gagggttctg acacatctgt accaattcca gtagtgccac 900

tacggggtgt ggacgactcc tacccgcccc agaagaagtc cttcatgatg 950

ctcaagtaca tgcacgacca ctacttggac aagtatgaat ggtttatgag 1000

agcagatgat gacgtgtaca tcaaaggaga ccgtctggag aacttcctga 1050

ggagtttgaa cagcagcgag cccctctttc ttgggcagac aggcctgggc 1100

accacggaag aaatgggaaa actggccctg gagcctggtg agaacttctg 1150

catggggggg cctggcgtga tcatgagccg ggaggtgctt cggagaatgg 1200

tgccgcacat tggcaagtgt ctccgggaga tgtacaccac ccatgaggac 1250

gtggaggtgg gaaggtgtgt ccggaggttt gcaggggtgc agtgtgtctg 1300
gtcttatgag atgcagcagc ttttttatga gaattacgag cagaacaaaa 1350
aggggtacat tagagatctc cataacagta aaattcacca agctatcaca 1400
ttacacccca acaaaaaccc accctaccag tacaggctcc acagctacat 1450
gctgagccgc aagatatccg agctccgcca tcgcacaata cagctgcacc 1500
gcgaaattgt cctgatgagc aaatacagca acacagaaat tcataaagag 1550
gacctccagc tgggaatccc tccctccttc atgaggtttc agccccgcca 1600
gcgagaggag attctggaat gggagtttct gactggaaaa tacttgtatt 1650
cggcagttga cggccagccc cctcgaagag gaatggactc cgcccagagg 1700
gaagccttgg acgacattgt catgcaggtc atggagatga tcaatgccaa 1750
cgccaagacc agagggcgca tcattgactt caaagagatc cagtacggct 1800
accgccgggt gaaccccatg tatggggctg agtacatcct ggacctgctg 1850
cttctgtaca aaaagcacaa agggaagaaa atgacggtcc ctgtgaggag 1900
gcacgcgtat ttacagcaga ctttcagcaa aatccagttt gtggagcatg 1950
aggagctgga tgcacaagag ttggccaaga gaatcaatca ggaatctgga 2000
tccttgtcct ttctctcaaa ctccctgaag aagctcgtcc cctttcagct 2050
ccctgggtcg aagagtgagc acaaagaacc caaagataaa aagataaaca 2100
tactgattcc tttgtctggg cgtttcgaca tgtttgtgag atttatggga 2150
aactttgaga agacgtgtct tatccccaat cagaacgtca agctcgtggt 2200
tctgcttttc aattctgact ccaaccctga caaggccaaa caagttgaac 2250
tgatgacaga ttaccgcatt aagtacccta aagccgacat gcagattttg 2300
cctgtgtctg gagagttttc aagagccctg gccctggaag taggatcctc 2350
ccagtttaac aatgaatctt tgctcttctt ctgcgacgtc gacctcgtct 2400
ttactacaga attccttcag cgatgtcgag caaatacagt tctgggccaa 2450
caaatatatt ttccaatcat cttcagccag tatgacccaa agattgttta 2500
tagtgggaaa gttcccagtg acaaccattt tgcctttact cagaaaactg 2550
gcttctggag aaactatggg tttggcatca cgtgtattta taagggagat 2600
cttgtccgag tgggtggctt tgatgtttcc atccaaggct gggggctgga 2650
ggatgtggac cttttcaaca aggttgtcca ggcaggtttg aagacgttta 2700
ggagccagga agtaggagta gtccacgtcc accatcctgt cttttgtgat 2750
cccaatcttg accccaaaca gtacaaaatg tgcttggggt ccaaagcatc 2800
gacctatggg tccacacagc agctggctga gatgtggctg gaaaaaaatg 2850
atccaagtta cagtaaaagc agcaataata atggctcagt gaggacagcc 2900
taatgtccag ctttgctgga aaagacgttt ttaattatct aatttatttt 2950

tcaaaaattt tttgtatgat cagtttttga agtccgtata caaggatata 3000
ttttacaagt ggttttctta cataggactc ctttaagatt gagctttctg 3050
aacaagaagg tgatcagtgt ttgcctttga acacatcttc ttgctgaaca 3100
ttatgtagca gacctgctta actttgactt gaaatgtacc tgatgaacaa 3150
aactttttta aaaaaatgtt ttcttttgag accctttgct ccagtcctat 3200
ggcagaaaac gtgaacattc ctgcaaagta ttattgtaac aaaacactgt 3250
aactctggta aatgttctgt tgtgattgtt aacattccac agattctacc 3300
ttttgtgttt tgtttttttt tttttacaat tgttttaaag ccatttcatg 3350
ttccagttgt aagataagga aatgtgataa tagctgtttc atcattgtct 3400
tcaggagagc tttccagagt tgatcatttc ccctcatggt actctgctca 3450
gcatggccac gtaggttttt tgtttgtttt gttttgttct ttttttgaga 3500
cggagtctca ctctgttacc caggctggaa tgcagtggcg caatcttggc 3550
tcactttaac ctccacttcc ctggttcaag caattcccct gcctttgcct 3600
cccgagtagc tgggattaca ggcacacacc accacgccca gctagttttt 3650
ttgtattttt agtagagacg gggtttcacc atgcaagccc agctggccac 3700
gtaggtttta aagcaagggg cgtgaagaag gcacagtgag gtatgtggct 3750
gttctcgtgg tagttcattc ggcctaaata gacctggcat taaatttcaa 3800
gaaggatttg gcattttctc ttcttgaccc ttctctttaa agggtaaaat 3850
attaatgttt agaatgacaa agatgaatta ttacaataaa tctgatgtac 3900
acagactgaa acacacacac atacacccta atcaaaacgt tggggaaaaa 3950
tgtatttggt tttgttcctt tcatcctgtc tgtgttatgt gggtggagat 4000
ggttttcatt ctttcattac tgttttgttt tatcctttgt atctgaaata 4050
cctttaattt atttaatatc tgttgttcag agctctgcca tttcttgagt 4100
acctgttagt tagtattatt tatgtgtatc gggagtgtgt ttagtctgtt 4150
ttatttgcag taaaccgatc tccaaagatt tccttttgga aacgcttttt 4200
cccctcctta atttttatat tccttactgt tttactaaat attaagtgtt 4250
ctttgacaat tttggtgctc atgtgttttg gggacaaaag tgaaatgaat 4300
ctgtcattat accagaaagt taaattctca gatcaaatgt gccttaataa 4350
atttgttttc atttagattt caaacagtga tagacttgcc attttaatac 4400
acgtcattgg agggctgcgt atttgtaaat agcctgatgc tcatttggaa 4450
aaataaacca gtgaacaata tttttctatt gtacttttca gaaccatttt 4500
gtctcattat tcctgtttta gctgaagaat tgtattacat ttggagagta 4550
aaaaacttaa acacg 4565
<210> 8

<211> 802
<212> PRT
<213> Homo sapien

<400> 8
Met Ala Ala Arg Gly Arg Arg Ala Trp Leu Ser Val Leu Leu Gly
1               5                   10                  15

Leu Val Leu Gly Phe Val Leu Ala Ser Arg Leu Val Leu Pro Arg
                20                  25                  30

Ala Ser Glu Leu Lys Arg Ala Gly Pro Arg Arg Arg Ala Ser Pro
                35                  40                  45

Glu Gly Cys Arg Ser Gly Gln Ala Ala Ala Ser Gln Ala Gly Gly
                50                  55                  60

Ala Arg Gly Asp Ala Arg Gly Ala Gln Leu Trp Pro Pro Gly Ser
                65                  70                  75

Asp Pro Asp Gly Gly Pro Arg Asp Arg Asn Phe Leu Phe Val Gly
                80                  85                  90

Val Met Thr Ala Gln Lys Tyr Leu Gln Thr Arg Ala Val Ala Ala
                95                  100                 105

Tyr Arg Thr Trp Ser Lys Thr Ile Pro Gly Lys Val Gln Phe Phe
                110                 115                 120

Ser Ser Glu Gly Ser Asp Thr Ser Val Pro Ile Pro Val Val Pro
                125                 130                 135

Leu Arg Gly Val Asp Asp Ser Tyr Pro Pro Gln Lys Lys Ser Phe
                140                 145                 150

Met Met Leu Lys Tyr Met His Asp His Tyr Leu Asp Lys Tyr Glu
                155                 160                 165

Trp Phe Met Arg Ala Asp Asp Asp Val Tyr Ile Lys Gly Asp Arg
                170                 175                 180

Leu Glu Asn Phe Leu Arg Ser Leu Asn Ser Ser Glu Pro Leu Phe
                185                 190                 195

Leu Gly Gln Thr Gly Leu Gly Thr Thr Glu Glu Met Gly Lys Leu
                200                 205                 210

Ala Leu Glu Pro Gly Glu Asn Phe Cys Met Gly Gly Pro Gly Val
                215                 220                 225

Ile Met Ser Arg Glu Val Leu Arg Arg Met Val Pro His Ile Gly
                230                 235                 240

Lys Cys Leu Arg Glu Met Tyr Thr Thr His Glu Asp Val Glu Val
                245                 250                 255

Gly Arg Cys Val Arg Arg Phe Ala Gly Val Gln Cys Val Trp Ser
                260                 265                 270

Tyr Glu Met Gln Gln Leu Phe Tyr Glu Asn Tyr Glu Gln Asn Lys
                275                 280                 285

Lys Gly Tyr Ile Arg Asp Leu His Asn Ser Lys Ile His Gln Ala
                290                 295                 300

Ile Thr Leu His Pro Asn Lys Asn Pro Pro Tyr Gln Tyr Arg Leu
                305                 310                 315

His Ser Tyr Met Leu Ser Arg Lys Ile Ser Glu Leu Arg His Arg
                320                 325                 330

Thr Ile Gln Leu His Arg Glu Ile Val Leu Met Ser Lys Tyr Ser
                335                 340                 345

Asn Thr Glu Ile His Lys Glu Asp Leu Gln Leu Gly Ile Pro Pro
                350                 355                 360

Ser Phe Met Arg Phe Gln Pro Arg Gln Arg Glu Glu Ile Leu Glu
                365                 370                 375

Trp Glu Phe Leu Thr Gly Lys Tyr Leu Tyr Ser Ala Val Asp Gly
                380                 385                 390

Gln Pro Pro Arg Arg Gly Met Asp Ser Ala Gln Arg Glu Ala Leu
                395                 400                 405

Asp Asp Ile Val Met Gln Val Met Glu Met Ile Asn Ala Asn Ala
                410                 415                 420

Lys Thr Arg Gly Arg Ile Ile Asp Phe Lys Glu Ile Gln Tyr Gly
                425                 430                 435

Tyr Arg Arg Val Asn Pro Met Tyr Gly Ala Glu Tyr Ile Leu Asp
                440                 445                 450

Leu Leu Leu Leu Tyr Lys Lys His Lys Gly Lys Lys Met Thr Val
                455                 460                 465

Pro Val Arg Arg His Ala Tyr Leu Gln Gln Thr Phe Ser Lys Ile
                470                 475                 480

Gln Phe Val Glu His Glu Glu Leu Asp Ala Gln Glu Leu Ala Lys
                485                 490                 495

Arg Ile Asn Gln Glu Ser Gly Ser Leu Ser Phe Leu Ser Asn Ser
                500                 505                 510

Leu Lys Lys Leu Val Pro Phe Gln Leu Pro Gly Ser Lys Ser Glu
                515                 520                 525

His Lys Glu Pro Lys Asp Lys Lys Ile Asn Ile Leu Ile Pro Leu
                530                 535                 540

Ser Gly Arg Phe Asp Met Phe Val Arg Phe Met Gly Asn Phe Glu
                545                 550                 555

Lys Thr Cys Leu Ile Pro Asn Gln Asn Val Lys Leu Val Val Leu
                560                 565                 570

Leu Phe Asn Ser Asp Ser Asn Pro Asp Lys Ala Lys Gln Val Glu
                575                 580                 585

Leu Met Thr Asp Tyr Arg Ile Lys Tyr Pro Lys Ala Asp Met Gln
                590                 595                 600

Ile Leu Pro Val Ser Gly Glu Phe Ser Arg Ala Leu Ala Leu Glu
                605                 610                 615

Val Gly Ser Ser Gln Phe Asn Asn Glu Ser Leu Leu Phe Phe Cys
                620                 625                 630

Asp Val Asp Leu Val Phe Thr Thr Glu Phe Leu Gln Arg Cys Arg
                635                 640                 645

Ala Asn Thr Val Leu Gly Gln Gln Ile Tyr Phe Pro Ile Ile Phe
                650                 655                 660

Ser Gln Tyr Asp Pro Lys Ile Val Tyr Ser Gly Lys Val Pro Ser
                665                 670                 675

Asp Asn His Phe Ala Phe Thr Gln Lys Thr Gly Phe Trp Arg Asn
                680                 685                 690

Tyr Gly Phe Gly Ile Thr Cys Ile Tyr Lys Gly Asp Leu Val Arg
                695                 700                 705

Val Gly Gly Phe Asp Val Ser Ile Gln Gly Trp Gly Leu Glu Asp
                710                 715                 720

Val Asp Leu Phe Asn Lys Val Val Gln Ala Gly Leu Lys Thr Phe
                725                 730                 735

Arg Ser Gln Glu Val Gly Val Val His Val His His Pro Val Phe
                740                 745                 750

Cys Asp Pro Asn Leu Asp Pro Lys Gln Tyr Lys Met Cys Leu Gly
                755                 760                 765

Ser Lys Ala Ser Thr Tyr Gly Ser Thr Gln Gln Leu Ala Glu Met
                770                 775                 780

Trp Leu Glu Lys Asn Asp Pro Ser Tyr Ser Lys Ser Ser Asn Asn
                785                 790                 795

Asn Gly Ser Val Arg Thr Ala
                800

<210> 9
<211> 2176
<212> DNA
<213> Homo sapien

<400> 9
tcctgtctca ggcaggccct gcgcctccta tgcggagatg ctactgccac 50

tgctgctgtc ctcgctgctg ggcgggtccc aggctatgga tgggagattc 100

tggatacgag tgcaggagtc agtgatggtg ccggagggcc tgtgcatctc 150

tgtgccctgc tctttctcct acccccgaca agactggaca gggtctaccc 200

cagcttatgg ctactggttc aaagcagtga ctgagacaac caagggtgct 250

cctgtggcca caaaccacca gagtcgagag gtggaaatga gcacccgggg 300

ccgattccag ctcactgggg atcccgccaa ggggaactgc tccttggtga 350

tcagagacgc gcagatgcag gatgagtcac agtacttctt tcgggtggag 400

agaggaagct atgtgagata taatttcatg aacgatgggt tctttctaaa 450

agtaacagcc ctgactcaga agcctgatgt ctacatcccc gagaccctgg 500

agcccgggca gccggtgacg gtcatctgtg tgtttaactg ggcctttgag 550

gaatgtccac ccccttcttt ctcctggacg ggggctgccc tctcctccca 600

aggaaccaaa ccaacgacct cccacttctc agtgctcagc ttcacgccca 650

gaccccagga ccacaacacc gacctcacct gccatgtgga cttctccaga 700

aagggtgtga gcgtacagag gaccgtccga ctccgtgtgg cctatgcccc 750

cagagacctt gttatcagca tttcacgtga caacacgcca gccctggagc 800

cccagcccca gggaaatgtc ccatacctgg aagcccaaaa aggccagttc 850

ctgcggctcc tctgtgctgc tgacagccag ccccctgcca cactgagctg 900

ggtcctgcag aacagagtcc tctcctcgtc ccatccctgg ggccctagac 950

ccctggggct ggagctgccc ggggtgaagg ctggggattc agggcgctac 1000

acctgccgag cggagaacag gcttggctcc cagcagcgag ccctggacct 1050

ctctgtgcag tatcctccag agaacctgag agtgatggtt tcccaagcaa 1100

acaggacagt cctggaaaac cttgggaacg gcacgtctct cccagtactg 1150

gagggccaaa gcctgtgcct ggtctgtgtc acacacagca gccccccagc 1200

caggctgagc tggacccaga ggggacaggt tctgagcccc tcccagccct 1250

cagaccccgg ggtcctggag ctgcctcggg ttcaagtgga gcacgaagga 1300

gagttcacct gccacgctcg gcacccactg ggctcccagc acgtctctct 1350

cagcctctcc gtgcactact ccccgaagct gctgggcccc tcctgctcct 1400

gggaggctga gggtctgcac tgcagctgct cctcccaggc cagcccggcc 1450

ccctctctgc gctggtggct tggggaggag ctgctggagg ggaacagcag 1500

ccaggactcc ttcgaggtca cccccagctc agccgggccc tgggccaaca 1550

gctccctgag cctccatgga gggctcagct ctggcctcag gctccgctgt 1600

gaggcctgga acgtccatgg ggcccagagt ggatccatcc tgcagctgcc 1650

agataagaag ggactcatct caacggcatt ctccaacgga gcgtttctgg 1700

gaatcggcat cacggctctt cttttcctct gcctggccct gatcatcatg 1750

aagattctac cgaagagacg gactcagaca gaaaccccga ggcccaggtt 1800

ctcccggcac agcacgatcc tggattacat caatgtggtc ccgacggctg 1850

gccccctggc tcagaagcgg aatcagaaag ccacaccaaa cagtcctcgg 1900

acccctcttc caccaggtgc tccctcccca gaatcaaaga agaaccagaa 1950

aaagcagtat cagttgccca gtttcccaga acccaaatca tccactcaag 2000

ccccagaatc ccaggagagc caagaggagc tccattatgc cacgctcaac 2050

ttcccaggcg tcagacccag gcctgaggcc cggatgccca agggcaccca 2100

ggcggattat gcagaagtca agttccaatg agggtctctt aggctttagg 2150

actgggactt cggctaggga ggaagg 2176

<210> 10
<211> 697
<212> PRT
<213> Homo sapien

<400> 10
Met Leu Leu Pro Leu Leu Leu Ser Ser Leu Leu Gly Gly Ser Gln
1               5                   10                  15

Ala Met Asp Gly Arg Phe Trp Ile Arg Val Gln Glu Ser Val Met
                20                  25                  30

Val Pro Glu Gly Leu Cys Ile Ser Val Pro Cys Ser Phe Ser Tyr
                35                  40                  45

Pro Arg Gln Asp Trp Thr Gly Ser Thr Pro Ala Tyr Gly Tyr Trp
                50                  55                  60

Phe Lys Ala Val Thr Glu Thr Thr Lys Gly Ala Pro Val Ala Thr
                65                  70                  75

Asn His Gln Ser Arg Glu Val Glu Met Ser Thr Arg Gly Arg Phe
                80                  85                  90

Gln Leu Thr Gly Asp Pro Ala Lys Gly Asn Cys Ser Leu Val Ile
                95                  100                 105

Arg Asp Ala Gln Met Gln Asp Glu Ser Gln Tyr Phe Phe Arg Val
                110                 115                 120

Glu Arg Gly Ser Tyr Val Arg Tyr Asn Phe Met Asn Asp Gly Phe
                125                 130                 135

Phe Leu Lys Val Thr Ala Leu Thr Gln Lys Pro Asp Val Tyr Ile
                140                 145                 150

Pro Glu Thr Leu Glu Pro Gly Gln Pro Val Thr Val Ile Cys Val
                155                 160                 165

Phe Asn Trp Ala Phe Glu Glu Cys Pro Pro Pro Ser Phe Ser Trp
                170                 175                 180

Thr Gly Ala Ala Leu Ser Ser Gln Gly Thr Lys Pro Thr Thr Ser
                185                 190                 195

His Phe Ser Val Leu Ser Phe Thr Pro Arg Pro Gln Asp His Asn
                200                 205                 210

Thr Asp Leu Thr Cys His Val Asp Phe Ser Arg Lys Gly Val Ser
                215                 220                 225

Val Gln Arg Thr Val Arg Leu Arg Val Ala Tyr Ala Pro Arg Asp
                230                 235                 240

Leu Val Ile Ser Ile Ser Arg Asp Asn Thr Pro Ala Leu Glu Pro
                245                 250                 255

Gln Pro Gln Gly Asn Val Pro Tyr Leu Glu Ala Gln Lys Gly Gln
                260                 265                 270

Phe Leu Arg Leu Leu Cys Ala Ala Asp Ser Gln Pro Pro Ala Thr
                275                 280                 285

Leu Ser Trp Val Leu Gln Asn Arg Val Leu Ser Ser Ser His Pro
                290                 295                 300

Trp Gly Pro Arg Pro Leu Gly Leu Glu Leu Pro Gly Val Lys Ala
                305                 310                 315

Gly Asp Ser Gly Arg Tyr Thr Cys Arg Ala Glu Asn Arg Leu Gly
                320                 325                 330

Ser Gln Gln Arg Ala Leu Asp Leu Ser Val Gln Tyr Pro Pro Glu
                335                 340                 345

Asn Leu Arg Val Met Val Ser Gln Ala Asn Arg Thr Val Leu Glu
                350                 355                 360

Asn Leu Gly Asn Gly Thr Ser Leu Pro Val Leu Glu Gly Gln Ser

365                     370                     375

Leu Cys Leu Val Cys Val Thr His Ser Ser Pro Pro Ala Arg Leu
                380                 385                 390

Ser Trp Thr Gln Arg Gly Gln Val Leu Ser Pro Ser Gln Pro Ser
                395                 400                 405

Asp Pro Gly Val Leu Glu Leu Pro Arg Val Gln Val Glu His Glu
                410                 415                 420

Gly Glu Phe Thr Cys His Ala Arg His Pro Leu Gly Ser Gln His
                425                 430                 435

Val Ser Leu Ser Leu Ser Val His Tyr Ser Pro Lys Leu Leu Gly
                440                 445                 450

Pro Ser Cys Ser Trp Glu Ala Glu Gly Leu His Cys Ser Cys Ser
                455                 460                 465

Ser Gln Ala Ser Pro Ala Pro Ser Leu Arg Trp Trp Leu Gly Glu
                470                 475                 480

Glu Leu Leu Glu Gly Asn Ser Ser Gln Asp Ser Phe Glu Val Thr
                485                 490                 495

Pro Ser Ser Ala Gly Pro Trp Ala Asn Ser Ser Leu Ser Leu His
                500                 505                 510

Gly Gly Leu Ser Ser Gly Leu Arg Leu Arg Cys Glu Ala Trp Asn
                515                 520                 525

Val His Gly Ala Gln Ser Gly Ser Ile Leu Gln Leu Pro Asp Lys
                530                 535                 540

Lys Gly Leu Ile Ser Thr Ala Phe Ser Asn Gly Ala Phe Leu Gly
                545                 550                 555

Ile Gly Ile Thr Ala Leu Leu Phe Leu Cys Leu Ala Leu Ile Ile
                560                 565                 570

Met Lys Ile Leu Pro Lys Arg Arg Thr Gln Thr Glu Thr Pro Arg
                575                 580                 585

Pro Arg Phe Ser Arg His Ser Thr Ile Leu Asp Tyr Ile Asn Val
                590                 595                 600

Val Pro Thr Ala Gly Pro Leu Ala Gln Lys Arg Asn Gln Lys Ala
                605                 610                 615

Thr Pro Asn Ser Pro Arg Thr Pro Leu Pro Pro Gly Ala Pro Ser
                620                 625                 630

Pro Glu Ser Lys Lys Asn Gln Lys Lys Gln Tyr Gln Leu Pro Ser
                635                 640                 645

Phe Pro Glu Pro Lys Ser Ser Thr Gln Ala Pro Glu Ser Gln Glu
                650                 655                 660

Ser Gln Glu Glu Leu His Tyr Ala Thr Leu Asn Phe Pro Gly Val
                665                 670                 675

Arg Pro Arg Pro Glu Ala Arg Met Pro Lys Gly Thr Gln Ala Asp
                680                 685                 690

Tyr Ala Glu Val Lys Phe Gln
                695

<210> 11
<211> 1724
<212> DNA
<213> Homo sapien

<400> 11
ccttcatacc ggcccttccc ctcggctttg cctggacagc tcctgcctcc 50

cgcagggccc acctgtgtcc cccagcgccg ctccacccag caggcctgag 100

cccctctctg ctgccagaca ccccctgctg cccactctcc tgctgctcgg 150

gttctgaggc acagcttgtc acaccgaggc ggattctctt tctctttctc 200

ttctggccca cagccgcagc aatggcgctg agttcctctg ctggagttca 250

tcctgctagc tgggttcccg agctgccggt ctgagcctga ggcatggagc 300

ctcctggaga ctgggggcct cctccctgga gatccacccc cagaaccgac 350

gtcttgaggc tggtgctgta tctcaccttc ctgggagccc cctgctacgc 400

cccagctctg ccgtcctgca aggaggacga gtacccagtg ggctccgagt 450

gctgccccaa gtgcagtcca ggttatcgtg tgaaggaggc ctgcggggag 500

ctgacgggca cagtgtgtga accctgccct ccaggcacct acattgccca 550

cctcaatggc ctaagcaagt gtctgcagtg ccaaatgtgt gacccagcca 600

tgggcctgcg cgcgagccgg aactgctcca ggacagagaa cgccgtgtgt 650

ggctgcagcc caggccactt ctgcatcgtc caggacgggg accactgcgc 700

cgcgtgccgc gcttacgcca cctccagccc gggccagagg gtgcagaagg 750

gaggcaccga gagtcaggac accctgtgtc agaactgccc cccggggacc 800

ttctctccca atgggaccct ggaggaatgt cagcaccaga ccaagtgcag 850

ctggctggtg acgaaggccg gagctgggac cagcagctcc cactgggtat 900

ggtggtttct ctcagggagc ctcgtcatcg tcattgtttg ctccacagtt 950

ggcctaatca tatgtgtgaa aagaagaaag ccaaggggtg atgtagtcaa 1000

ggtgatcgtc tccgtccagc ggaaaagaca ggaggcagaa ggtgaggcca 1050

cagtcattga ggccctgcag gcccctccgg acgtcaccac ggtggccgtg 1100

gaggagacaa taccctcatt cacggggagg agcccaaacc actgacccac 1150

agactctgca ccccgacgcc agagatacct ggagcgacgg ctgctgaaag 1200

aggctgtcca cctggcgaaa ccaccggagc ccggaggctt gggggctccg 1250

ccctgggctg gcttccgtct cctccagtgg agggagaggt ggggcccctg 1300

ctggggtaga gctggggacg ccacgtgcca ttcccatggg ccagtgaggg 1350

cctggggcct ctgttctgct gtggcctgag ctccccagag tcctgaggag 1400

gagcgccagt tgcccctcgc tcacagacca cacacccagc cctcctgggc 1450

cagcccagag ggcccttcag accccagctg tctgcgcgtc tgactcttgt 1500

ggcctcagca ggacaggccc cgggcactgc ctcacagcca aggctggact 1550

gggttggctg cagtgtggtg tttagtggat accacatcgg aagtgatttt 1600

ctaaattgga tttgaattcc ggtcctgtct tctatttgtc atgaaacagt 1650

gtatttgggg agatgctgtg ggaggatgta aatatcttgt ttctcctcaa 1700

aaaaaaaaaa aaaaaaaaaa aaaa 1724

<210> 12
<211> 283
<212> PRT
<213> Homo sapien

<400> 12

<210> 13
<211> 1002
<212> DNA
<213> Homo sapien

<400> 13
tgcagtctgt ctgagggcgg ccgaagtggc tggctcattt aagatgaggc 50

ttctgctgct tctcctagtg gcggcgtctg cgatggtccg gagcgaggcc 100

tcggccaatc tgggcggcgt gccagcaaga gattaaagat gcagtacgcc 150

acggggccgc tgctcaagtt ccagatttgt gtttcctgag gttataggcg 200

ggtgtttgag gagtacatgc gggttattag ccagcggtac ccagacatcc 250

gcattgaagg agagaattac ctccctcaac caatatatag acacatagca 300

tctttcctgt cagtcttcaa actagtatta ataggcttaa taattgttgg 350

caaggatcct tttgctttct ttggcatgca agctcctagc atctggcagt 400

ggggccaaga aaataaggtt tatgcatgta tgatggtttt cttcttgagc 450

aacatgattg agaaccagtg tatgtcaaca ggtgcatttg agataacttt 500

aaatgatgta cctgtgtggt ctaagctgga atctggtcac cttccatcca 550

tgcaacaact tgttcaaatt cttgacaatg aaatgaagct caatgtgcat 600

atggattcaa tcccacacca tcgatcatag caccacctat cagcactgaa 650

aactcttttg cattaaggga tcattgcaag agcagcgtga ctgacattat 700

gaaggcctgt actgaagaca gcaagctgtt agtacagacc agatgctttc 750

ttggcaggct cgttgtacct cttggaaaac ctcaatgcaa gatagtgttt 800

cagtgctggc atattttgga attctgcaca ttcatggagt gcaataatac 850

tgtatagctt tcccccacct cccacaaaat cacccagtta atgtgtgtgt 900

gtgtgttttt tttaaggtaa acattactac ttgtaacttt ttttctttag 950

tcatatttgg aaaaagtaga aaattggagt tacatttgga ttttttttcc 1000

aa 1002

<210> 14
<211> 163
<212> PRT
<213> Homo sapien

<220>
<221> Unsure
<222> 17
<223> Unknown amino acid

<400> 14
Met Gln Tyr Ala Thr Gly Pro Leu Leu Lys Phe Gln Ile Cys Val
  1               5                  10                  15

Ser Xaa Gly Tyr Arg Arg Val Phe Glu Glu Tyr Met Arg Val Ile
                 20                  25                  30

Ser Gln Arg Tyr Pro Asp Ile Arg Ile Glu Gly Glu Asn Tyr Leu
                35                  40                  45

Pro Gln Pro Ile Tyr Arg His Ile Ala Ser Phe Leu Ser Val Phe
                50                  55                  60

Lys Leu Val Leu Ile Gly Leu Ile Ile Val Gly Lys Asp Pro Phe
                65                  70                  75

Ala Phe Phe Gly Met Gln Ala Pro Ser Ile Trp Gln Trp Gly Gln
                80                  85                  90

Glu Asn Lys Val Tyr Ala Cys Met Met Val Phe Phe Leu Ser Asn
                95                  100                 105

Met Ile Glu Asn Gln Cys Met Ser Thr Gly Ala Phe Glu Ile Thr
                110                 115                 120

Leu Asn Asp Val Pro Val Trp Ser Lys Leu Glu Ser Gly His Leu
                125                 130                 135

Pro Ser Met Gln Gln Leu Val Gln Ile Leu Asp Asn Glu Met Lys
                140                 145                 150

Leu Asn Val His Met Asp Ser Ile Pro His His Arg Ser
                155                 160

<210> 15
<211> 3002
<212> DNA
<213> Homo sapien

<400> 15
gtggcttggt attcactggc aggtttcaga catttagatc tttcttttaa 50

tgactaacac catgcctatc tgtggagaag ctggcaacat gtcacacctg 100

gaaattgttt ttcaacatta atactattat ttggcagtaa tccagattgc 150

ttttgccacc aacctgaaga catatagagg cagaaggaca ggaataattc 200

tatttgtttc ctgttttgaa acttccatct gtaaggctat caaaaggaga 250

tgtgagagag ggtattgagt ctggcctgac aatgcagttc ttaaaccaaa 300

ggtccattat gcttctcctc tctgagaatc ctgacttacc tcaacaacgg 350

agacatggca cagtagccag cttggagact tctcagccaa tgctctgaga 400

tcaagtcgaa gacccaatat acagggtttt gagctcatct tcatcattca 450

tatgaggaaa taagtggtaa aatccttgga aatacaatga gactcatcag 500

aaacatttac atattttgta gtattgttat gacagcagag ggtgatgctc 550

cagagctgcc agaagaaagg gaactgatga ccaactgctc caacatgtct 600

ctaagaaagg ttcccgcaga cttgacccca gccacaacga cactggattt 650

atcctataac ctcctttttc aactccagag ttcagatttt cattctgtct 700

ccaaactgag agttttgatt ctatgccata acagaattca acagctggat 750

ctcaaaacct ttgaattcaa caaggagtta agatatttag atttgtctaa 800

taacagactg aagagtgtaa cttggtattt actggcaggt ctcaggtatt 850

tagatctttc ttttaatgac tttgacacca tgcctatctg tgaggaagct 900

ggcaacatgt cacacctgga aatcctaggt ttgagtgggg caaaaataca 950
aaaatcagat ttccagaaaa ttgctcatct gcatctaaat actgtcttct 1000
taggattcag aactcttcct cattatgaag aaggtagcct gcccatctta 1050
aacacaacaa aactgcacat tgttttacca atggacacaa atttctgggt 1100
tcttttgcgt gatggaatca agacttcaaa aatattagaa atgacaaata 1150
tagatggcaa aagccaattt gtaagttatg aaatgcaacg aaatcttagt 1200
ttagaaaatg ctaagacatc ggttctattg cttaataaag ttgatttact 1250
ctgggacgac cttttcctta tcttacaatt tgtttggcat acatcagtgg 1300
aacactttca gatccgaaat gtgacttttg gtggtaaggc ttatcttgac 1350
cacaattcat ttgactactc aaatactgta atgagaacta taaaattgga 1400
gcatgtacat ttcagagtgt tttacattca acaggataaa atctatttgc 1450
ttttgaccaa aatggacata gaaaacctga caatatcaaa tgcacaaatg 1500
ccacacatgc ttttcccgaa ttatcctacg aaattccaat atttaaattt 1550
tgccaataat atcttaacag acgagttgtt taaaagaact atccaactgc 1600
ctcacttgaa aactctcatt ttgaatggca ataaactgga gacactttct 1650
ttagtaagtt gctttgctaa caacacaccc ttggaacact tggatctgag 1700
tcaaaatcta ttacaacata aaaatgatga aaattgctca tggccagaaa 1750
ctgtggtcaa tatgaatctg tcatacaata aattgtctga ttctgtcttc 1800
aggtgcttgc ccaaaagtat tcaaatactt gacctaaata ataaccaaat 1850
ccaaactgta cctaaagaga ctattcatct gatggcctta cgagaactaa 1900
atattgcatt taattttcta actgatctcc ctggatgcag tcatttcagt 1950
agactttcag ttctgaacat tgaaatgaac ttcattctca gcccatctct 2000
ggattttgtt cagagctgcc aggaagttaa aactctaaat gcgggaagaa 2050
atccattccg gtgtacctgt gaattaaaaa atttcattca gcttgaaaca 2100
tattcagagg tcatgatggt tggatggtca gattcataca cctgtgaata 2150
ccctttaaac ctaaggggaa ttaggttaaa agacgttcat ctccacgaat 2200
tatcttgcaa cacagctctg ttgattgtca ccattgtggt tattatgcta 2250
gttctggggt tggctgtggc cttctgctgt ctccactttg atctgccctg 2300
gtatctcagg atgctaggtc aatgcacaca aacatggcac agggttagga 2350
aaacaaccca agaacaactc aagagaaatg tccgattcca cgcatttatt 2400
tcatacagtg aacatgattc tctgtgggtg aagaatgaat tgatccccaa 2450
tctagagaag gaagatggtt ctatcttgat ttgcctttat gaaagctact 2500
ttgaccctgg caaaagcatt agtgaaaata ttgtaagctt cattgagaaa 2550
agctataagt ccatctttgt tttgtctccc aactttgtcc agaatgagtg 2600

gtgccattat gaattttact ttgcccacca caatctcttc catgaaaatt 2650

ctgatcatat aattcttatc ttactggaac ccattccatt ctattgcatt 2700

cccaccaggt atcataaact gaaagctctc ctggaaaaaa aagcatactt 2750

ggaatggccc aaggataggc gtaaatgtgg gcttttctgg gcaaaccttc 2800

gagctgctat taatgttaat gtattagcca ccagagaaat gtatgaactg 2850

cagacattca cagagttaaa tgaagagtct cgaggttcta caatctctct 2900

gatgagaaca gattgtctat aaaatcccac agtccttggg aagttgggga 2950

ccacatacac tgttgggatg tacattgata caacctttat gatggcaatt 3000

tg 3002

<210> 16
<211> 811
<212> PRT
<213> Homo sapien

<400> 16
Met Arg Leu Ile Arg Asn Ile Tyr Ile Phe Cys Ser Ile Val Met
1               5                   10                  15

Thr Ala Glu Gly Asp Ala Pro Glu Leu Pro Glu Glu Arg Glu Leu
                20                  25                  30

Met Thr Asn Cys Ser Asn Met Ser Leu Arg Lys Val Pro Ala Asp
                35                  40                  45

Leu Thr Pro Ala Thr Thr Thr Leu Asp Leu Ser Tyr Asn Leu Leu
                50                  55                  60

Phe Gln Leu Gln Ser Ser Asp Phe His Ser Val Ser Lys Leu Arg
                65                  70                  75

Val Leu Ile Leu Cys His Asn Arg Ile Gln Gln Leu Asp Leu Lys
                80                  85                  90

Thr Phe Glu Phe Asn Lys Glu Leu Arg Tyr Leu Asp Leu Ser Asn
                95                  100                 105

Asn Arg Leu Lys Ser Val Thr Trp Tyr Leu Leu Ala Gly Leu Arg
                110                 115                 120

Tyr Leu Asp Leu Ser Phe Asn Asp Phe Asp Thr Met Pro Ile Cys
                125                 130                 135

Glu Glu Ala Gly Asn Met Ser His Leu Glu Ile Leu Gly Leu Ser
                140                 145                 150

Gly Ala Lys Ile Gln Lys Ser Asp Phe Gln Lys Ile Ala His Leu
                155                 160                 165

His Leu Asn Thr Val Phe Leu Gly Phe Arg Thr Leu Pro His Tyr
                170                 175                 180

Glu Glu Gly Ser Leu Pro Ile Leu Asn Thr Thr Lys Leu His Ile
                185                 190                 195

Val Leu Pro Met Asp Thr Asn Phe Trp Val Leu Leu Arg Asp Gly
                200                 205                 210

Ile Lys Thr Ser Lys Ile Leu Glu Met Thr Asn Ile Asp Gly Lys

                215                 220                 225

Ser Gln Phe Val Ser Tyr Glu Met Gln Arg Asn Leu Ser Leu Glu
                230                 235                 240

Asn Ala Lys Thr Ser Val Leu Leu Leu Asn Lys Val Asp Leu Leu
                245                 250                 255

Trp Asp Asp Leu Phe Leu Ile Leu Gln Phe Val Trp His Thr Ser
                260                 265                 270

Val Glu His Phe Gln Ile Arg Asn Val Thr Phe Gly Gly Lys Ala
                275                 280                 285

Tyr Leu Asp His Asn Ser Phe Asp Tyr Ser Asn Thr Val Met Arg
                290                 295                 300

Thr Ile Lys Leu Glu His Val His Phe Arg Val Phe Tyr Ile Gln
                305                 310                 315

Gln Asp Lys Ile Tyr Leu Leu Leu Thr Lys Met Asp Ile Glu Asn
                320                 325                 330

Leu Thr Ile Ser Asn Ala Gln Met Pro His Met Leu Phe Pro Asn
                335                 340                 345

Tyr Pro Thr Lys Phe Gln Tyr Leu Asn Phe Ala Asn Asn Ile Leu
                350                 355                 360

Thr Asp Glu Leu Phe Lys Arg Thr Ile Gln Leu Pro His Leu Lys
                365                 370                 375

Thr Leu Ile Leu Asn Gly Asn Lys Leu Glu Thr Leu Ser Leu Val
                380                 385                 390

Ser Cys Phe Ala Asn Asn Thr Pro Leu Glu His Leu Asp Leu Ser
                395                 400                 405

Gln Asn Leu Leu Gln His Lys Asn Asp Glu Asn Cys Ser Trp Pro
                410                 415                 420

Glu Thr Val Val Asn Met Asn Leu Ser Tyr Asn Lys Leu Ser Asp
                425                 430                 435

Ser Val Phe Arg Cys Leu Pro Lys Ser Ile Gln Ile Leu Asp Leu
                440                 445                 450

Asn Asn Asn Gln Ile Gln Thr Val Pro Lys Glu Thr Ile His Leu
                455                 460                 465

Met Ala Leu Arg Glu Leu Asn Ile Ala Phe Asn Phe Leu Thr Asp
                470                 475                 480

Leu Pro Gly Cys Ser His Phe Ser Arg Leu Ser Val Leu Asn Ile
                485                 490                 495

Glu Met Asn Phe Ile Leu Ser Pro Ser Leu Asp Phe Val Gln Ser
                500                 505                 510

Cys Gln Glu Val Lys Thr Leu Asn Ala Gly Arg Asn Pro Phe Arg
                515                 520                 525

Cys Thr Cys Glu Leu Lys Asn Phe Ile Gln Leu Glu Thr Tyr Ser
                530                 535                 540

Glu Val Met Met Val Gly Trp Ser Asp Ser Tyr Thr Cys Glu Tyr
                545                 550                 555

Pro Leu Asn Leu Arg Gly Ile Arg Leu Lys Asp Val His Leu His
                560                 565                 570

Glu Leu Ser Cys Asn Thr Ala Leu Leu Ile Val Thr Ile Val Val
                575                 580                 585

Ile Met Leu Val Leu Gly Leu Ala Val Ala Phe Cys Cys Leu His
                590                 595                 600

Phe Asp Leu Pro Trp Tyr Leu Arg Met Leu Gly Gln Cys Thr Gln
                605                 610                 615

Thr Trp His Arg Val Arg Lys Thr Thr Gln Glu Gln Leu Lys Arg
                620                 625                 630

Asn Val Arg Phe His Ala Phe Ile Ser Tyr Ser Glu His Asp Ser
                635                 640                 645

Leu Trp Val Lys Asn Glu Leu Ile Pro Asn Leu Glu Lys Glu Asp
                650                 655                 660

Gly Ser Ile Leu Ile Cys Leu Tyr Glu Ser Tyr Phe Asp Pro Gly
                665                 670                 675

Lys Ser Ile Ser Glu Asn Ile Val Ser Phe Ile Glu Lys Ser Tyr
                680                 685                 690

Lys Ser Ile Phe Val Leu Ser Pro Asn Phe Val Gln Asn Glu Trp
                695                 700                 705

Cys His Tyr Glu Phe Tyr Phe Ala His His Asn Leu Phe His Glu
                710                 715                 720

Asn Ser Asp His Ile Ile Leu Ile Leu Leu Glu Pro Ile Pro Phe
                725                 730                 735

Tyr Cys Ile Pro Thr Arg Tyr His Lys Leu Lys Ala Leu Leu Glu
                740                 745                 750

Lys Lys Ala Tyr Leu Glu Trp Pro Lys Asp Arg Arg Lys Cys Gly
                755                 760                 765

Leu Phe Trp Ala Asn Leu Arg Ala Ala Ile Asn Val Asn Val Leu
                770                 775                 780

Ala Thr Arg Glu Met Tyr Glu Leu Gln Thr Phe Thr Glu Leu Asn
                785                 790                 795

Glu Glu Ser Arg Gly Ser Thr Ile Ser Leu Met Arg Thr Asp Cys
                800                 805                 810

Leu

<210> 17
<211> 1911
<212> DNA
<213> Homo sapien

<400> 17
ccctgcgcgg ctgctggacc gacgggcgca cccaggtagg ggggcggctg 50

agccgcgcag tgcggaccct cgcggggaac tgcgccgccg ccaccatgtc 100

tcaggaaggt gtggagctgg agaagagcgt ccggcgcctc cgggagaagt 150

ttcatgggaa ggtatcctcc aagaaggcgg gggctctgat gaggaaattc 200

ggcagcgacc acacgggagt ggggcgctcc atcgtgtacg gggtaaagca 250
aaaagatggc caagaactaa gtaacgatct ggatgcccag gatccaccag 300
aagatatgaa gcaggaccgg gacattcagg cagtggcgac ctccctcctg 350
ccactgacag aagccaacct acgcatgttt caacgtgccc aggacgacct 400
tatccctgct gtggaccggc agtttgcctg ctcctcctgc gaccacgtct 450
ggtggcgccg cgtgccccag cggaaggagg tatcccggtg ccggaaatgc 500
cggaagcgct acgagccagt gccagctgac aagatgtggg gcctggctga 550
gttccactgc ccgaagtgtc ggcacaactt ccggggctgg gcacagatgg 600
ggtccccgtc cccctgctac gggtgcggct tccccgtgta tccaacacgg 650
atcctccccc cgcgccggga ccgggacccg gatcgccgca gcacccacac 700
tcactcctgc tcagctgccg actgctacaa ccggcgagag ccccacgtgc 750
ctgggacatc ctgtgctcac cccaagagcc ggaagcagaa ccacctgccc 800
aaagtgctcc accccagcaa ccctcacatt agcagtggcc ccactgtggc 850
cacctgcttg agccagggtg gcctcctgga agacctggac aacctcatcc 900
tggaggacct gaaggaggag gaggaggaag aggaggaggt ggaggacgag 950
gagggcgggc ccagggagtg acccctgcca ggtgcagata caaaccagac 1000
acggtctgtg gctactttgt gttattataa gatatgagct caaaccgaga 1050
tatgaatgac cttggggagc catctgaggc caagatattg acggggggga 1100
ttcctgggtc ccattttcag cgcccagggt cacagatcca cagtgggaag 1150
ttctgtggga cacattggca ctgagccaca aagaaggtgt ggccagaaca 1200
acttgggctc ctgctgacca atgtcctcta gggcctaggg gacagaggaa 1250
cacagagtca cagcttcagg ggccgaatga gcatggcggc cttcctgaga 1300
gaatatgccc caccacgaaa ctcagcccag tagacaccat cctggtagcg 1350
gcttcggtag tggccgccgt ggtgccacac accgttgagg ttggagtggg 1400
cacaggcatg gtaccaccag cctccccgct ggtacagggc acagttacct 1450
gaggggagag agagagtcca tgtcctctca ccagaataaa agcctctacc 1500
tgcacctcac agtgcaaggc ttttgccagg catcccctgg cccctcccat 1550
tcttattgaa tacaagccct gatcttccat ctcctcagca aaaaaatagg 1600
agccctggcc ccccaacttt cttcagagta atagccttaa ttccttccct 1650
atctccttac caaagtacaa gtcacatctt tcccaccttt tctgcaaact 1700
aggagtctac cgttcattcc tttatcaaag aaaagtatct acttcctttc 1750
tagaataaga gtactagctc tcaccctctg ccctttactt gaacaggagt 1800
cttgattctt tttttgcctc atcagagaag gaatctggac tccccatccc 1850
cccaccagga taaaagtcct gacctttgtt ctcttgacgg aataaaagct 1900

tgcttatcct t 1911

<210> 18
<211> 291
<212> PRT
<213> Homo sapien

<400> 18
Met Ser Gln Glu Gly Val Glu Leu Glu Lys Ser Val Arg Arg Leu
1               5                   10                  15

Arg Glu Lys Phe His Gly Lys Val Ser Ser Lys Lys Ala Gly Ala
                20                  25                  30

Leu Met Arg Lys Phe Gly Ser Asp His Thr Gly Val Gly Arg Ser
                35                  40                  45

Ile Val Tyr Gly Val Lys Gln Lys Asp Gly Gln Glu Leu Ser Asn
                50                  55                  60

Asp Leu Asp Ala Gln Asp Pro Pro Glu Asp Met Lys Gln Asp Arg
                65                  70                  75

Asp Ile Gln Ala Val Ala Thr Ser Leu Leu Pro Leu Thr Glu Ala
                80                  85                  90

Asn Leu Arg Met Phe Gln Arg Ala Gln Asp Asp Leu Ile Pro Ala
                95                  100                 105

Val Asp Arg Gln Phe Ala Cys Ser Ser Cys Asp His Val Trp Trp
                110                 115                 120

Arg Arg Val Pro Gln Arg Lys Glu Val Ser Arg Cys Arg Lys Cys
                125                 130                 135

Arg Lys Arg Tyr Glu Pro Val Pro Ala Asp Lys Met Trp Gly Leu
                140                 145                 150

Ala Glu Phe His Cys Pro Lys Cys Arg His Asn Phe Arg Gly Trp
                155                 160                 165

Ala Gln Met Gly Ser Pro Ser Pro Cys Tyr Gly Cys Gly Phe Pro
                170                 175                 180

Val Tyr Pro Thr Arg Ile Leu Pro Pro Arg Arg Asp Arg Asp Pro
                185                 190                 195

Asp Arg Arg Ser Thr His Thr His Ser Cys Ser Ala Ala Asp Cys
                200                 205                 210

Tyr Asn Arg Arg Glu Pro His Val Pro Gly Thr Ser Cys Ala His
                215                 220                 225

Pro Lys Ser Arg Lys Gln Asn His Leu Pro Lys Val Leu His Pro
                230                 235                 240

Ser Asn Pro His Ile Ser Ser Gly Pro Thr Val Ala Thr Cys Leu
                245                 250                 255

Ser Gln Gly Gly Leu Leu Glu Asp Leu Asp Asn Leu Ile Leu Glu
                260                 265                 270

Asp Leu Lys Glu Glu Glu Glu Glu Glu Glu Glu Val Glu Asp Glu
                275                 280                 285

Glu Gly Gly Pro Arg Glu
                290

<210> 19
<211> 1603
<212> DNA
<213> Homo sapien

<400> 19
ggtggtccag gaaaaggcgc tccgtcatgg ggatccagac gagccccgtc 50

ctgctggcct ccctgggggt ggggctggtc actctgctcg gcctggctgt 100

gggctcctac ttggttcgga ggtcccgccg gcctcaggtc actctcctgg 150

accccaatga aaagtacctg ctacgactgc tagacaagac gactgtgagc 200

cacaacacca agaggttccg ctttgccctg cccaccgccc accacactct 250

ggggctgcct gtgggcaaac atatctacct ctccacccga attgatggca 300

acctggtcat caggccatac actcctgtca ccagtgatga ggatcaaggc 350

tatgtggatc ttgtcatcaa ggtctacctg aagggtgtgc accccaaatt 400

tcctgaggga gggaagatgt ctcagtacct ggatagcctg aaggttgggc 450

atgtggtgga gtttcggggg ccaagcgggt tgctcactta cactggaaaa 500

gggcatttta acattcagcc caacaagaaa tctccaccag aaccccgagt 550

ggcgaagaaa ctgggaatga ttgccggcgg gacaggaatc accccaatgc 600

tacagctgat ccgggccatc ctgaaagtcc ctgaagatcc aacccagtgc 650

tttctgcttt ttgccaacca gacagaaaag gatatcatct tgcgggagga 700

cttagaggaa ctgcaggccc gctatcccaa tcgctttaag ctctggttca 750

ctctggatca tcccccaaaa gattgggcct acagcaaggg ctttgtgact 800

gccgacatga tccgggaaca cctgcccgct ccaggggatg atgtgctggt 850

actgctttgt gggccacccc caatggtgca gctggcctgc catcccaact 900

tggacaaact gggctactca caaaagatgc gattcaccta ctgagcatcc 950

tccagcttcc ctggtgctgt tcgctgcagt tgttccccat cagtactcaa 1000

gcactataag ccttagattc ctttcctcag agtttcaggt tttttcagtt 1050

acatctagag ctgaaatctg gatagtacct gcaggaacaa tattcctgta 1100

gccatggaag aggcccaagg ctcagtcact ccttggatgg cctcctaaat 1150

ctccccgtgg caacaggtcc aggagaggcc catggagcag tctcttccat 1200

ggagtaagaa ggaagggagc atgtacgctt ggtccaagat tggctagttc 1250

cttgatagca tcttactctc accttctttg tgtctgtgat gaaaggaaca 1300

gtctgtgcaa tgggttttac ttaaacttca ctgttcaacc tatgagcaaa 1350

tctgtatgtg tgagtataag ttgagcatag catacttcca gaggtggtct 1400

tatggagatg gcaagaaagg aggaaatgat ttcttcagat ctcaaaggag 1450

tctgaaatat catatttctg tgtgtgtctc tctcagcccc tgcccaggct 1500

agagggaaac agctactgat aatcgaaaac tgctgtttgt ggcaggaacc 1550

cctggctgtg caaataatac tggctgaggc ccctgtgtga tattgaaaaa 1600

aaa 1603

<210> 20
<211> 305
<212> PRT
<213> Homo sapien

<400> 20
Met Gly Ile Gln Thr Ser Pro Val Leu Leu Ala Ser Leu Gly Val
1               5                   10                  15

Gly Leu Val Thr Leu Leu Gly Leu Ala Val Gly Ser Tyr Leu Val
                20                  25                  30

Arg Arg Ser Arg Arg Pro Gln Val Thr Leu Leu Asp Pro Asn Glu
                35                  40                  45

Lys Tyr Leu Leu Arg Leu Leu Asp Lys Thr Thr Val Ser His Asn
                50                  55                  60

Thr Lys Arg Phe Arg Phe Ala Leu Pro Thr Ala His His Thr Leu
                65                  70                  75

Gly Leu Pro Val Gly Lys His Ile Tyr Leu Ser Thr Arg Ile Asp
                80                  85                  90

Gly Asn Leu Val Ile Arg Pro Tyr Thr Pro Val Thr Ser Asp Glu
                95                  100                 105

Asp Gln Gly Tyr Val Asp Leu Val Ile Lys Val Tyr Leu Lys Gly
                110                 115                 120

Val His Pro Lys Phe Pro Glu Gly Gly Lys Met Ser Gln Tyr Leu
                125                 130                 135

Asp Ser Leu Lys Val Gly His Val Val Glu Phe Arg Gly Pro Ser
                140                 145                 150

Gly Leu Leu Thr Tyr Thr Gly Lys Gly His Phe Asn Ile Gln Pro
                155                 160                 165

Asn Lys Lys Ser Pro Pro Glu Pro Arg Val Ala Lys Lys Leu Gly
                170                 175                 180

Met Ile Ala Gly Gly Thr Gly Ile Thr Pro Met Leu Gln Leu Ile
                185                 190                 195

Arg Ala Ile Leu Lys Val Pro Glu Asp Pro Thr Gln Cys Phe Leu
                200                 205                 210

Leu Phe Ala Asn Gln Thr Glu Lys Asp Ile Ile Leu Arg Glu Asp
                215                 220                 225

Leu Glu Glu Leu Gln Ala Arg Tyr Pro Asn Arg Phe Lys Leu Trp
                230                 235                 240

Phe Thr Leu Asp His Pro Pro Lys Asp Trp Ala Tyr Ser Lys Gly
                245                 250                 255

Phe Val Thr Ala Asp Met Ile Arg Glu His Leu Pro Ala Pro Gly
                260                 265                 270

Asp Asp Val Leu Val Leu Leu Cys Gly Pro Pro Pro Met Val Gln
                275                 280                 285

Leu Ala Cys His Pro Asn Leu Asp Lys Leu Gly Tyr Ser Gln Lys

290                 295                 300

Met Arg Phe Thr Tyr
                305

<210> 21
<211> 2728
<212> DNA
<213> Homo sapien

<400> 21
accgcggaaa gcatgttgtg gctgttccaa tcgctcctgt ttgtcttctg 50

ctttggccca gggaatgtag tttcacaaag cagcttaacc ccattgatgg 100

tgaacgggat tctgggggag tcagtaactc ttcccctgga gtttcctgca 150

ggagagaagg tcaacttcat cacttggctt ttcaatgaaa catctcttgc 200

cttcatagta ccccatgaaa ccaaaagtcc agaaatccac gtgactaatc 250

cgaaacaggg aaagcgactg aacttcaccc agtcctactc cctgcaactc 300

agcaacctga agatggaaga cacaggctct tacagagccc agatatccac 350

aaagacctct gcaaagctgt ccagttacac tctgaggata ttaagacaac 400

tgaggaacat acaagttacc aatcacagtc agctatttca gaatatgacc 450

tgtgagctcc atctgacttg ctctgtggag gatgcagatg acaatgtctc 500

attcagatgg gaggccttgg gaaacacact ttcaagtcag ccaaacctca 550

ctgtctcctg ggaccccagg atttccagtg aacaggacta cacctgcata 600

gcagagaatg ctgtcagtaa tttatccttc tctgtctctg cccagaagct 650

ttgcgaagat gttaaaattc aatatacaga taccaaaatg attctgttta 700

tggtttctgg gatatgcata gtcttcggtt tcatcatact gctgttactt 750

gttttgagga aaagaagaga ttccctatct ttgtctactc agcgaacaca 800

gggccccgag tccgcaagga acctagagta tgtttcagtg tctccaacga 850

acaacactgt gtatgcttca gtcactcatt caaacaggga aacagaaatc 900

tggacaccta gagaaaatga tactatcaca atttactcca caattaatca 950

ttccaaagag agtaaaccca ctttttccag ggcaactgcc cttgacaatg 1000

tcgtgtaagt tgctgaaagg cctcagagga attcgggaat gacacgtctt 1050

ctgatcccat gagacagaac aaagaacagg aagcttggtt cctgttgttc 1100

ctggcaacag aatttgaata tctaggatag gatgatcacc tccagtcctt 1150

cggacttaaa cctgcctacc tgagtcaaac acctaaggat aacatcattt 1200

ccagcatgtg gttcaaataa tattttccaa tccacttcag gccaaaacat 1250

gctaaagata acacaccagc acattgactc tctctttgat aactaagcaa 1300

atggaattat ggttgacaga gagtttatga tccagaagac aaccacttct 1350

ctccttttag aaagcagcag gattgactta ttgagaaata atgcagtgtg 1400

ttggttacat gtgtagtctc tggagttgga tgggcccatc ctgatacaag 1450

ttgagcatcc cttgtctgaa atgcttggga ttagaaatgt ttcagatttc 1500

aatttttttt cagattttgg aatatttgca ttatatttag cggttgagta 1550

tccaaatcca aaaatccaaa attcaaaatg ctccaataag catttccctt 1600

gagtttcatt gatgtcgatg cagtgctcaa aatctcagat tttggagcat 1650

tttggatatt ggatttttgg atttgggatg ctcaacttgt acaatgttta 1700

ttagacacat ctcctgggac atactgccta accttttgga gccttagtct 1750

cccagactga aaaaggaaga ggatggtatt acatcagctc cattgtttga 1800

gccaagaatc taagtcatcc ctgactccag tgtctttgtc accaggccct 1850

ttggactcta cctcagaaat atttcttgga ccttccactt ctcctccaac 1900

tccttgacca ccatcctgta tccaaccatc accacctcta acctgaatcc 1950

taccttaaga tcagaacagt tgtcctcact tttgttcttg tccctctcca 2000

acccactctc cacaagatgg ccagagtaat gtttttaata taaattggat 2050

ccttcagttt cctgcttaaa accctgcagg tttcccaatg cactcagaaa 2100

gaaatccagt ttccatggcc ctggatggtc tggcccacct ccagcctcag 2150

ctagcattac ccttctgaca ctctctatgt agcctccctg atcttctttc 2200

agctcctcta ttaaaggaaa agttctttat gttaattatt tacatcttcc 2250

tgcaggccct tcctctgcct gctggggtcc tcctattctt taggtttaat 2300

tttaaatatg tcacctccta agagaaacct tcccagacca ctctttctaa 2350

aatgaatctt ctaggctggg catggtggct cacacctgta atcccagtac 2400

tttgggaggc caagggggga gatcacttga ggtcaggagt tcaagaccag 2450

cctggccaac ttggtgaaac cccgtcttta ctaaaaatac aaaaaaatta 2500

gccaggcgtg gtggtgcacc cctaaaatcc cagctacttg agagactgag 2550

gcaggagaat cgcttgaacc caggaggtgg aggttccagt gagccaaaat 2600

catgccaatg tattccagtc tgggtgacag agtgagactc tgtctcaaaa 2650

aataaataaa taaaataaaa tgaaatagat cttataaaaa aaaaaaaaaa 2700

aaaaaaaaaa aaaaaaaaaa aaaaaaaa 2728

<210> 22
<211> 331
<212> PRT
<213> Homo sapien

<400> 22
Met Leu Trp Leu Phe Gln Ser Leu Leu Phe Val Phe Cys Phe Gly
1               5                   10                  15

Pro Gly Asn Val Val Ser Gln Ser Ser Leu Thr Pro Leu Met Val
                20                  25                  30

Asn Gly Ile Leu Gly Glu Ser Val Thr Leu Pro Leu Glu Phe Pro
                35                  40                  45

Ala Gly Glu Lys Val Asn Phe Ile Thr Trp Leu Phe Asn Glu Thr
                50                  55                  60

Ser Leu Ala Phe Ile Val Pro His Glu Thr Lys Ser Pro Glu Ile
                65                  70                  75

His Val Thr Asn Pro Lys Gln Gly Lys Arg Leu Asn Phe Thr Gln
                80                  85                  90

Ser Tyr Ser Leu Gln Leu Ser Asn Leu Lys Met Glu Asp Thr Gly
                95                  100                 105

Ser Tyr Arg Ala Gln Ile Ser Thr Lys Thr Ser Ala Lys Leu Ser
                110                 115                 120

Ser Tyr Thr Leu Arg Ile Leu Arg Gln Leu Arg Asn Ile Gln Val
                125                 130                 135

Thr Asn His Ser Gln Leu Phe Gln Asn Met Thr Cys Glu Leu His
                140                 145                 150

Leu Thr Cys Ser Val Glu Asp Ala Asp Asp Asn Val Ser Phe Arg
                155                 160                 165

Trp Glu Ala Leu Gly Asn Thr Leu Ser Ser Gln Pro Asn Leu Thr
                170                 175                 180

Val Ser Trp Asp Pro Arg Ile Ser Ser Glu Gln Asp Tyr Thr Cys
                185                 190                 195

Ile Ala Glu Asn Ala Val Ser Asn Leu Ser Phe Ser Val Ser Ala
                200                 205                 210

Gln Lys Leu Cys Glu Asp Val Lys Ile Gln Tyr Thr Asp Thr Lys
                215                 220                 225

Met Ile Leu Phe Met Val Ser Gly Ile Cys Ile Val Phe Gly Phe
                230                 235                 240

Ile Ile Leu Leu Leu Leu Val Leu Arg Lys Arg Arg Asp Ser Leu
                245                 250                 255

Ser Leu Ser Thr Gln Arg Thr Gln Gly Pro Glu Ser Ala Arg Asn
                260                 265                 270

Leu Glu Tyr Val Ser Val Ser Pro Thr Asn Asn Thr Val Tyr Ala
                275                 280                 285

Ser Val Thr His Ser Asn Arg Glu Thr Glu Ile Trp Thr Pro Arg
                290                 295                 300

Glu Asn Asp Thr Ile Thr Ile Tyr Ser Thr Ile Asn His Ser Lys
                305                 310                 315

Glu Ser Lys Pro Thr Phe Ser Arg Ala Thr Ala Leu Asp Asn Val
                320                 325                 330

Val

<210> 23
<211> 4796
<212> DNA
<213> Homo sapien

<400> 23
gagaggacga ggtgccgctg cctggagaat cctccgctgc cgtcggctcc 50

cggagcccag ccctttccta acccaaccca acctagccca gtcccagccg 100
ccagcgcctg tccctgtcac ggaccccagc gttaccatgc atcctgccgt 150
cttcctatcc ttacccgacc tcagatgctc ccttctgctc ctggtaactt 200
gggtttttac tcctgtaaca actgaaataa caagtcttga tacagagaat 250
atagatgaaa ttttaaacaa tgctgatgtt gctttagtaa atttttatgc 300
tgactggtgt cgtttcagtc agatgttgca tccaattttt gaggaagctt 350
ccgatgtcat taaggaagaa tttccaaatg aaaatcaagt agtgtttgcc 400
agagttgatt gtgatcagca ctctgacata gcccagagat acaggataag 450
caaataccca accctcaaat tgtttcgtaa tgggatgatg atgaagagag 500
aatacagggg tcagcgatca gtgaaagcat tggcagatta catcaggcaa 550
caaaaaagtg accccattca agaaattcgg gacttagcag aaatcaccac 600
tcttgatcgc agcaaaagaa atatcattgg atattttgag caaaaggact 650
cggacaacta tagagttttt gaacgagtag cgaatatttt gcatgatgac 700
tgtgcctttc tttctgcatt tggggatgtt tcaaaaccgg aaagatatag 750
tggcgacaac ataatctaca aaccaccagg gcattctgct ccggatatgg 800
tgtacttggg agctatgaca aattttgatg tgacttacaa ttggattcaa 850
gataaatgtg ttcctcttgt ccgagaaata acatttgaaa atggagagga 900
attgacagaa gaaggactgc cttttctcat actctttcac atgaaagaag 950
atacagaaag tttagaaata ttccagaatg aagtagctcg gcaattaata 1000
agtgaaaaag gtacaataaa ctttttacat gccgattgtg acaaatttag 1050
acatcctctt ctgcacatac agaaaactcc agcagattgt cctgtaatcg 1100
ctattgacag ctttaggcat atgtatgtgt ttggagactt caaagatgta 1150
ttaattcctg gaaaactcaa gcaattcgta tttgacttac attctggaaa 1200
actgcacaga gaattccatc atggacctga cccaactgat acagccccag 1250
gagagcaagc ccaagatgta gcaagcagtc cacctgagag ctccttccag 1300
aaactagcac ccagtgaata taggtatact ctattgaggg atcgagatga 1350
gctttaaaaa cttgaaaaac agtttgtaag cctttcaaca gcagcatcaa 1400
cctacgtggt ggaaatagta aacctatatt ttcataattc tatgtgtatt 1450
tttattttga ataaacagaa agaaattttg ggtttttaat ttttttctcc 1500
ccgactcaaa atgcattgtc atttaatata gtagcctctt aaaaaaaaaa 1550
aaacctgcta ggattaaaaa ataaaaatca gaggcctatc tccactttaa 1600
atctgtcctg taaaagtttt ataaatcaaa tgaaaggtga cattgccaga 1650
aacttaccat taacttgcac tactagggta gggaggactt aggatgtttc 1700
ctgtgtcgta tgtgcttttc tttctttcat atgatcaatt ctgttggtat 1750

tttcagtatc tcatttctca aagctaaaga gatatacatt ctggatactt 1800
gggaggggaa taaattaaag ttttcacact gtgtactgtg ttttactgat 1850
tggttggata ttgcttatga aaattccata gtggtatttt tttggattct 1900
taatgtgtaa cttaaacata ctttgaagtg gaggagagtc ataagacaga 1950
acatttggca ggaattgtcc ttatgaaaca agaaaaagaa aatgaaaagt 2000
attattaagc ttctgtgttt gtctaaaaat gtggcatatg gatggcattt 2050
aaaactttga atgaattata cctaaatctg ggacagggag gtgacagtgg 2100
aacaggctac caatcagaac tagatgactt ttaaggctcc tcctattatg 2150
agacttcaat ttccaaagag aagaactagc agagaaattg tatttcagta 2200
attttaagct ccttctgtct tgtagagtct tgttatagtt gtataaatca 2250
aaaacacaga ataaggaaca tatttaactt tttttcatta taaaatggtt 2300
agaggaccct accccctcta gattccctga tttccccagg cctgcagcat 2350
acagtaagat gggtccctgt gccaggcctc aatactgcca gggaataaaa 2400
ccagagggag aggaccctca gtgtcatatc aggaagccca gtgccagagg 2450
acagacaggt tcaaaactgg cttttcctct gggcctgggt tggtgctata 2500
ggccaagggt cattttatac ttgggtataa atcaatccca gtttgggaaa 2550
agattatttt taagcttaaa aggctgacat gtgccattat atgtagtatg 2600
taatatatgt aacatcttcc aattctttta aaataaaatt aatatttata 2650
atggatattt aatgattgtt atttttaaaa accagcttat aattcctcgt 2700
tatgcatgat ttatccaaag tttccatagt tttattcaaa ataataaatg 2750
ttaataaggt gataaggggt atatttaatg tattgtatca aattgtgaat 2800
aagaaagtag gatggagctt tctagaggtt gggccttagt tctgttatcc 2850
tcattgcttt taaccaataa gttaaatgaa gttagagtta tggtcttcag 2900
gttagattat ggaccagatc tgtgagggtc agcatggaaa ttcacattca 2950
acaaggtagc acacaggacc aagagcagca catgcaatca actggaataa 3000
tatagtaatc ctgtaactgg gtttgaaaaa ataatcaaca aaagatacaa 3050
ttcaagggtt aggttgcaga gagctggctt gagagtagtt attatgaaaa 3100
aggcctcaag gagtacgtgt tcagtatgct ctaagatgat aaagtggctg 3150
ttaaaaaggg agttgatttg aggaagtatt acttagcatt catgcatatt 3200
gggcttaggc tctagccctg ccactatcat tgtcttctct ggactgtgaa 3250
gtcactgagg acaaggaaac taaatttaat gtctgtatca ctagtgccta 3300
gaatttctgg acacttagta gtcaccatca ggcgtttatt taatgaatga 3350
gaagcaaagt gaccttggtt acttttttac cctgaggggc tcagcactca 3400
ttaggacttg gtgcctaatt ttataaaaag tcactaagct caagtgcttg 3450

gatgaaagga cagcgtggat aaaaaggttt ttaaaacatg gatgttaagg 3500
ctgttttgct tggagaagac ttgggactgg gacagtcttt agatattatt 3550
tgaaatgctg gcactgtcta tctggatccc agggcttgaa ctaggatttg 3600
aggaagtcac agggaagcag atttcagtct gacatttatt cagtgcaagt 3650
tttttggtgc tgtagtatat gatgaaagat gtaaagctga ataaagcatt 3700
atttctgccc tagagttgtt cacagcctag tcaggcatat ggatatgtaa 3750
acaatgactg taacgtgtta tagatgtaaa gacaaaataa aggttaaaga 3800
gggcataaag gagcactcaa ttgcagagat ttgaggacat tatttttatt 3850
ttgagcttta aaaagatgaa taggtgttct caggaggtag ggatctggct 3900
gagagggaat aatctgagca aaggtatgaa acagcctaat gcattagaga 3950
aaaaagttct tttagtaagg catttggggt tggggaagct agaaaaagaa 4000
atgggagctg gtcacacagg gccttgtgtg ccagactaag gggtttgtag 4050
tatatattgt aggcagaaga gatccatcaa cagattgcaa gcaaggaagt 4100
atgttcactt taaagtttga gaaagaatag tgtggaagca cgtctcaaat 4150
ttagacttac ttgttccccc tctgaaccgt gaatcagacc atttcaggta 4200
gaagtcttcc ccggtttatc tgatctactc ggggcctcag gcttctcagc 4250
tgggaagaga ggatgcaaga ccagactgaa gaacacggtt gagtccccag 4300
aaccaaaagg gggcctttct gcttcttagc cagctacctc ttcgagtttt 4350
tcaaattgtg agggggacca taaaaggatg gaaactttta gatgacattc 4400
tacaaattat ttttttcttt aaattaaaag aacctagcca ataagataga 4450
gaatgggcat ctaaggcatc tcagagctct ctgatgaagc caggttgtca 4500
aagatcattt gcaaaagaag ggaaaactgg catgacaaaa gctacagaga 4550
ggagagtgaa atatagaagt gtttgaaatg ttcaagctca caataagctt 4600
aaatttatag aaaatgctaa ggttgtcaag aaggcttttt tttttttctt 4650
ttttaaacct gagggcaaaa aggaatggat aaagtagtgt aatggattga 4700
caatcaggaa gaacagaata actcagtttt tttttctcct acaaggagat 4750
atggctggac caaaataaaa tgacatgaaa ttgcaaaaat gaaaat 4796

<210> 24
<211> 451
<212> PRT
<213> Homo sapien

<400> 24
Arg Gly Arg Gly Ala Ala Ala Trp Arg Ile Leu Arg Cys Arg Arg
1               5                   10                  15

Leu Pro Glu Pro Ser Pro Phe Leu Thr Gln Pro Asn Leu Ala Gln
                20                  25                  30

Ser Gln Pro Pro Ala Pro Val Pro Val Thr Asp Pro Ser Val Thr
                35                  40                  45

Met His Pro Ala Val Phe Leu Ser Leu Pro Asp Leu Arg Cys Ser
                50                  55                  60

Leu Leu Leu Leu Val Thr Trp Val Phe Thr Pro Val Thr Thr Glu
                65                  70                  75

Ile Thr Ser Leu Asp Thr Glu Asn Ile Asp Glu Ile Leu Asn Asn
                80                  85                  90

Ala Asp Val Ala Leu Val Asn Phe Tyr Ala Asp Trp Cys Arg Phe
                95                  100                 105

Ser Gln Met Leu His Pro Ile Phe Glu Glu Ala Ser Asp Val Ile
                110                 115                 120

Lys Glu Glu Phe Pro Asn Glu Asn Gln Val Val Phe Ala Arg Val
                125                 130                 135

Asp Cys Asp Gln His Ser Asp Ile Ala Gln Arg Tyr Arg Ile Ser
                140                 145                 150

Lys Tyr Pro Thr Leu Lys Leu Phe Arg Asn Gly Met Met Met Lys
                155                 160                 165

Arg Glu Tyr Arg Gly Gln Arg Ser Val Lys Ala Leu Ala Asp Tyr
                170                 175                 180

Ile Arg Gln Gln Lys Ser Asp Pro Ile Gln Glu Ile Arg Asp Leu
                185                 190                 195

Ala Glu Ile Thr Thr Leu Asp Arg Ser Lys Arg Asn Ile Ile Gly
                200                 205                 210

Tyr Phe Glu Gln Lys Asp Ser Asp Asn Tyr Arg Val Phe Glu Arg
                215                 220                 225

Val Ala Asn Ile Leu His Asp Asp Cys Ala Phe Leu Ser Ala Phe
                230                 235                 240

Gly Asp Val Ser Lys Pro Glu Arg Tyr Ser Gly Asp Asn Ile Ile
                245                 250                 255

Tyr Lys Pro Pro Gly His Ser Ala Pro Asp Met Val Tyr Leu Gly
                260                 265                 270

Ala Met Thr Asn Phe Asp Val Thr Tyr Asn Trp Ile Gln Asp Lys
                275                 280                 285

Cys Val Pro Leu Val Arg Glu Ile Thr Phe Glu Asn Gly Glu Glu
                290                 295                 300

Leu Thr Glu Glu Gly Leu Pro Phe Leu Ile Leu Phe His Met Lys
                305                 310                 315

Glu Asp Thr Glu Ser Leu Glu Ile Phe Gln Asn Glu Val Ala Arg
                320                 325                 330

Gln Leu Ile Ser Glu Lys Gly Thr Ile Asn Phe Leu His Ala Asp
                335                 340                 345

Cys Asp Lys Phe Arg His Pro Leu Leu His Ile Gln Lys Thr Pro
                350                 355                 360

Ala Asp Cys Pro Val Ile Ala Ile Asp Ser Phe Arg His Met Tyr
                365                 370                 375

Val Phe Gly Asp Phe Lys Asp Val Leu Ile Pro Gly Lys Leu Lys

380                 385                 390

Gln Phe Val Phe Asp Leu His Ser Gly Lys Leu His Arg Glu Phe
                395                 400                 405

His His Gly Pro Asp Pro Thr Asp Thr Ala Pro Gly Glu Gln Ala
                410                 415                 420

Gln Asp Val Ala Ser Ser Pro Pro Glu Ser Ser Phe Gln Lys Leu
                425                 430                 435

Ala Pro Ser Glu Tyr Arg Tyr Thr Leu Leu Arg Asp Arg Asp Glu
                440                 445                 450

Leu

<210> 25
<211> 810
<212> DNA
<213> Homo sapien

<400> 25
gctggagccg ggccggggcg atgtggagcg cgggccgcgg cggggctgcc 50

tggccggtgc tgttggggct gctgctggcg ctgttagtgc cgggcggtgg 100

tgccgccaag accggtgcgg agctcgtgac ctgcgggtcg gtgctgaagc 150

tgctcaatac gcaccaccgc gtgcggctgc actcgcacga catcaaatac 200

ggatccggca gcggccagca atcggtgacc ggcgtagagg cgtcggacga 250

cgcgaatagc tactggcgga tccgcggcgg ctcggagggc gggtgcccgt 300

gcgggtcccc ggtgcgctgc gggcaggcgg tgaggctcac gcatgtgctt 350

acgggcaaga acctgcacac gcaccacttc ccgtcgccgc tgtccaacaa 400

ccaggaggtg agtgcctttg gggaagacgg cgagggcgac gacctggacc 450

tatggacagt gcgctgctct ggacagcact gggagcgtga ggctgctgtg 500

cgcttacagc atgtgggcac ctctgtgttc ctgtcagtca cgggtgagca 550

gtatggaagc cccatccgtg ggcagcatga ggtccacggc atgcccagtg 600

ccaacacgca caatacgtgg aaggccatgg aaggcatctt catcaagcct 650

agtgtggagc cctctgcagg tcacgatgaa ctctgagtgt gtggatggat 700

gggtggatgg agggtggcag gtggggcgtc tgcagggcca ctcttggcag 750

agactttggg tttgtagggg tcctcaagtg cctttgtgat taaagaatgt 800

tggtctatga 810

<210> 26
<211> 221
<212> PRT
<213> Homo sapien

<400> 26
Met Trp Ser Ala Gly Arg Gly Gly Ala Ala Trp Pro Val Leu Leu
  1               5                  10                  15

Gly Leu Leu Leu Ala Leu Leu Val Pro Gly Gly Gly Ala Ala Lys
                 20                  25                  30

Thr Gly Ala Glu Leu Val Thr Cys Gly Ser Val Leu Lys Leu Leu
                35                  40                  45

Asn Thr His His Arg Val Arg Leu His Ser His Asp Ile Lys Tyr
                50                  55                  60

Gly Ser Gly Ser Gly Gln Gln Ser Val Thr Gly Val Glu Ala Ser
                65                  70                  75

Asp Asp Ala Asn Ser Tyr Trp Arg Ile Arg Gly Gly Ser Glu Gly
                80                  85                  90

Gly Cys Pro Cys Gly Ser Pro Val Arg Cys Gly Gln Ala Val Arg
                95                  100                 105

Leu Thr His Val Leu Thr Gly Lys Asn Leu His Thr His His Phe
                110                 115                 120

Pro Ser Pro Leu Ser Asn Asn Gln Glu Val Ser Ala Phe Gly Glu
                125                 130                 135

Asp Gly Glu Gly Asp Asp Leu Asp Leu Trp Thr Val Arg Cys Ser
                140                 145                 150

Gly Gln His Trp Glu Arg Glu Ala Ala Val Arg Leu Gln His Val
                155                 160                 165

Gly Thr Ser Val Phe Leu Ser Val Thr Gly Glu Gln Tyr Gly Ser
                170                 175                 180

Pro Ile Arg Gly Gln His Glu Val His Gly Met Pro Ser Ala Asn
                185                 190                 195

Thr His Asn Thr Trp Lys Ala Met Glu Gly Ile Phe Ile Lys Pro
                200                 205                 210

Ser Val Glu Pro Ser Ala Gly His Asp Glu Leu
                215                 220

<210> 27
<211> 1256
<212> DNA
<213> Homo sapien

<400> 27
acgaggggag ctccggctgc gtcttcccgc agcgctaccc gccatgcgcc 50

tgccgcgccg ggccgcgctg gggctcctgc cgcttctgct gctgctgccg 100

cccgcgccgg aggccgccaa gaagccgacg ccctgccacc ggtgccgggg 150

gctggtggac aagtttaacc aggggatggt ggacaccgca aagaagaact 200

ttggcggcgg gaacacggct tgggaggaaa agacgctgtc caagtacgag 250

tccagcgaga ttcgcctgct ggagatcctg gaggggctgt gcgagagcag 300

cgacttcgaa tgcaatcaga tgctagaggc gcaggaggag cacctggagg 350

cctggtggct gcagctgaag agcgaatatc ctgacttatt cgagtggttt 400

tgtgtgaaga cactgaaagt gtgctgctct ccaggaacct acggtcccga 450

ctgtctcgca tgccagggcg gatcccagag gccctgcagc gggaatggcc 500

actgcagcgg agatgggagc agacagggcg acgggtcctg ccggtgccac 550

atggggtacc agggcccgct gtgcactgac tgcatggacg gctacttcag 600

ctcgctccgg aacgagaccc acagcatctg cacagcctgt gacgagtcct 650

gcaagacgtg ctcgggcctg accaacagag actgcggcga gtgtgaagtg 700

ggctgggtgc tggacgaggg cgcctgtgtg gatgtggacg agtgtgcggc 750

cgagccgcct ccctgcagcg ctgcgcagtt ctgtaagaac gccaacggct 800

cctacacgtg cgaagatgtg gacgagtgct cactagcaga aaaaacctgt 850

gtgaggaaaa acgaaaactg ctacaatact ccagggagct acgtctgtgt 900

gtgtcctgac ggcttcgaag aaacggaaga tgcctgtgtg ccgccggcag 950

aggctgaagc cacagaagga gaaagcccga cacagctgcc ctcccgcgaa 1000

gacctgtaat gtgccggact taccctttaa attattcaga aggatgtccc 1050

gtggaaaatg tggccctgag gatgccgtct cctgcagtgg acagcggcgg 1100

ggagaggctg cctgctctct aacggttgat tctcatttgt cccttaaaca 1150

gctgcatttc ttggttgttc ttaaacagac ttgtatattt tgatacagtt 1200

ctttgtaata aaattgacca ttgtaggtaa tcaggaaaaa aaaaaaaaaa 1250

aaaaaa 1256

<210> 28
<211> 321
<212> PRT
<213> Homo sapien

<400> 28
Met Arg Leu Pro Arg Arg Ala Ala Leu Gly Leu Leu Pro Leu Leu
1               5                   10                  15

Leu Leu Leu Pro Pro Ala Pro Glu Ala Ala Lys Lys Pro Thr Pro
                20                  25                  30

Cys His Arg Cys Arg Gly Leu Val Asp Lys Phe Asn Gln Gly Met
                35                  40                  45

Val Asp Thr Ala Lys Lys Asn Phe Gly Gly Gly Asn Thr Ala Trp
                50                  55                  60

Glu Glu Lys Thr Leu Ser Lys Tyr Glu Ser Ser Glu Ile Arg Leu
                65                  70                  75

Leu Glu Ile Leu Glu Gly Leu Cys Glu Ser Ser Asp Phe Glu Cys
                80                  85                  90

Asn Gln Met Leu Glu Ala Gln Glu Glu His Leu Glu Ala Trp Trp
                95                  100                 105

Leu Gln Leu Lys Ser Glu Tyr Pro Asp Leu Phe Glu Trp Phe Cys
                110                 115                 120

Val Lys Thr Leu Lys Val Cys Cys Ser Pro Gly Thr Tyr Gly Pro
                125                 130                 135

Asp Cys Leu Ala Cys Gln Gly Gly Ser Gln Arg Pro Cys Ser Gly
                140                 145                 150

Asn Gly His Cys Ser Gly Asp Gly Ser Arg Gln Gly Asp Gly Ser
                155                 160                 165

Cys Arg Cys His Met Gly Tyr Gln Gly Pro Leu Cys Thr Asp Cys
                170                 175                 180

Met Asp Gly Tyr Phe Ser Ser Leu Arg Asn Glu Thr His Ser Ile
                185                 190                 195

Cys Thr Ala Cys Asp Glu Ser Cys Lys Thr Cys Ser Gly Leu Thr
                200                 205                 210

Asn Arg Asp Cys Gly Glu Cys Glu Val Gly Trp Val Leu Asp Glu
                215                 220                 225

Gly Ala Cys Val Asp Val Asp Glu Cys Ala Ala Glu Pro Pro Pro
                230                 235                 240

Cys Ser Ala Ala Gln Phe Cys Lys Asn Ala Asn Gly Ser Tyr Thr
                245                 250                 255

Cys Glu Asp Val Asp Glu Cys Ser Leu Ala Glu Lys Thr Cys Val
                260                 265                 270

Arg Lys Asn Glu Asn Cys Tyr Asn Thr Pro Gly Ser Tyr Val Cys
                275                 280                 285

Val Cys Pro Asp Gly Phe Glu Glu Thr Glu Asp Ala Cys Val Pro
                290                 295                 300

Pro Ala Glu Ala Glu Ala Thr Glu Gly Glu Ser Pro Thr Gln Leu
                305                 310                 315

Pro Ser Arg Glu Asp Leu
                320

51

1

1